# EUROPEAN PATENT SPECIFICATION

(11) **EP 1 385 877 B1**
(45) Date of publication and mention of the grant of the patent: **06.10.2010**
(21) Application number: 01954140.8
(22) Date of filing: 03.08.2001
(51) Int. Cl.: C07K 14/47

(54) **TAPP2 POLYPEPTIDES**
TAPP2 POLYPEPTIDE
POLYPEPTIDES TAPP2

(30) Priority: 03.08.2000 GB 0018908; 05.09.2000 GB 0021685
(43) Date of publication of application: 04.02.2004
(73) Proprietor: Medical Research Council, 20 Park Crescent London W1B 1AL (GB)
(72) Inventor: DOWLER, Simon, Medical Research Council, MSI/WTB Complex Uni. of Dundee DD1 5EH (GB); CAMPBELL, David, Medical Research Council, MSI/WTB Complex Uni. of Dundee DD1 5EH (GB); GRAY, Alexander, MS I/WTB Complex Uni. of Dundee DD1 5EH (GB); DOWNES, Peter, Dundee DD1 5EH (GB); ALESSI, Dario, Med Resch Counc.Prot. Phospho.Unit, MSI/WTB Complex Uni. of Dundee DD1 5EH (GB)
(74) Representative: Wainwright, Jane Helen
(86) International application number: PCT/GB2001/003481
(87) International publication number: WO 2002/012276

(56) References cited:
- WO-A-99/57144
- DATABASE EMBL [Online] accession no. E16311, 9 August 1999 (1999-08-09) MAEKAWA K. ET AL.: "Human mRNA for BAS-GRIP protein." XP002191726 & JP 10 155491 A (SHIONOGI & CO LTD) 16 June 1998 (1998-06-16)
- DATABASE BIOSIS [Online] BIOSCIENCES INFORMATION SERVICE, PHILADELPHIA, PA, US; DOWLER, S. (1) ET AL: "Cloning and characterisation of novel phosphoinositide effector proteins." retrieved from STN Database accession no. PREV200200069430 XP002191728 & BIOCHEMICAL SOCIETY TRANSACTIONS, (2001) VOL. 29, NO. 3, PP. A77. PRINT. MEETING INFO.: 673RD BRISTOL MEETING OF THE BIOCHEMICAL SOCIETY BRISTOL, LONDON, UK APRIL 10-12, 2001,
- DATABASE BIOSIS [Online] BIOSCIENCES INFORMATION SERVICE, PHILADELPHIA, PA, US; KIMBER, W. A. (1) ET AL: "Interactions of the novel PtdIns(3,4)P2 binding proteins TAPP1 and TAPP2 with PDZ domain containing proteins." retrieved from STN Database accession no. PREV200200069437 XP002191727 & BIOCHEMICAL SOCIETY TRANSACTIONS, (2001) VOL. 29, NO. 3, PP. A79. PRINT. MEETING INFO.: 673RD BRISTOL MEETING OF THE BIOCHEMICAL SOCIETY BRISTOL, LONDON, UK APRIL 10-12, 2001,
- DATABASE EMBL [Online] 20 September 2000 (2000-09-20) DOWLER, S. J. ET AL.: "Identification of PH domains ...." Database accession no. AF286160 XP002191729
- DATABASE BIOSIS [Online] BIOSCIENCES INFORMATION SERVICE, PHILADELPHIA, PA, US; DOWLER, SIMON ET AL: "Identification of pleckstrin-homology-domain-containing proteins with novel phosphoinositide-binding specificities." retrieved from STN Database accession no. PREV200000532827 XP002191730 & BIOCHEMICAL JOURNAL, (1 OCTOBER, 2000) VOL. 351, NO. 1, PP. 19-31. PRINT.,
- MIKAMI, KOJI ET AL: "Isolation of an Arabidopsis thaliana cDNA encoding a pleckstrin homology domain protein, a putative homologue of human pleckstrin." JOURNAL OF EXPERIMENTAL BOTANY, (MAY, 1999) VOL. 50, NO. 334, PP. 729-730., XP001056981
- DATABASE GENBANK [Online] 16 April 1999 (1999-04-16) JACKSON, T. R.: "Identification of centaurin beta2 ....." Database accession no. AJ238248 XP002191731
- LEMMON MARK A ET AL: "Signal-dependent membrane targeting by pleckstrin homology (PH) domains." BIOCHEMICAL JOURNAL, vol. 350, no. 1, 2000, pages 1-18, XP002226376 ISSN: 0264-6021
- BALLA T ET AL: "How accurately can we image inositol lipids in living cells?" TRENDS IN PHARMACOLOGICAL SCIENCES, ELSEVIER TRENDS JOURNAL, CAMBRIDGE, GB, vol. 21, no. 7, 1 July 2000 (2000-07-01), pages 238-241, XP004209781 ISSN: 0165-6147
- DATABASE MEDLINE [Online] US NATIONAL LIBRARY OF MEDICINE (NLM), BETHESDA, MD, US; KIMBER WENDY A ET AL: "Evidence that the tandem-pleckstrin-homology-domain-containi ng protein TAPP1 interacts with Ptd(3,4)P2 and the multi-PDZ-domain- containing protein MUPP1 in vivo." retrieved from STN Database accession no. 2002076611 XP002191732 & BIOCHEMICAL JOURNAL, (2002 FEB 1) 361 (PT 3) 525-36.,

## Description

The present invention relates to polypeptides, polynucleotides and uses thereof, in particular to polypeptides comprising a PH (pleckstrin homology) domain.

Stimulation of cells with growth factors and insulin activates members of the phosphoinositide 3-kinase (PI 3-kinase) family which phosphorylate phosphatidylinositol 4,5-bisphosphate (PtdIns(4,5)P₂) at the D-3 position of the inositol ring to generate the lipid second messenger, PtdIns(3,4,5)P₃ [1]. A group of proteins has been identified that possess a certain type of pleckstrin homology (PH) domain which interacts specifically with PtdIns(3,4,5)P₃ and often its immediate breakdown product, PtdIns(3,4)P₂, also thought to be a signalling lipid (reviewed in Lemmon & Fergusson (2000) Biochem J 350, 1-18). These include the serine/threonine-specific protein kinases, PKB and PDK1 [2], Bruton's tyrosine kinase BTK [3], the adaptor proteins DAPP1 [4, 5] and Gab1 [6], as well as the ADP Ribosylation Factor (ARF) GTPase activating protein (GAP) centaurin-α [7] and the ARF guanine nucleotide exchange factor, Grp1 [8,9].

The molecular basis by which certain PH domains are able to interact with PtdIns(3,4,5)P₃ has not been established definitively. However, recent work indicates that six conserved residues that lie at the N-terminal region of the PH domain in a K-X-Sm-X₆₋₁₁-R/K-X-R-Hyd-Hyd motif (where X is any amino acid, Sm is a small amino acid and Hyd is a hydrophobic amino acid), appear to correlate with high affinity binding of PtdIns(3,4,5)P₃ [10]. To date, all of the specific PtdIns(3,4,5)P₃ binding proteins identified possess this Putative PtdIns(3,4,5)P₃ Binding Motif (PPBM) (Table 1).

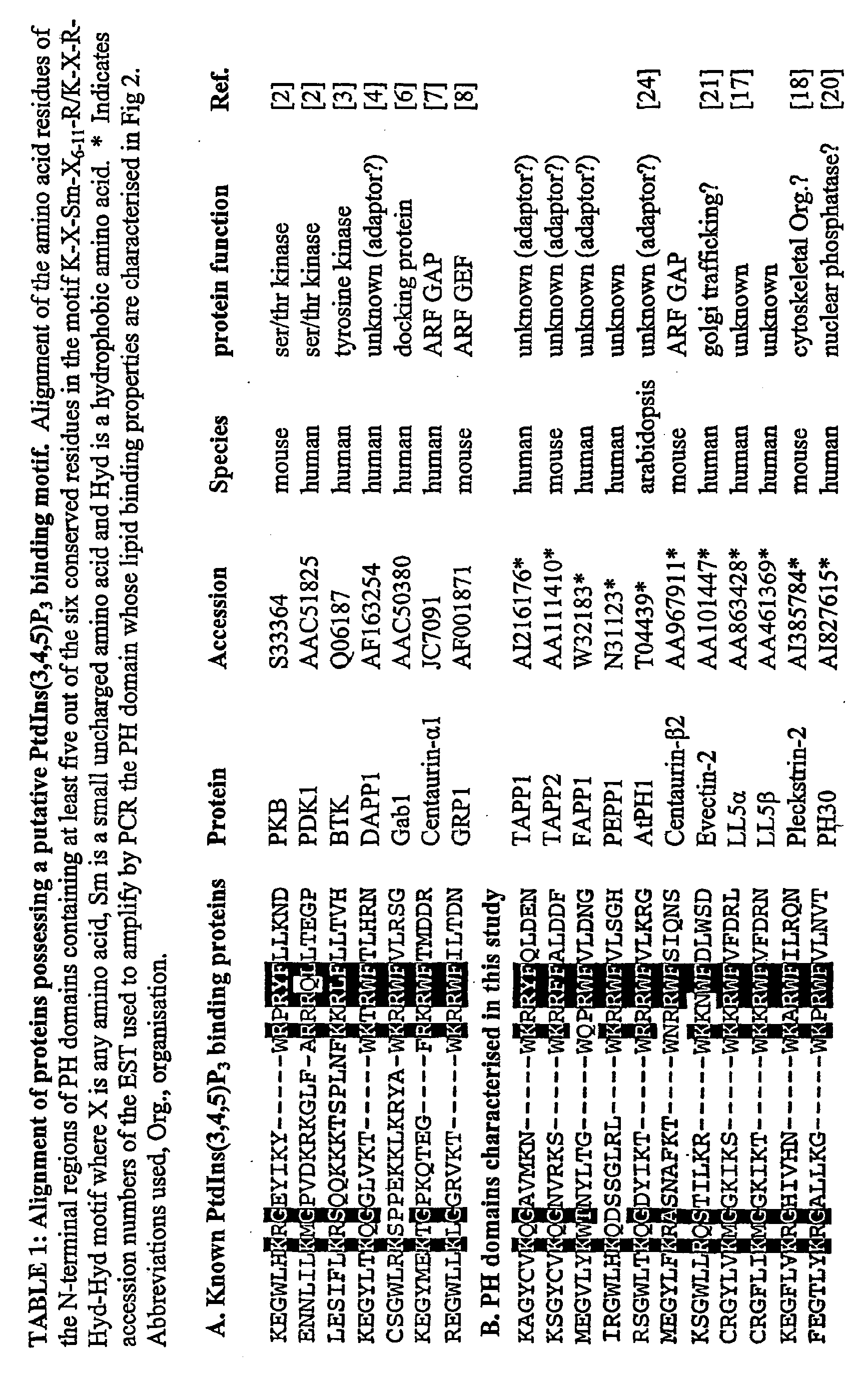

Mutation of certain of the conserved residues in the PPBM in some PH domains has been shown to abolish interaction with PtdIns(3,4,5)P₃ [10]. Significantly, recent structural studies of the PH domain of BTK bound to the head group of PtdIns(3,4,5)P₃ indicate that the basic amino acids in the PPBM may form direct interactions with the monoester phosphate groups of PtdIns(3,4,5)P₃ [11, 12].

We have identified and characterised proteins that bind specifically to a phosphoinositide other than PtdIns(3,4,5)P₃, in particular PtdIns3P, PtdIns3,4P₂ or PtdIns4P. The proteins each possess a PH domain which is considered to contain a PPBM and which binds the said phosphoinositide but not to PtdIns(3,4,5)P₃. These proteins may play important roles in triggering cellular processes that are regulated by other phosphoinositides. The proteins/PH domains may be useful in drug screening assays, in particular for compounds that may be useful in treating cancer, diabetes or stroke. They may also be useful in measuring concentrations and/or locations of the phosphoinositide lipids PtdIns3P, PtdIns3,4P₂ and PtdIns4P.

An aspect of the invention provides the use of a polypeptide capable of binding specifically to PtdIns(3,4)P₂, but not capable of binding to PtdIns(3,4,5)P₃, in as screening method for identifying a compound suitable for modulating signalling by PtdIns(3,4)P₂, wherein the polypeptide comprises a PH domain capable of binding to PtdIns(3,4)P₂ but not capable of binding to PtdIns(3,4,5)P₃ wherein the polypeptide may comprise the amino acid sequence 
MPYVDRQNRICGFLDIEEHENSGKFLRRYFILDTQANCLLWYMDNPQ
NLAMGAGAVGALQLTYISKVSIATPKQKPKTPFCFVINALSQRYFLQAND
QKDMKDWVEALNQASKITVPKGGGLPMTTEVLKSLAAPPALEKKPQVAYK
TEIIGGVVVHTPISQNGGDGQEGSEPGSHTILRRSQSYIPTSGCRASTGP
PLIKSGYCVKQGNVRKSWKRRFFALDDFTICYFKCEQDREPLRTIFFKDV
LKTHECLVKSGDLLMRDNLFEIITSSRTFYVQADSPEDMHSWIKEIGAAV
QALKCHP (partial human TAPP2 amino acid sequence)
 or 
MPYVDRQNRICGFLDIEDNENSGKFLRRYFILDTQANCLLWYMDNPQNLA
VGAGAVGSLQLTYISKVSIATPKQKPKTPFCFVINALSQRYFLQANDQKD
LKDWVEALNQASKITVPKAGTVPLATEVLKNLTAPPTLEKKPQVAYKTEI
IGGVVVQTPISQNGGDGQEGCEPGTHAFLRRSQSYIPTSGCRPSTGPPLI
KSGYCVKQGNVRKSWKRRFFALDDFTICYFKCEQDREPLRTIPLKDVLKT
HECLVKSGDLLMRDNLFEIITTSRTFYVQADSPEDMHSWIEGIGAAVQAL
KCHPREPSFSRSISLTRPGSSTLTSAPNSILSRRRPPAEEKRGLCKAPSV
ASSWQPWTPVPQAEEKPLSVEHAPEDSLFMPNPGESTATGVLASSRVRHR
SEPQHPKEKPFVFNLDDENIRTSDV (mouse TAPP2 amino acid sequence)
 or a fragment thereof, said fragment being capable of binding specifically to PtdIns(3,4)P₂ but not capable of binding to PtdIns(3,4,5)P₃, or the polypeptide may comprise a fusion of a said fragment thereof.

Polypeptides capable of binding to PtdIns(3,4)P₂, but not capable of binding to PtdIns(3,4,5)P₃ have not previously been identified as such. Screening methods making use of such a polypeptide have not previously been proposed.

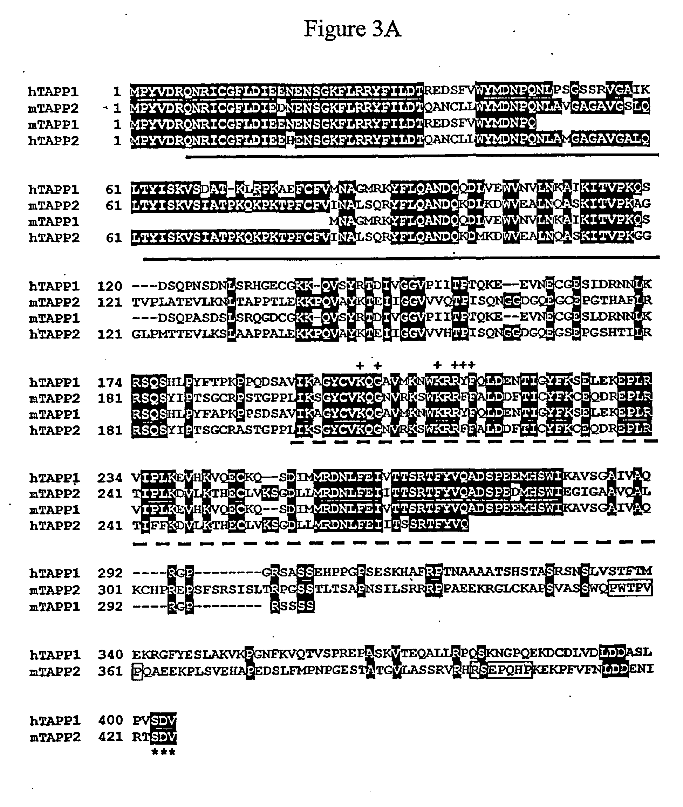

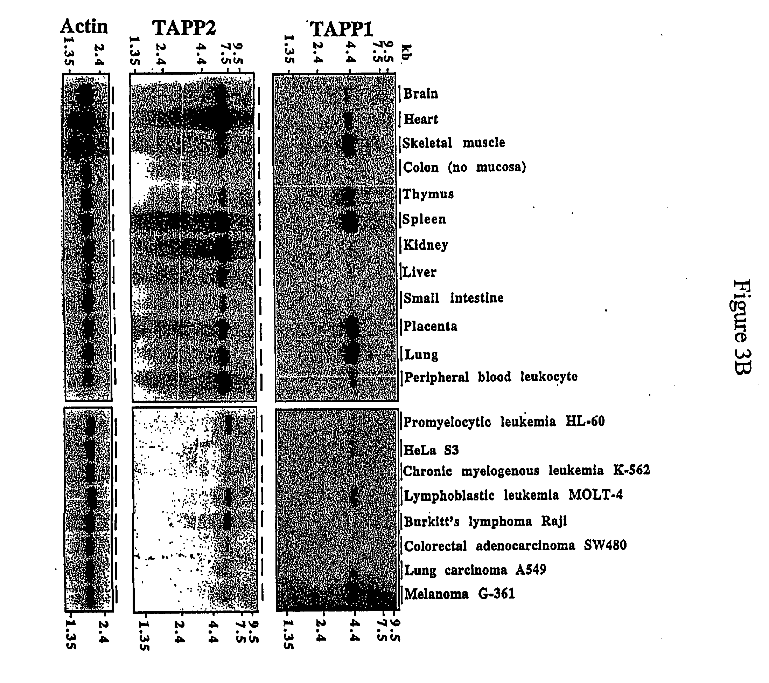

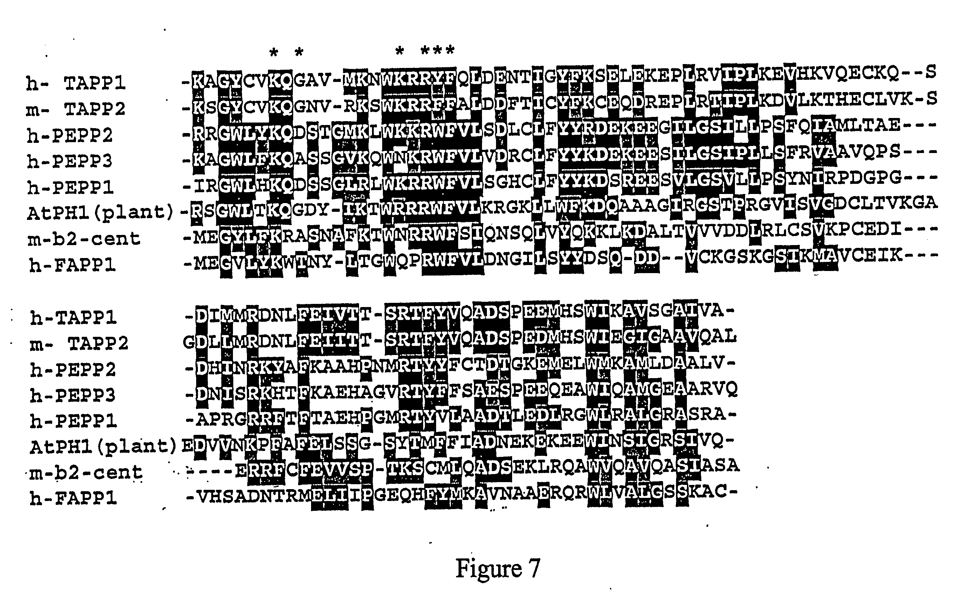

The term Plecktrin Homology (PH) domain is well known to those skilled in the art. These domains or ∼100 residues are found in over 70 other proteins and are predicted to fold into a similar 3-dimensional structures and may mediate protein-lipid, protein-protein interactions, or both (Gibson, T.J. et al (1994) Trends Biochem. Sci. 19, 349-353; Shaw, G. (1996) Bioessays 18, 35-46). Polypeptides with PH domains of determined tertiary sructure include plecktrin, spectrin, dynamin, and phospholipase C-γ. Although the percentage identity is poor between PH domains in general there are certain positions that show high levels of residue type conservation. The residues thought to be required for high affinity interaction with PtdIns(3,4,5)P₃ lie in the Putative Ptdbs(3;4,5)P₃ Binding Motif (PPBM) near the N-terminal end of the PH domain.. A single position (Tryptophan, position 280 of TAPP1 - see Figure 3), near the C-terminal end of the PH domain, shows complete identity throughout the domain family, as shown in Figure 7. Secondary structure predictions indicate that residues 450-530 of PDK1, for example, (positions 1-80) are likely to contain regions of β-sheet, while the residues between 531-550 (positions 80-100) are likely to form an extended α-helix, a prediction that is consistent with the known structures of other PH domains (Gibson, T.J. et al (1994) Trends Biochem. Sci. 19, 349-353; Shaw, G. (1996) Bioessays 18, 35-46; [24]).

The term Putative PtdIns(3,4,5)P3 Binding Motif (PPBM) is known to those skilled in the art. The motif is K-X-Sm-X₆₋₁₁-R/K-X-R-Hyd-Hyd motif (where X is any amino acid, Sm is a small, preferably uncharged, amino acid and Hyd is a hydrophobic amino acid) and lies near the N-terminal end of the PH domain. By a small amino acid is included glycine, alanine, threonine and serine. An aspartate or proline amino acid residue (for example) may alternatively be present at the position in the motif where a small amino acid is preferred. By a hydrophobic amino acid is meant tyrosine, leucine, isoleucine, tryptophan and phenylalanine. A glutamine amino acid residue (for example) may alternatively be present at the first position where a hydrophobic amino acid residue is preferred. A glutamine, asparagine or histidine amino acid residue may be present at a position where a lysine or arginine residue is preferred. It is strongly preferred that an acidic or hydrophobic residue is not present at a position where a lysine or arginine residue is preferred, or at the position in the motif where a small amino acid is preferred. It is preferred that the PH domain has at least five of the six specified residues of the PPBM. It is particularly preferred that the PH domain has both hydrophobic amino acids of the motif and/or the first lysine (K) residue of the motif. It is preferred that the PH domain also has a tryptophan residue at the position equivalent to position 280 of TAPP1, as discussed above.

It is preferred that the said polypeptide binds specifically to PtdIns(3,4)P₂, ie is able to bind to PtdIns(3,4)P₂, and is substantially unable to bind to other phosphoinositides, in particular PtdIns(3,4,5)P₃.

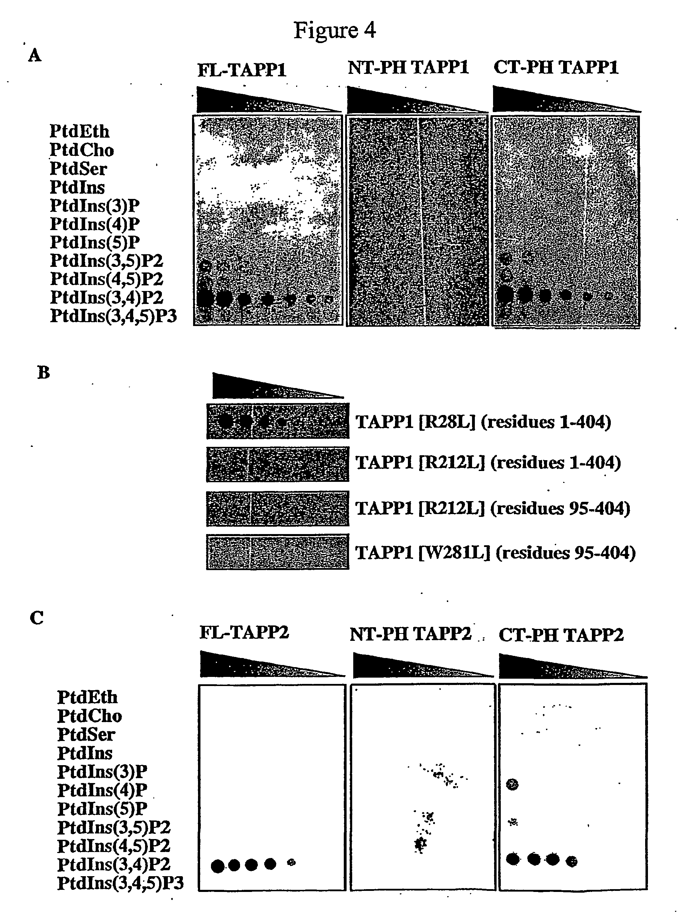

By "able to bind" is meant that binding of the said polypeptide to the said phosphoinositide can be detected using a surface plasmon resonance or protein lipid overlay technique as described in Example 1 and the legends to Table 2 and Figure 4. By "substantially unable to bind" is meant that binding of the said polypeptide to the said phosphoinositide is not detected, or is only weakly detected using a surface plasmon resonance or protein lipid overlay technique as described in Example 1 and the legends to Table 2 and Figure 4. It is preferred that the polypeptide binds to PtdIns(3,4)P₂, with at least two, preferably 3, 5, 10, 15, 20, 30 or 50-fold higher affinity than to other phosphoinositides, in particular PtdIns5P, PtdIns(4,5)P₂, PtdIns(3,4,5)P₃, PtdIns3P, PtdIns4P and PtdIns(3,5)P₂.

It is preferred that the binding of the said polypeptide to PtdIns(3,4)P₂, has an apparent K_{D} of less than about 2000 nM, 1000 nM or 500 nM, preferably less than about 400 or 350 nM, for example between about 350 nM and 10 nM, when measured using the method described in Example 1. It is preferred that the binding of the said polypeptide to other phosphoinositides, particularly PtdIns5P, PtdIns(4,5)P₂, PtdIns(3,4,5)P₃, PtdIns3P, PtdIns4P and PtdIns(3,5)P₂, has an apparent K_{D} of more than about 2000 nM, 1000 nM or 500 nM when measured using the method described in Example 1.

Examples of polypeptides that bind specifically to PtdIns(3,4)P₂ are considered to include mammalian (for example human and mouse) TAPP (for example TAPP1 and TAPP2), and fragments and fusions thereof that comprise the C-terminal PH domain, as discussed further below and in Example 1. Further examples are considered to include fragments, variants,

**Table 2: Apparent K_{d} of PEPP1, FAPP1 wild type and mutant TAPP1 and TAPP2 for binding to phosphoinositides as measured by surface plasmon resonance. The binding of the indicated GST-fusion proteins phosphoinositides incorporated into supported phosphatidylcholine monolayers was measured as described in the experimental section. The affinities (apparent K_{d}) were determined by global fitting of the association and dissociation curves to a 1:1 binding model. Abbreviations used, FL full length protein; NT-PH, N-terminal PH domain; CT-PH, C-terminal PH domain; NB, no binding detected; ND, not determined.**

| **Phosphoinositide** | **PEPP1** | **FAPP1** | **FL-TAPP1** | **FL-TAPP2** | **FL-TAPP1** **[R212L]** | **FL-TAPP1** **[R28L]** | **CT-PH** **TAPP1** | **CT-PH** **TAPP1** **[R212L]** | **NT-PH** **TAPP1** |
|---|---|---|---|---|---|---|---|---|---|
| **PtdIns 3*P*** | 325 nM | NB | NB | NB | ND | ND | ND | ND | ND |
| **PtdIns 4*P*** | NB | 20 nM | NB | NB | ND | ND | ND | ND | ND |
| **PtdIns 5*P*** | NB | NB | NB | NB | ND | ND | ND | ND | ND |
| **PtdIns(3,4)*P*₂** | NB | NB | 5 nM | 30 nM | NB | 28nM | 27 nM | NB | NB |
| **PtdIns(3,5)*P*₂** | NB | NB | NB | NB | ND | ND | ND | ND | ND |
| **PtdIns(4,5)P₂** | NB | NB | NB | NB | ND | ND | ND | ND | ND |

**Table 2: Relative affinities of PEPP1, FAPP1 wild type and mutant TAPP1 and TAPP2 for binding to phosphoinositides as measured by surface plasmon resonance. The binding of the indicated GST-fusion proteins phosphoinositides incorporated into supported phosphatidylcholine monolayers was measured as described in the experimental section. The apparent affinities were determined by global fitting of the association and dissociation curves to a 1:1 binding modeland were used to rank the binding affinity relative to that of TAPP1 to PtdIns(3,4)P₂ which was approximately 5 nM. Abbreviations used, FL full length protein; NT-PH, N-terminal PH domain; CT-PH, C-terminal PH domain; NB, no binding detected; ND, not determined.**

| **Phosphoinositide** | **DAPP1** | **PDK1** | **PEPP1** | **FAPP1** | **FL-TAPP1** | **FL-TAPP2** | **FL-TAPP1 [R212L]** | **FL-TAPP1 [R28L]** | **CT-PH TAPP1** | **CT-PH TAPP1 [R212L]** | **NT-PH TAPP1** |
|---|---|---|---|---|---|---|---|---|---|---|---|
| **PtdIns 3*P*** | NB | NB | 65 | NB | NB | NB | ND | ND | ND | ND | ND |
| **PtdIns 4*P*** | NB | NB | NB | 4 | NB | NB | ND | ND | ND | ND | ND |
| **PtdIns 5*P*** | NB | NB | NB | NB | NB | NB | ND | ND | ND | ND | ND |
| **PtdIns(3,4)*P*₂** | ND | ND | NB | NB | 1 | 5 | NB | 5.6 | 5.4 | 19.6 | NB |
| **PtdIns(3,4,5)*P*₃** | 0.6 | **12** | NB | NB | NB | NB | ND | ND | ND | ND | ND |
| **PtdIns(3,5)*P*₂** | NB | NB | NB | NB | NB | NB | ND | ND | ND | ND | ND |
| **PtdIns(4,5)*P*₂** | NB | NB | NB | NB | NB | NB | ND | ND | ND | ND | ND |

derivatives or fusions thereof, or fusions of fragments, variants or derivatives, that retain the said phosphoinositide binding properties, as discussed further below.

Examples of polypeptides that bind specifically to PtdIns4P are considered to include FAPP, for example mammalian FAPP (for example human or mouse FAPP) or *Xenopus* or Zebrafish FAPP, for example human FAPP1 or FAPP2 and fragments and fusions thereof that comprise a PH domain, as discussed further below and in Example 1. Further examples are considered to include fragments, variants, derivatives or fusions thereof, or fusions of fragments, variants or derivatives, that retain the said phosphoinositide binding properties, as discussed further below.

Examples of polypeptides that bind specifically to PtdIns3P are considered to include mammalian (for example human and mouse) PEPP (for example PEPP1, PEPP2 and PEPP3) and plant (for example *Arabidopsis*) AtPH1, and fragments and fusions thereof that comprise a PH domain, as discussed further below and in Example 1. Further examples are considered to include fragments, variants, derivatives or fusions thereof, or fusions of fragments, variants or derivatives, that retain the said phosphoinositide binding properties, as discussed further below.

Examples of polypeptides that bind specifically to PtdIns(3,5)P₂ are considered to include centaurin-β2 (for example mammalian, for example human or mouse, or *Drosophila* or *C*. *elegans*), and fragments and fusions thereof that comprise the C-terminal PH domain, as discussed further below and in Example 1. Further examples are considered to include fragments, variants, derivatives or fusions thereof, or fusions of fragments, variants or derivatives, that retain the said phosphoinositide binding properties, as discussed further below.

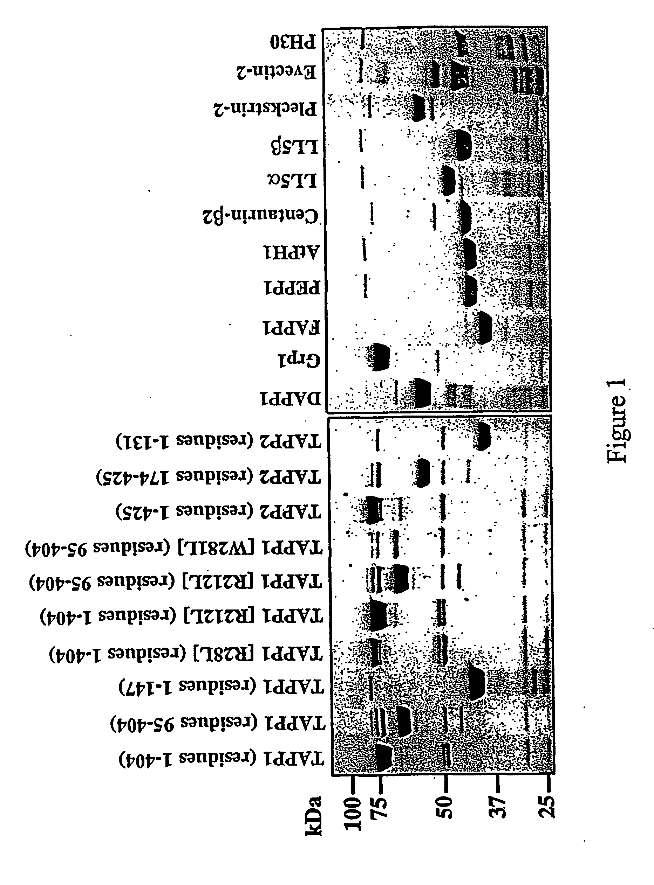

Fragments of TAPP, PEPP, FAPP, AtPH1 and centaurin-β2 (for example fragments comprising PH domains) are discussed in Example 1, for example in the section relation to cloning of PH domains and in Figure 1.

Suitably, the screaning method of the invention comprises the steps of (1) exposing the said polypeptide to PtdIns(3,4)P₂, in the presence of a test compound; (2) determining whether the test compound modulates binding of PtdIns(3,4)P₂ to the said polypeptide; and (3) selecting a compound which modulates binding of PtdIns(3,4)P₂ to the said polypeptide.

Further suitable methods are described in relation to the following aspects of the invention.

A further aspect of the invention provides a method of identifying a compound that modulates the phospholipid binding activity of a polypeptide capable of binding to PtdIns(3,4)P₂, but not capable of binding to PtdIns(3,4,5)P₃, according to the preceding aspect the method comprising contacting a compound with the said polypeptide and determining whether the phospholipid binding activity of the said polypeptide is changed in the presence of the compound from that in the absence of said compound.

Preferences and examples are as indicated in relation to the preceding aspect of the invention.

The binding of polypeptides comprising a PH domain having the required properties to phospholipids is described in Example 1. It is preferred that modulation of the binding to PtdIns(3,4)P₂, is measured. Methods of detecting binding of the said polypeptide to phospholipids are described in Example 1 and include a protein-lipid overlay assay in which the lipid is spotted onto a support, for example Hybond-C extra membrane, and protein bound to the support by virtue of interaction with the lipid is detected, for example using an antibody-based method, as well know to those skilled in the art. A surface plasmon resonance assay, for example as described in Example 1 or in Plant et al (1995) Analyt Biochem 226(2), 342-348, may alternatively be used. Methods may make use of a said polypeptide comprising a PH domain, or fragment thereof, that is labelled, for example with a radioactive or fluorescent labeL Suitable methods may also be described in, for example, Shirai et al (1998) Biochim Biophys Acta 1402(3), 292-302 (use of an affinity column prepared using phosphatidylinositol analogues) and Rao et al (1999) J Biol Chem 274, 37893-37900 (use of avidin-coated beads bound to biotinylated phosphatidylinositol analogues).

It will be understood that it will be desirable to identify compounds that may modulate the activity of the polypeptide *in vivo*. Thus it will be understood that reagents (including any fragment of the polypeptide) and conditions used in the method may be chosen such that the interactions between the said polypeptide and a phosphoinositide, for example PtdIns(3,4)P₂, are substantially the same as between the wild-type, preferably human polypeptide (for example TAPP) and the phosphoinositide or interacting polypeptide *in vivo*.

In one embodiment, the compound decreases the relevant binding activity of said polypeptide. For example, the compound may bind substantially reversibly or substantially irreversibly to the relevant binding site of said polypeptide. In a further example, the compound may bind to a portion of said polypeptide that is not the binding site so as to interfere with the binding of the said phosphoinositide-binding polypeptide to the phosphoinositide. In a still further example, the compound may bind to a portion of said polypeptide so as to decrease said polypeptide's binding activity by an allosteric effect. This allosteric effect may be an allosteric effect that is involved in the natural regulation of the said polypeptide's activity.

The compound may, for example, change the configuration of the polypeptide so that it is substantially unable to bind to the particular phosphoinositide. The compound may be capable of affecting the intracellular location of the polypeptide; for example, it may inhibit or promote the translocation of the polypeptide to a membrane, for example the plasma membrane or golgi, vacuole, lysosome or endosome membrane. Possible association with cellular membranes of polypeptides comprising a PH domain with the required phosphoinositide binding properties are discussed further in Example 1. The compound may modulate any interaction of the polypeptide with further identical polypeptide molecules (ie self-association, for example dimerisation). It will be appreciated that a compound that, for example, is capable of modulating the phosphorylation or other post-translational modification of the polypeptide may thereby, for example, modulate the ability of the polypeptide to bind to a phosphoinositide. A compound that is capable of modulating the ability of the polypeptide to bind to a phosphoinositide may thereby modulate the intracellular location of the polypeptide molecule and/or modulate any post-translational modification, for example phosphorylation, of the polypeptide.

In a further embodiment, the compound increases the binding activity of said polypeptide. For example, the compound may bind to a portion of said polypeptide that is not the relevant binding site so as to aid the binding of the said polypeptide to the phospholipid, as appropriate. In a still further example, the compound may bind to a portion of said polypeptide so as to increase said polypeptide's binding activity by an allosteric effect. This allosteric effect may be an allosteric effect that is involved in the natural regulation of the said polypeptide's activity.

An example of a compound that may be capable of inhibiting binding of a phosphoinositide to a said polypeptide is InsP₄, the head group of PtdIns(3,4,5)P₃. Ins(1,3,4)P₃, the head group of PtdIns(1,3,4)P₃, may be capable of inhibiting binding of PtdIns(3,4)P₂ to TAPP.

Conveniently, the appropriate methods make use of the methods described in Example 1 for detecting and/or quantifying the interaction between a polypeptide and a phospholipid, for example a protein-lipid overlay or surface plasmon resonance method, as discussed above. It is preferred that a GST-tagged fusion of the polypeptide of the invention or a fragment therof is used. Methods in which radioactively or fluorescently labelled lipids are used may also be useful.

Methods of detecting protein-protein interactions are well known to those skilled in the art. Suitable methods include yeast two-hybrid interactions, co-purification, ELISA, co-immunoprecipitation methods and cellular response assays. Cellular response assays may be carried out in a variety of cell types, for example in adipocytes or adipocyte cell lines, in a skeletal muscle cell line (such as the L6 myotubule cell line), liver cells or liver cell lines or cancer cells or cancer cell lines.

Platelets may be preferred when the polypeptide is TAPP. NIH Swiss mouse embryo cells NIH/3T3 (available from the American Type Culture Collection (ATCC) of Rockville, MD, USA (ATCC) as CRL 1658) and human embryonic kidney 293 cells (also available from the ATCC) are examples of cell lines that may be used when investigating the effect of hydrogen peroxide or other cellular stress treatment.

The method may be performed *in vitro,* either in intact cells or tissues, with broken cell or tissue preparations or at least partially purified components. Alternatively, they may be performed *in vivo*. The cells tissues or organisms in/on which the method is performed may be transgenic. In particular they may be transgenic for the said polypeptide capable of binding a specific phosphoinositide.

Other methods of detecting polypeptide/polypeptide interactions include ultrafiltration with ion spray mass spectroscopy/HPLC methods or other physical and analytical methods. Fluorescence Energy Resonance Transfer (FRET) methods, for example, well known to those skilled in the art, may be used, in which binding of two fluorescent labelled entities may be measured by measuring the interaction of the fluorescent labels when in close proximity to each other.

This may be done in a whole cell system or using purified or partially purified components. Similarly, expression of a protein encoded by an RNA transcribed from a promoter regulated by the polypeptide may be measured. The protein may be one that is physiologically regulated by the polypeptide or may be a "reporter" protein, as well known to those skilled in the art (ie a recombinant construct may be used). A reporter protein may be one whose activity may easily be assayed, for example (β-galactosidase, chloramphenicol acetyltransferase or luciferase (see, for example, Tan *et al*(1996)). In a further example, the reporter gene may be fatal to the cells, or alternatively may allow cells to survive under otherwise fatal conditions. Cell survival can then be measured, for example using colorimetric assays for mitochondrial activity, such as reduction of WST-1 (Boehringer). WST-1 is a formosan dye that undergoes a change in absorbance on receiving electrons via succinate dehydrogenase.

Promoters whose activity may be regulated by a signalling pathway in which the polypeptide may be involved may be identified using microarray technology, as known to those skilled in the art, in which the expression of multiple genes may be examined simultaneously, for example in stimulated and unstimulated cells expressing the wild-type polypeptide or a dominant negative mutation. Differences in expression patterns between the different cells/activation states indicate genes/promoters which the polypeptide may regulate. An example of a dominant negative mutant of TAPP is a fragment of TAPP comrpising the C-terminal PH domain, but not the N-terminal PH domain and/or putative SH3 binding domain (TAPP2) and/or PDZ binding sequence. Thus, transcription of these genes may be assessed or the promoter for such a gene may be used in a reporter construct as described above.

Insulin exerts important effects on gene expression in multiple tissues (O'Brien, R. M. & Granner, D. K (1996) Physiol. Rev. 76, 1109-1161). In the liver, insulin suppresses the expression of a number of genes which contain a conserved insulin response sequence (IRS)¹ (CAAAAC/TAA), including insulin-like growth factor binding protein-1 (IGFBP-1), apolipoprotein CIII (apoCIII), phosphoenol-pyruvate carboxykinase (PEPCK) and glucose-6 phosphatase (G6Pase) (Goswami, R et al (1994) Endocrinol. 134, 2531-2539; Suwanickul, A et al (1993) J. Biol Chem. 268, 17063-17068; Li, W. W et al (1995) J. Clin. Invest 96, 2601-2605; O'Brien, R. M et al (1990) Science 249, 533-537; Streeper, R. S et al (1997) J. Biol Chem. 272, 11698-11701). Thus, transcription of these genes may be assessed or promoters from these genes may be used in a reporter construct as described above, for example when the polypeptide is TAPP. Microarray technology may be used in assessing transcription of genes or reporter constructs, as known to those skilled in the art.

The transcription of a gene indicated above (or any other that is regulated by cellular stress, a growth factor or insulin signalling) may be measured by measurement of changes in the enzymatic or other activity of the said gene product, for example in a cell. Suitable methods will be well known to those skilled in the art.

It will be necessary to perform various control assays, as known to those skilled in the art, in order to determine that a compound is affecting signalling *via* the said phosphoinositide-binding polypeptide, rather than having some other effect on processes leading to whatever measurement is made. For example, it may be necessary to determine what effect the compound being tested has on the activity rather than the activation of a polypeptide, for example a protein kinase, that may be acting downstream (in the signalling pathway) of the said phosphoinositide-binding polypeptide but upstream of the effect being measured.

**Table 3. Tissue origin ofESTs encoding TAPP1, TAPP2, PEPP1, and FAPP1. ESTs which we have sequenced have their I.M.A.G.E. Consortium Clone ID in parentheses.**

| **Protein** | **Species** | **Tissue** | **NCBI Accession (I.M.A.G.E. Clone ID)** |
|---|---|---|---|
| **TAPP1** | Human | Parathyroid tumour | W56032, W63712 (326517) |
| | | Foetal heart | AA054961 |
| | | Lung | AI191308, AI216176 (1884429) |
| | | Colon | AI709038 |
| | | Kidney | AA875839, AI343801 |
| | | Skeletal muscle | AA211648 |
| | | Melanocyte | N31136 |
| | | Testes | AI343801 |
| | | Olfactory epithelium | AL046495 |
| | | Germinal centre B cell | AA740729 (1286305) |
| | | Foetal Liver | H78048, H90955 |
| | | Uterus | AA150283 (491669) |
| | | Placenta | R62858 |
| | | Testis | AA429617 |
| | | Foetal liver | R91752 |
| | Mouse | Thymus | AA762924 |
| | | Kidney | AI987596 (2158944) |
| | | Embryo | AA388896 (569145) |
| | Zebrafish | Pooled | AI497344, AI878142 |
| | | Fin regenerates | AW595189 |
| **TAPP2** | Human | Germinal centre B cells | AA721234 (1300983) |
| | | Foetal lung | AI185428 (1742690) |
| | | Pooled tumours | AA975814 (1589519) |
| | | Brain | AA985353, AW408638 |
| | Mouse | Embryo | AA111410 (557355) |
| | | Thymus | AA118260, AI447504 (574391) |
| | | Myotubes | AI592480, AI591454 (1162924) |
| | Zebrafish | Pooled | AI497344, AI878142 |
| | chicken | Bursa of Fabricius | AJ393764, AJ395418,AJ393899 |
| **FAPP1** | Human | Multiple sclerosis | N79274 (287618) |
| | | Germinal centre B cells | AA481205 (815143), AA481224 (815169), AI221252 (1842552) |
| | | Bowel | BE136879 |
| | | Testis | AA431220 |
| | | Lung carcinoid | AW340998, AW341035 |
| | | Foetal heart | W73345 |
| | | Colon tumour | AI337400 |
| | | Pancreatic islet | W52895 (338749) |
| | | Aorta endothelial | AA301959 |
| | | Germ cell tumour | AI341371 |
| | | Pooled | AI246428, AI242688, AA453702 (813820), AA724575 |
| | | Parathyroid tumour | (1327281) |
| | Mouse | Uterus | W32183 (321321) |
| | | Total fetus | AI161122 (1721404) |
| | | Embryo | AA463817 (796517) |
| | | Macrophages | AA681116 (1134498) |
| | | Tumour | AA867335 (1293870) |
| | | Spleen | AW412246 (2812588) |
| | Rat | Total foetus | AA184412 |
| | | Heart | AA048334 (477463) |
| | Xenopus | Ovary | AA419963 (847595) |
| | Zebrafish | Pooled | AI177017 |
| | | Unfertilised egg | AI071963 |
| | | Pooled | AW644282 |
| | | | A174299 |
| **PEPP1** | Human | Melanocytes | N49341 (272085), N31123 (265349) |
| | | Melanoma | AL135424 (DEFZp762M2115), AL135565 |
| **PEPP2** | Human | Kidney | A1808805 |
| | | Brain | AA232124 |
| | | Foetal liver and spleen | W91917 |
| | | Germ cell line | AI638629 |

The following methods of isolating a nucleic acid encoding a polypeptide are given for purposes of illustration and are not considered to be exhaustive.

The polypeptide may be cleaved, for example using trypsin, cyanogen bromide, V8 protease formic acid, or another specific cleavage reagent. The digest may be chromatographed on a Vydac C18 column or subjected to SDS-PAGE to resolve the peptides. The N-terminal sequence of the peptides may then be determined using standard methods.

The sequences are used to isolate a nucleic acid encoding the peptide sequences using standard PCR-based strategies. Degenerate oligonucleotide mixtures, each comprising a mixture of all possible sequences encoding a part of the peptide sequences, are designed and used as PCR primers or probes for hybridisation analysis of PCR products after Southern blotting. mRNA prepared from cells in which the polypeptide may be expressed is used as the template for reverse transcriptase, to prepare cDNA, which is then used as the template for the PCR reactions.

Positive PCR fragments are subcloned and used to screen cDNA libraries to isolate a full length clone for the polypeptide.

Alternatively, the sequences of initial subcloned PCR fragments may be determined, and the sequence may then be extended by known PCR-based techniques to obtain a full length sequence.

Alternatively, the initial PCR sequence may be used to screen electronic databases of expressed sequence tags (ESTs) or other known sequences. By this means, related sequences may be identified which may be useful in isolating a full length sequence using the two approaches described above.

Sequences are determined using the Sanger dideoxy method. The encoded amino acid sequences may be deduced by routine methods.

Techniques used are essentially as described in Sambrook et al (1989) Molecular cloning, a laboratory manual, Cold Spring Harbor Laboratory Press, Cold Spring Harbor, New York.

Alternatively, antibodies may be raised against the polypeptide.

The antibodies are used to screen a λgt11 expression library made from cDNA copied from mRNA from cells in which the polypeptide may be expressed.

Positive clones are identified and the insert sequenced by the Sanger method as mentioned above. The encoded amino acid sequence may be deduced by routine methods.

It will be appreciated that it may be desirable to express the polypeptide encoded by the isolated nucleic acid in order to determine that the polypeptide has the expected properties, for example expected ability to bind to a said phosphoinositide-binding polypeptide, for example TAPP.

It will be appreciated that the above methods may be performed within a cell, for example using the yeast two hybrid system as is well known in the art. It will further be appreciated that a transgenic animal in which a said phosphoinositide-binding polypeptide gene is altered and/or a recombinant said phosphoinositide-binding polypeptide gene is present, for example a rodent, in particular a mouse, may be useful in, for example, identifying polypeptides that interact with the said phosphoinositide-binding polypeptide.

The interacting polypeptide may be a receptor molecule, for example a receptor molecule present in/on the surface of a cell, for example a platelet, adipocyte, muscle or skin cell. The receptor molecule may be a transmembrane polypeptide or complex, as know to those skilled in the art. It will be appreciated that known receptors, for example platelet integrin receptors, are not included.

It will be appreciated that screening assays which are capable of high throughput operation will be particularly preferred. Examples may include cell based assays and protein-protein binding assays. An SPA-based (Scintillation Proximity Assay; Amersham International) system may be used. For example, beads comprising scintillant and an interacting polypeptide (which term it will be appreciated includes a polypeptide which capable of interacting with a polypeptide of the invention or fragment thereof and is a fragment of a polypeptide, for example a naturally occuring polypeptide, that is also capable of interacting with a polypeptide of the invention or fragment thereof) may be prepared. The beads may be mixed with a sample comprising, for example, the phosphatidylinositol-binding polypeptide into which a radioactive label has been incorporated and with the test compound. Conveniently this is done in a 96-well format. The plate is then counted using a suitable scintillation counter, using known parameters for the particular radioactive label in an SPA assay. Only the radioactive label that is in proximity to the scintillant, ie only that bound to the phosphoinositide-binding polypeptide that is bound to the interacting polypeptide anchored on the beads, is detected. Variants of such an assay, for example in which the interacting polypeptide is immobilised on the scintillant beads *via* binding to an antibody or antibody fragment, may also be used. Phosphoinositides or analogues thereof may be immobilised on SPA beads, for example using methods as described in Shirai et al (1998) Biochim Biophys Acta 1402(3), 292-302 or in Rao et al (1999) J Biol Chem 274, 37893-37900.

It will be appreciated that the screening assays of the invention are useful for identifying compounds which may be useful in the treatment of diabetes, defects of glycogen metabolism, cancer (including melanoma), inflammatory conditions, ischaemic conditions, for example stroke, thrombosis or tendency to thrombosis (for example useful as an antithrombotic agent).

The compound may be a drug-like compound or lead compound for the development of a drug-like compound for each of the above methods of identifying a compound. It will be appreciated that the said methods may be useful as screening assays in the development of pharmaceutical compounds or drugs, as well known to those skilled in the art.

The term "drug-like compound" is well known to those skilled in the art, and may include the meaning of a compound that has characteristics that may make it suitable for use in medicine, for example as the active ingredient in a medicament. Thus, for example, a drug-like compound may be a molecule that may be synthesised by the techniques of organic chemistry, less preferably by techniques of molecular biology or biochemistry, and is preferably a small molecule, which may be of less than 5000 daltons molecular weight. A drug-like compound may additionally exhibit features of selective interaction with a particular protein or proteins and be bioavailable and/or able to penetrate cellular membranes, but it will be appreciated that these features are not essential.

The term "lead compound" is similarly well known to those skilled in the art, and may include the meaning that the compound, whilst not itself suitable for use as a drug (for example because it is only weakly potent against its intended target, non-selective in its action, unstable, difficult to synthesise or has poor bioavailability) may provide a starting-point for the design of other compounds that may have more desirable characteristics.

It will be appreciated that the compound may be a polypeptide that is capable of competing with the polypeptide of the invention for binding to the interacting polypeptide. Thus, it will be appreciated that a screening method as described above may be useful in identifying polypeptides that may also interact with the interacting polypeptide, for example a receptor molecule.

It will be understood that it will be desirable to identify compounds that may modulate the activity of the polypeptide(s) *in vivo*. Thus it will be understood that reagents and conditions used in the method may be chosen such that the interactions between the said polypeptide and the interacting polypeptide are substantially the same as between the said polypeptide or a fragment therof and a naturally occuring interacting polypeptide *in vivo*.

The "drug-like compounds" and "lead compounds" identified in the screening assays of the invention are suitably screened in further screens to determine their potential usefulness in treating diabetes, defects of glycogen metabolism, cancer (including melanoma), inflammatory conditions, ischaemic conditions, for example stroke, or thrombosis or tendency to thrombosis. Additional screens which may be carried out include determining the effect of the compounds on blood glucose levels, tumour growth or blood clotting tendency/time, as appropriate. This may typically be done in rodents.

A further aspect of the invention is a kit of parts useful in carrying out a method, for example a detecting and/or quantifying method, of the invention. Such a kit will comprise a said phosphoinositide-binding polypeptide of the preceding aspects of the invention (or a suitable fragment, thereof).

Preferences for the phosphoinositide-binding polypeptide are as set out in relation to earlier aspects of the invention. It is particularly preferred that the phosphoinositide-binding polypeptide (or fragment thereof) is a naturally occurring polypeptide or naturally occurring allelic variant thereof.

Conveniently, the said phosphoinositide-binding polypeptide or fragment, used in the methods is one which is produced by recombinant DNA technology.

It will be appreciated that it may be desirable to carry out a method of the invention, for example a compound screening method of the invention, in the presence of the phosphoinositide to which the said phosphoinositide-binding protein is capable of binding. Expression of a constitutively active phosphoinositide (PI) kinase may be desirable in relation to a cell-based assay, in order to elevate the level of the appropriate phosphoinositide in the cell. For example, (over)expression of a Class 1A PI3 kinase may be useful in relation to TAPP, as it may increase the level of PtdIns(3,4,5)P₃ and thereby the level of PtdIns(3,4)P₂. Overexpression of a Class II PI3 kinase may be useful in relation to PEPP or AtPH1, as it may increase the level of PtdIns3P, whilst overexpression of a PI4 kinase may be useful in relation to FAPP, as it may increase the level of PtdIns4P. Overexpression of Fab1[38, 39] may be useful in, relation to centaurin-β2, as it may increase the level of Ptd(3,5)P_{2.}

It will be appreciated that by "suitable" we mean that the said components in the method are those that have interactions or activities which are substantially the same as those of the said phosphoinositide-binding polypeptide or as the case may be but which may be more convenient to use in an assay. For example, fusions of the said phosphoinositide-binding polypeptide are particularly useful since said fusion may contain a moiety which may allow the fusion to be purified readily.

A further aspect of the invention provides a method of detecting and/or quantifying PtdIns(3,4)P₂, in a sample wherein the sample is exposed to a polypeptide capable of binding to PtdIns(3,4)P2, but not capable of binding to PtdIns(3,4,5)P₃ according to the preceding aspects of the invention and the binding of the said polypeptide to any PtdIns(3,4)P₂ present is detected. Preferences for the said polypeptide are as indicated in relation to the preceding and following aspects of the invention. Methods of detecting binding of the PtdIns(3,4)P₂ to the said polypeptide are discussed above and in Examples 1 and 3. The polypeptides may be used to determine the location of the PtdIns(3,4)P₂ using *in situ* techniques, as well known to those skilled in the art. The cells may be living cells, or fixed using conventional methods, for example formaldehyde fixing. Particularly in relation to investigating living cells, it is preferred that the said polypeptide comprises a chromophore, for example a green fluorescent protein moiety (GFP; including mutated GFPs, for example blue, yellow or cyan fluorescent proteins), for example as a fusion protein which is expressed in the cell, as well known to those skilled in the art. GFPs are produced naturally by *Aequorea victoria* but, as is well known in the art and described, for example, in Mitra et al (1996) Gene 173, 13-17; Cubitt et al (1995) Trends Biochem. Sci. 20, 448-454; Miyawaki et al (1997) Nature 388, 882-887; Patterson et al (1997) Biophys J. 73, 2782-2690; Heim & Tsien (1996) Curr. Biol. 6, 178-182; and Heim et al (1995) Nature 373, 663-664, mutant GFPs are available which have modified spectral characteristics. Certain GFPs and mutant GFPs are available from Clontech Laboratories UK Ltd, Wade Road, Basingstoke, Hants RG24 8NE.

The methods may be used in assays for detecting or quantifying (measuring) enzyme activity, for example lipid phosphatases or inositol lipid kinases, for example Fablp (a stress-activated phosphatidylinositol 3-phosphate 5-kinase), which converts PtdIns3P to PtdIns(3,5)P_{2.} Thus, a PH domain which binds to PtdIns3P (for example the PH domain of PEPP1 or AtPH1) may be used to monitor the level of PtdIns3P and thereby Fablp activity. This is discussed further in Example 3. Such a lipid kinase/phosphatase assay may be performed *in vitro* (for example using techniques described above and in Examples 1 and 3) or *in vivo,* for example in cells, using techniques as described above. The methods may be used in identifying modulators (for example inhibitors or activators) of the enzyme activity, as will be apparent to those skilled in the art. Thus, the invention provides a method for identifying a modulator of a lipid kinase or phosphatase activity wherein the lipid kinase or phosphatase activity is measured in the presence (and preferably also in the absence, or in the presence of more than one concentration) of the compound using such a method. The invention further provides a kit of parts useful in carrying out such a detection/quantification or screening method. Suitable components for such a kit include reagents and enzymes of the types mentioned in Example 3, for example a PH domain of the invention and a phosphoinositide which binds to the said PH domain or a lipid which is converted into a phosphoinositide which binds to the said PH domain by an enzyme, for example lipid kinase or phosphatase.

A further aspect of the invention provides a composition comprising a polypeptide capable of binding specifically to PtdIns(3,4)P_{2,} but not capable of binding to PtdIns(3,4,5)P₃, wherein the polypeptide comprises a PH domain capable of binding to PfdIns(3,4)P₂ but not capable of binding to PtdIns(3,4,5)P₃ wherein the polypeptide comprises the amino acid sequence 
MPYVDRQNRICGFLDIEEHENSGKFLRRYFILDTQANCLLWYMDNPQ
NLAMGAGAVGALQLTYISKVSIATPKQKPKTPFCFVINALSQRYFLQAND
QKDMKDWVEALNQASKITVPKGGGLPMTTEVLKSLAAPPALEKKPQVAYK
TEIIGGVVVHTPISQNGGDGQEGSEPGSHTILRRSQSYIPTSGCRASTGP
PLIKSGYCVKQGNVRKSWKRRFFALDDFTICYFKCEQDREPLRTIFFKDV
LKTHECLVKSGDLLMRDNLFEIITSSRTFYVQADSPEDMHSWIKEIGAAV
QALKCHP (partial human TAPP2 amino acid sequence)
 or 
MPYVDRQNRICGFLDIEDNENSGKFLRRYFILDTQANCLLWYMDNPQNLA
VGAGAVGSLQLTYISKVSIATPKQKPKTPFCFVINALSQRYFLQANDQKD
LKDWVEALNQASKITVPKAGTVPLATEVLKNLTAPPTLEKKPQVAYKTEI
IGGVVVQTPISQNGGDGQEGCEPGTHAFLRRSQSYIPTSGCRPSTGPPLI
KSGYCVKQGNVRKSWKRRFFALDDFTICYFKCEQDREPLRTIPLKDVLKT
HECLVKSGDLLMRDNLFEIITTSRTFYVQADSPEDMHSWIEGIGAAVQAL
KCHPREPSFSRSISLTRPGSSTLTSAPNSILSRRRPPAEEKRGLCKAPSV
ASSWQPWTPVPQAEEKPLSVEHAPEDSLFMPNPGESTATGVLASSRVRHR
SEPQHPKEKPFVFNLDDENIRTSDV (mouse TAPP2 amino acid sequence)
 or a fragment thereof capable of binding specifically to PtdIns(3,4)P₂ but not capable of binding to PtdIns(3,4,5)P₃ or the polypeptide may comprise a fusion of a said fragment thereof, wherein the composition comprises a protein content that consists of at least 50% by weight of said polypeptide.

Further preferences for the said phosphoinositide-binding polypeptide of the invention, for example concerning phosphoinositide binding specificity, are as indicated above in relation to the phosphoinositide-binding polypeptides in relation to the screening/use aspacts of the invention.

It is not considered that a PI 4-kinase polypeptide (or recombinant polypeptide comprising a PH domain therefrom) as described in Stevenson et al (1998) J Biol Chem 273, 22761-22767 is capable of binding to PtdIns(3,4)P₂, PtdIns3P, PtdIns4P and/or PtdIns(3,5)P₂)but is not capable of binding to PtdIns(3,4,5)P_{3.} For the avoidance of doubt, the polypeptides described in Stevenson *et al* (1998) (ie PI 4 kinases and PH domains thereof from *Arabidopsis,* carrot, yeast STT4, rat, human PIK4Kα and bovine brain PI4K200 are excluded from the polypeptides of the invention These polypeptides are further not considered to comprise a PH domain which has at least five of the six residues of a Putative PtdIns(3,4,5)P₃ Binding Motif (PPBM).

It is not considered that PLCδ₁ is capable of binding to PtdIns(3,4)P₂, PtdIns3P, PtdIns4P and/or PtdIns(3,5)P₂) but is not capable of binding to PtdIns(3,4,5)P₃. For the avoidance of doubt, PLCδ₁ is excluded from the polypeptides of the invention.

A polypeptide of the invention may be useful in accordance with the uses or screens of the preceding aspects of the invention, as indicated above. Examples of polypeptides of the invention include TAPP2 fragments thereof, or fusions of fragments, for example a fragment comprising a phosphoinositide-binding PH domain. The said fragment, or fusion retains the phosphoinositide binding properties of the polypeptide of the invention from which it is derived/derivable, as discussed further below.

An embodiment of the preceding aspect of the invention provides a composition comprising a polypeptide comprising the amino acid sequence 
MPYVDRQNRICGFLDIEEHENSGKFLRRYFILDTQANCLLWYMDNPQ
NLAMGAGAVGALQLTYISKVSIATPKQKPKTPFCFVINALSQRYFLQAND
QKDMKDWVEALNQASKITVPKGGGLPMTTEVLKSLAAPPALEKKPQVAYK
TEIIGGVVVHTPISQNGGDGQEGSEPGSHTILRRSQSYIPTSGCRASTGP
PLIKSGYCVKQGNVRKSWKRRFFALDDFTICYFKCEQDREPLRTIFFKDV
LKTHECLVKSGDLLMRDNLFEIITSSRTFYVQADSPEDMHSWIKEIGAAV
QALKCHP
 (partial human TAPP2 amino acid sequence)
or 
MPYVDRQNRICGFLDIEDNENSGKFLRRYFILDTQANCLLWYMDNPQNLA
VGAGAVGSLQLTYISKVSIATPKQKPKTPFCFVINALSQRYFLQANDQKD
LKDWVEALNQASKITVPKAGTVPLATEVLKNLTAPPTLEKKPQVAYKTEI
IGGVVVQTPISQNGGDGQEGCEPGTHAFLRRSQSYIPTSGCRPSTGPPLI
KSGYCVKQGNVRKSWKRRFFALDDFTICYFKCEQDREPLRTIPLKDVLKT
HECLVKSGDLLMRDNLFEIITTSRTFYVQADSPEDMHSWIEGIGAAVQAL
KCHPREPSFSRSISLTRPGSSTLTSAPNSILSRRRPPAEEKRGLCKAPSV
ASSWQPWTPVPQAEEKPLSVEHAPEDSLFMPNPGESTATGVLASSRVRHR
SEPQHPKEKPFVFNLDDENIRTSDV
 (mouse TAPP2 amino acid sequence; see also Accession No AF286161)
or a fragment, thereof, or a fusion of a said fragment, thereof.

Further TAPP polypeptides include the chicken TAPP2 sequence as given in Accession No AF302149. Human TAPP2 may have the sequence given in Accession No AF 286164, which is a fragment of the sequence given above, as follows:

MPYVDRQNRICGFLDIEEHENSGKFLRRYFILDTQANCLLWYMDNPQ
NLAMGAGAVGALQLTYISKVSIATPKQKPKTPFCFVINALSQRYFLQAND
QKDMKDWVEALNQASKITVPKGGGLPMTTEVLKSLAAPPALEKKPQVAYK
TEIIGGVVVHTPISQNGGDGQEGSEPGSHTILRRSQSYIPTSGCRASTGP
PLIKSGYCVKQGNVRKSWKRRFFALDDFTICYFKCEQDREPLRTIFFKDV
LKTHECLVKSGDLLMRDNLFEIITSSRTFYVQADSPEDMHSWIKEIGAAV
QALKCHP

Standard IUPAC one and three letter codes are used for amino acid sequences used in the specification, and the amino acid sequences are listed N-terminal to C-terminal as is conventional.

By "substantially pure" we mean that the said polypeptide is substantially free of other proteins. Thus, we include any composition that includes at least 30% of the protein content by weight as the said polypeptide, preferably at least 50%, more preferably at least 70%, still more preferably at least 90% and most preferably at least 95% of the protein content is the said polypeptide.

Thus, the invention also includes compositions comprising the said polypeptide and a contaminant wherein the contaminant comprises less than 70% of the composition by weight, preferably less than 50% of the composition, more preferably less than 30% of the composition, still more preferably less than 10% of the composition and most preferably less than 5% of the composition by weight.

The invention also includes the substantially pure said polypeptide when combined with other components *ex vivo,* said other components not being all of the components found in the cell in which said polypeptide is found. As is described below, the polypeptides of the invention can be produced using recombinant DNA technology.

Variants (whether naturally-occurring or otherwise) may be made using the methods of protein engineering and site-directed mutagenesis well known in the art using the recombinant polynucleotides described below.

By "fragment of said polypeptide" we include any fragment which retains activity or which is useful in some other way, for example, for use in raising antibodies or in a binding or other assay, or which fragment may have other functions as described in more detail below. Preferred fragments of TAPP are discussed further below.

By "fusion of said polypeptide" we include said polypeptide fused to any other polypeptide. For example, the said polypeptide may be fused to a polypeptide such as glutathione-S-transferase (GST) or protein A in order to facilitate purification of said polypeptide. Examples of such fusions are well known to those skilled in the art. Similarly, the said polypeptide may be fused to an oligo-histidine tag such as His6 or to an epitope recognised by an antibody such as the well known Myc tag epitope. Fusions to any fragment of said polypeptide are also included in the scope of the invention. It will be appreciated that fusions (or fragments thereof) which retain desirable properties, such as binding properties (for example, the ability to bind to a particular phosphoinositide) or the ability to change subcellular location in response to stress, insulin or growth factor signalling (in an intact cell) or other biological functions, of the said polypeptide (for example TAPP) are particularly preferred. It is also particularly preferred if the fusions are one which are suitable for use in the screening assays described earlier.

It will be appreciated that fusions which retain desirable properties, such as binding properties or other biological functions, of the said polypeptide are particularly preferred. It is also particularly preferred if the fusions are one which are suitable for use in the screening assays described above. It will be appreciated that before the present, invention, no requirement for producing any of the said polypeptides, or for variants or fusions or derivatives thereof, had not been appreciated in the art since their involvement in phosphoinositide signalling was not known. In particular it was not appreciated that the said polypeptides and variants and fusions thereof would be useful in screening methods for drugs and drug-like compounds.

By "variants" of the polypeptide we include insertions, deletions and substitutions, either conservative or non-conservative. In particular we include variants of the polypeptide where such changes do not substantially alter the activity of the said polypeptide. In particular we include variants of the polypeptide where such changes do not substantially alter the activity, for example the binding activity (for example to a phosphoinositide) of the said polypeptide. Variants of the said polypeptides do not include polypeptides which have the amino acid sequence of known polypeptides comprising a PH domain.

It will be appreciated that a variant that comprises substantially all of the sequence shown above (for example substantially full-length TAPP, PEPP or FAPP) may be particularly useful. By "substantially all" is meant at least 80%, preferably 90%, still more preferably 95%, 98% or 100% (ie all) of the said sequence. By "substantially full-length" is meant comprising at least 80%, preferably 90%, still more preferably 95%, 98% or 100% (ie all) of the sequence of the full length polypeptide.

By "conservative substitutions" is intended combinations such as Gly, Ala; Val, Ile, Leu; Asp, Glu; Asn, GIn; Ser, Thr; Lys, Arg; and Phe, Tyr.

It is particularly preferred if the polypeptide variant has an amino acid sequence which has at least 65% identity with either amino acid sequence given above, more preferably at least 75%, still more preferably at least 90%, yet more preferably at least 95%, and most preferably at least 98% or 99% identity with the appropriate amino acid sequence given above, most preferably with the amino acid sequence given above for human TAPP.

It is particularly preferred if the polypeptide variant has an amino acid sequence which has at least 90% identity with the amino acid sequence given above, more preferably at least 92%, still more preferably at least 95%, yet more preferably at least 96%, and most preferably at least 98% or 99% identity with the amino acid sequence given above.

The percent sequence identity between two polypeptides may be determined using suitable computer programs, for example the GAP program of the University of Wisconsin Genetic Computing Group and it will be appreciated that percent identity is calculated in relation to polypeptides whose sequences have been aligned optimally.

The alignment may alternatively be carried out using the Clustal W program (Thompson, J.D., Higgins, D.G. and Gibson, T.J. (1994), Clustal-W - improving the sensitivity of progressive multiple sequence alignment through sequence weighting, position specific gap penalties and weight matrix choice. Nuc. Acid Res. 22, 4673-4680).

The parameters used may be as follows:
Fast pairwise alignment parameters: K-tuple(word) size; 1, window size; 5, gap penalty; 3, number of top diagonals; 5. Scoring method: x percent.
Multiple alignment parameters: gap open penalty; 10, gap extension penalty; 0.05.
Scoring matrix: BLOSUM.

"Fragments" and "variants" also include those which are useful to prepare antibodies which will specifically bind the said polypeptide or mutant forms thereof lacking the function of the native polypeptide. Such variants and fragments will usually include at least one region of at least five consecutive amino acids which has at least 90% homology with the most homologous five or more consecutive amino acids region of the said polypeptide (ie when comparing forms of the polypeptide from different species). A fragment is less than 100% of the whole polypeptide.

The following peptides may be useful as TAPP1 (particularly human TAPP1) immunogens: YVDRQNRICGFLDIEENENSGK (this one would also be expected to recognise TAPP2) and RYTSRAGECSTYVGSHANVPS.

The following peptides may be useful as TAPP2 (particularly mouse TAPP2) immunogens: RVRHRSEPQHPKEKPFVFNL and KRGLCKAPSVASSWQPWTPVKK.

The amino acid sequences of TAPP 1 and TAPP2 are most dissimilar in the C-terminal region (excluding the extreme C-terminus), as is apparent from Figure 3A. Accordingly, a peptide with a sequence derived from the less-conserved C-terminal region of TAPP1 or TAPP2 may be useful in preparing antibodies that are specific for TAPP1 or TAPP2, respectively. A peptide with a sequence derived from the more conserved N-terminal region of TAPP1/TAPP2 may be useful in preparing antibodies that react with both TAPP1 and TAPP2.

It will be recognised by those skilled in the art that the polypeptide of the invention may be modified by known polypeptide modification techniques. These include the techniques disclosed in US Patent No 4,302,386 issued 24 November 1981 to Stevens. Such modifications may enhance the immunogenicity of the antigen, or they may have no effect on such immunogenicity. For example, a few amino acid residues may be changed. Alternatively, the antigen of the invention may contain one or more amino acid sequences that are not necessary to its immunogenicity. Unwanted sequences can be removed by techniques well known in the art. For example, the sequences can be removed via limited proteolytic digestion using enzymes such as trypsin or papain or related proteolytic enzymes.

Alternatively, smaller polypeptides corresponding to antigenic parts of the polypeptide may be chemically synthesised by methods well known in the art. These include the methods disclosed in US Patent No 4,290,944 issued 22 September 1981 to Goldberg.

Thus, the polypeptide of the invention includes a class of modified polypeptides, including synthetically derived polypeptides or fragments of the original polypeptide, having common elements of origin, structure, and immunogenicity that are within the scope of the present invention.

An additional embodiment of this aspect of the invention relates to a peptide or polypeptide which has the amino acid sequence of an epitope-bearing portion of a polypeptide of the invention, ie having an amino acid sequence described above. Such peptides or polypeptides include portions of a polypeptide of the invention with at least six or seven, preferably at least nine, and more preferably at least about 30 amino acids to about 50 amino acids, although epitope-bearing polypeptides of any length up to and including the complete amino acid sequence of a polypeptide of the invention described above also are included in the invention.

A particular embodiment of the invention provides a substantially pure TAPP polypeptide which consists of the amino acid sequence indicated above for human or mouse TAPP2 or naturally occurring allelic variants thereof

A preferred fragment of the TAPP polypeptide of the invention comprises the amino acid sequence of amino acids 1 to 147 of any of the given TAPP amino acid sequences. This fragment comprises the N-teiminal PH domain of TAPP. It is further preferred that the fragment does not comprise the amino acid sequence of about amino acids 190 to about 290 of the given amino acid sequence of TAPP. This fragment comprises the N-terminal PH domain of TAPP1 and does not comprise the C-terminal PH domain of TAPP1.

A further preferred fragment of the polypeptide of the invention comprises the amino acid sequence of amino acids 95 to 404 of any of the given TAPP amino acid sequences. This fragment comprises the C-terminal PH domain of TAPP1. It is further preferred that the fragment does not comprise the amino acid sequence of about amino acids 10 to 111 of the given amino acid sequence. This fragment comprises the C-terminal PH domain of TAPP1 and does not comprise the entire N-terminal domain of TAPP1.

Further preferred fragments of TAPP are discussed in Example 1, for example in the section relation to cloning of PH domains and in Figure 1.

Preferred fusions of these fragments include fusions as described in Example 1, for example fusions in which the said fragment has an N-terminal GST tag followed by a myc epitope tag or a FLAG (DYKDDDDK) epitope tag fused to the N-terminus of the said fragment.

A variant of the TAPP polypeptide of the invention which may be useful is a variant (or fragment, derivative or fusion of such a variant) wherein the residue equivalent to Arg212 of the given human TAPP1 amino acid sequence is mutated, for example to a leucine residue. Such a variant may be less able or unable to bind to PtdIns(3,4)P₂ (or other phosphoinositide), as described in Example 1.

Other variants of the polypeptide of the invention which may be useful are variants (or fragments, derivatives or fusions of such a variant) wherein the residue equivalent to any of the lysine or arginine residues of the PPBP is mutated to an acidic residue, for example glutamate or to a large hydrophobic residue, for example methionine. Such a variant may be less able or unable to bind to a phosphoinositide, as described in Example 1.

It will be appreciated that such fragments and variants may be useful in screening assays, medicine and/or in investigating the involvement of TAPP or other polypeptide of the invention in normal and diseased cells.

Thus, for example, it will be appreciated that a fragment of TAPP comprising the N-terminal (putative protein-binding) PH domain but not the C-terminal (phosphoinositide-binding) PH domain or a fragment of TAPP comprising the N-terminal PH domain but not the C-terminal PH domain may be capable of acting as an inhibitor, for example a dominant-negative inhibitor, of signalling *via* a signalling pathway in which TAPP may be involved, as discussed further below, for example signalling *via* an integrin receptor or a growth factor receptor. A variant of TAPP in which any of the conserved Lys/Arg sites in the PPBM is replaced with an acidic or hydrophobic residue, for example leucine, may act as a dominant negative mutant, which may bind to interacting polypeptides (for example *via* the N-terminal PH domain) but not to the phosphoinositide. Thus, such a fragment may be useful, for example, as an anti-cancer agent or in the promotion of apoptosis. Promotion of apoptosis may be beneficial in the resolution of inflammation. Inhibition of TAPP activity may inhibit platelet activation, which may be useful in reducing or preventing thrombosis. This may be important in patients at risk of thrombosis (for example obese patients or those with a history of thrombosis) and/or before, during or after surgery.

Over-expression of a substantially full-length native said polypeptide, for example a TAPP polypeptide may be useful in increasing signalling in which the said polypeptide is involved and therefore may also be useful in the treatment of diabetes or defects of glycogen regulation. It may also be useful in reducing apoptosis; thus, it may be useful in treating a patient in need of protection against apoptosis. Reducing apoptosis may be useful following ischaemic injury, for example stroke or myocardial infarction, and in tissue repair. It may also be useful in the treatment of patient before, after or during heart surgery.

It will be appreciated that a fusion of a polypeptide, variant or fragment of the invention wherein the fusion comprises a GST and/or FLAG or myc epitope portion may be particularly useful. For example, a GST tag may be useful in purifying or detecting the fusion protein, as described in Example 1, for example in detecting the interaction between the fusion protein and a phospholipid.

It is particularly preferred, although not essential, that the fragment of the said polypeptide, or the fusion of the fragment has at least 30% of the PtdIns(3,4)P₂, binding affinity of the said polypeptide, for example TAPP but is not capable of binding to PtdIns(3,4,5)P₃. It is more preferred if the fragment of the said polypeptide, or the fusion of the fragment has at least 50%, preferably at least 70% and more preferably at least 90% of the phosphoinositide binding activity of the said polypeptide, for example TAPP. However, it will be appreciated that fusions or fragments which are devoid of one or more binding activities as set out above may nevertheless be useful, for example as described above or by interacting with another polypeptide, or as antigens in raising antibodies. Methods of measuring the binding affinity with phosphoinositides are described, for example, in Example 1 below. Methods of measuring protein-protein interactions are well known to those skilled in the art and are discussed above.

By "residue equivalent to" a particular residue, for example the residue equivalent to Arg212 of human TAPP1, is included the meaning that the amino acid residue occupies a position in the native two or three dimensional structure of a polypeptide corresponding to the position occupied by the said particular residue, for example Arg212, in the native two or three dimensional structure of full-length human TAPP1.

The residue equivalent to a particular residue, for example Arg212 of full-length human TAPP1, may be identified by alignment of the sequence of the polypeptide with that of full-length human TAPP1 in such a way as to maximise the match between the sequences. The alignment may be carried out by visual inspection and/or by the use of suitable computer programs, for example the GAP program of the University of Wisconsin Genetic Computing Group, which will also allow the percent identity of the polypeptides to be calculated, or using the Align program (Pearson (1994) in: Methods in Molecular Biology, Computer Analysis of Sequence Data, Part II (Griffin, AM and Griffin, HG eds) pp 365-389, Humana Press, Clifton). Thus, residues identified in this manner are also "equivalent residues".

It will be appreciated that in the case of truncated forms of human TAPP1 or in forms where simple replacements of amino acids have occurred it is facile to identify the "equivalent residue".

Peptides may be synthesised by the Fmoc-polyamide mode of solid-phase peptide synthesis as disclosed by Lu et al (1981) J. Org. Chem. 46, 3433 and references therein. Temporary N-amino group protection is afforded by the 9-fluorenylmethyloxycarbonyl (Fmoc) group. Repetitive cleavage of this highly base-labile protecting group is effected using 20% piperidine in N,N-dimethylformamide. Side-chain functionalities may be protected as their butyl ethers (in the case of serine threonine and tyrosine), butyl esters (in the case of glutamic acid and aspartic acid), butyloxycarbonyl derivative (in the case of lysine and histidine), trityl derivative (in the case of cysteine) and 4-methoxy-2,3,6-trimethylbenzenesulphonyl derivative (in the case of arginine). Where glutamine or asparagine are C-terminal residues, use is made of the 4,4'-dimethoxybenzhydryl group for protection of the side chain amido functionalities. The solid-phase support is based on a polydimethylacrylamide polymer constituted from the three monomers dimethylacrylamide (backbone-monomer), bisacryloylethylene diamine (cross linker) and acryloylsarcosine methyl ester (functionalising agent). The peptide-to-resin cleavable linked agent used is the acid-labile 4-hydroxymethyl-phenoxyacetic acid derivative. All amino acid derivatives are added as their preformed symmetrical anhydride derivatives with the exception of asparagine and glutamine, which are added using a reversed N,N-dicyclohexylcarbodiimide/1-hydroxybenzotriazole mediated coupling procedure. All coupling and deprotection reactions are monitored using ninhydrin, trinitrobenzene sulphonic acid or isotin test procedures. Upon completion of synthesis, peptides are cleaved from the resin support with concomitant removal of side-chain protecting groups by treatment with 95% trifluoroacetic acid containing a 50% scavenger mix. Scavengers commonly used are ethanedithiol, phenol, anisole and water, the exact choice depending on the constituent amino acids of the peptide being synthesised. Trifluoroacetic acid is removed by evaporation *in vacuo,* with subsequent trituration with diethyl ether affording the crude peptide. Any scavengers present are removed by a simple extraction procedure which on lyophilisation of the aqueous phase affords the crude peptide free of scavengers. Reagents far peptide synthesis are generally available from Calbiochem-Novabiochem (UK) Ltd, Nottingham NG7 2QJ, UK. Purification may be effected by any one, or a combination of, techniques such as size exclusion chromatography, ionexchange chromatography and (principally) reverse-phase high performance liquid chromatography. Analysis of peptides may be carried out using thin layer chromatography, reverse-phase high performance liquid chromatography, amino-acid analysis after acid hydrolysis and by fast atom bombardment (FAB) mass spectrometric analysis.

A further embodiment of the invention provides a recombinant polynucleotide suitable for expressing a said phosphoinositide-binding protein of the preceding aspects of invention or suitable for expressing a fragment of said polypeptide or a fusion of a said fragment.

By "suitable for expressing" is meant that the polynucleotide is a polynucleotide that may be translated to form the polypeptide, for example RNA, or that the polynucleotide (which is preferably DNA) encoding the polypeptide of the invention is inserted into an expression vector, such as a plasmid, in proper orientation and correct reading frame for expression. The polynucleotide may be linked to the appropriate transcriptional and translational regulatory control nucleotide sequences recognised by any desired host; such controls may be incorporated in the expression vector.

A further embodiment of the invention is a replicable vector suitable for expressing a polypeptide as defined in the preceding aspect of the invention.

Preferences and exclusions for the said polynucleotide variant are equivalent to those in relation to the phosphoinositide-binding polypeptide of the invention. For example, the replicable vector may be suitable for expressing a fusion of the said phosphoinositide-binding polypeptide, in particular a GST fusion.

Characteristics of vectors suitable for replication in mammalian/eukaryotic cells are well known to those skilled in the art. It will be appreciated that a vector may be suitable for replication in both prokaryotic and eukaryotic cells.

In one preferred embodiment the polynucleotide comprises the nucleotide sequence: 
CGAGGGAGCGAGAGGCGCGGAGAGTTTGGCAGGCAGACCCAGAAATCCC
TGGAGCGCGGCGGACCCGGCGGCCGGAGGGCGACCCCGCCCGATGTAac
GCGCCCCGCCCGAGCCCCGGCCCCTGCaCGGGGGGGGTGATGTGAGCAG
AGCCCAGGAATGCCTTATGTGGATCGGCAGAACCGAATCTGTGGGTTTCT
GGACATCGAGGAGCATGAGAACAGCGGCAAGTTTCTGCGGAGGTACTTCA
TTCTGGACACCCAGGCTAACTGCCTCCTCTGGTATATGGACAACCCCCAG
AATCTGGCAATGGGGGCAGGAGCTGTTGGAGCTTTGCAGCTGACCTACAT
CTCGAAGGTGAGCATAGCTACCCCAAAACAGAAACCAAAAACTCCATTTT
GCTTTGTTATCAATGCCCTGTCTCAGAGATATTTCCTTCAAGCCAATGAT
CAGAAAGATATGAAGGACTGGGTTGAAGCCCTGAACCAAGCCAGCAAGAT
CACCGTTCCCaAAGGTGGGGGCCTACCCATGACCACTGAAGTTCTCAAGA
GCTTAgCAGCTCCTCCAGCCCTGGAGAAgAAgCCACAGGTGGCCTACAAG
ACGGAGATCATTGGAGGGGTGGTGGTCCACACACCCATCAGCCAGAACGG
 
TGGGGATGGGCAGGAAGGGAGTGAGCCCGGGTCCCACACCATCCTTcGAA
GGTcTCAGAGTTACATCCCCACGTCAGGCTGCCGTGCTTCCACTGGGCCT
CCCCTCATTAAGAGTGGTTACTGCGTGAAGCAAGGAATGTGCGGAAGAG
CTGGAAACGTCGcTTcTTTGCACTTGATGACTTTACCATCTGCTACTTCA
AGTGTGAGCAGGACCGAGAACCACTGCGCACCATATTTTTAAGGATGTT
cTGAAGACCCATGAATGTCTGGTCAAGTCTGGTGATCTcTTAATGAGGGA
CAACCTGTTTGAAATaaTAACAAGCTCCAGGACCTTCTACGTACAGGCAG
ACAGTCCAGAAGACATGCACAGCTGGATTAAGGAGATTGGCGCAGCTGTC
CAGGCCCTCAAGTGCCACCCC
 (partial human TAPP2)
or 
CCACGCGTCCGGCGGCGAAACTTCTCCGAGGTTCAAGCACAGGGGTGGTA
GCCCCTCAAGGACTGCCCGGGCAGCGGGTATGGAGGAGCGCA*AGAACG
TCCCAGGGTGATGTGAACAGAGCCCAGGAATGCCTTATGTGGATCGGCAG
AACCGAATCTGTGGGTTTCTGGATATTGAAGACAATGAGAACAGTGGCAA
ATTCCTCCGGAGATACTTTATCCTGGATACCCAGGCCAACTGCCTCCTCT
GGTACATGGACAATCCCCAAAACCTGGCCGTTGGGGCAGGAGCTGTCGGA
 
TCTCTGCAGCTGACCTACATCTCGAAGGTGAGCATAGCTACCCCAAAGCA
GAAACCTAAAACGCCATTCTGCTTCGTTATCAATGCCCTGTCTCAGAGAT
ATTTTCTTCAAGCCAATGACCAGAAAGATCTGAAGGACTGGGTAGAAGCC
TTGAACCAAGCCAGCAAGATCACTGTACCCAAAGCTGGACAGTACCCTT
GGCCACAGAAGTTCTCAAAAACTTAACAGCTCCTCCCACCCTAGAGAAGA
AGCCGCAGGTGGCCTACAAGACTGAGATCATCGGGGTGTGGTGGTACAA
ACGCCTATCAGCCAGAACGGTGGGATGGCAGGAAGGGTGCGAGCCAGG
GACTCACGCCTTCCTGCGAAGGTCTCAGAGCTACATCCCCACGTCAGGCT
GCCGCCCTTCCACTGGGCCTCCCCTCATTAAGAGTGGCTACTGTGTGAAG
CAAGGGAATGTGCGGAAGAGTTGGAAACGACGCTTCTTTGCCCTCGATGA
CTTTACCATCTGCTACTTCAAGTGTGAGCAGGACAGAGAGCCTCTGCGTA
CCATACCGCTCAAGGATGTTCTCAAGACTCACGAGTGTCTGGTCAAGTCT
GGTGATCTCTTAATGAGGGACAACCTGTTTGAAATCATAACCACCTCCAG
GACGTTCTACGTACAGGCGGACAGCCCTGAGGACATGCACAGCTGGATCG
AGGGGATTGGAGCAGCTGTCCAGGCTCTGAAGTGCCACCCTAGGGAGCCG
TCCTTTTCAAGGTCCATTTCTTTGACTCGACCTGGAAGTTCTACCCTTAC
AAGCGCGCCTAACTCCATCTTGTCAAGAAGGCGGCCACCAGCAGAAGAGA
AAAGAGGTCTCTGTAAGGCCCCTTCGGTGGCCTCCTCCTGGCAACCCTGG
ACACCTGTCCCCCAGGCTGAGGAAAAGCCGTTGTCGGTGGAGCATGCTCC
AGAGGACTCTCTATTCATGCCTAACCCTGGGGAGAGCACAGCTACAGGGG
TGCTGGCAAGTTCTCGAGTCAGGCACAGGTCGGAGCCCCAGCACCCCAAG
GAGAAGCCATTTGTATTCAACCTTGATGACGAAAACATACGAACCTCTGA
TGTGTGATATGCAGTGCCCGTTGCGTGCAGGAGAGCCAGGGGCTGTGACT
TATTTTCTCTGCCATGGTAGAGGACAGAGTCTAATGGCACTCACAGTGGA
GGGGCTCGTCTAGCTGGCTTGGTTTGCTATTATTGACACCATTTATTTAA
CTGGG
 (mouse TAPP2)
or a fragment as fusion thereof.

References for full length sequences of centaurin-β2 and AtPH1 are given in Example 1, for example in Table 1. Polynucleotides encoding full-length centaurin-β2 or AtPH1 are excluded from the polynucleotides of the invention.

It will be appreciated that sequences encoding other full length TAPP polypeptides, for example other mammalian TAPP polypeptides, may be obtained by routine use of methods well known to those skilled in the art, making use of the sequences shown above. Thus PCR methods may be used, particularly methods developed to generate 5' cDNA sequences (for example, the "RACE" method, as well known to those skilled in the art). Such methods may be used in conjunction with sequence database analysis, for example EST database analysis and sequencing, as well known to those skilled in the art.

It will be appreciated that an expressed sequence tag (EST) clone is not a recombinant polynucleotide as defined above as it lacks sequences necessary for the translation and therefore expression of the expressed sequence tag. EST sequences may be cloned in the vector Uni-ZAP XR, pT7T3D-Pac, pBluescript SK-, Lafmid BA or pCMV-SPORT2 vector.

A polynucleotide comprising a fragment of the recombinant polynucleotide encoding a polypeptide of the invention or a variant, fragment, fusion or derivative may also be useful. Preferably, the polynucleotide comprises a fragment which is at least 10 nucleotides in length, more preferably at least 14 nucleotides in length and still more preferably at least 18 nucleotides in length. Such polynucleotides are useful as PCR primers. A polynucleotide complementary to the polynucleotide (or a fragment thereof) encoding a polypeptide of the invention or a variant, fragment, fusion or derivative may also be useful. Such complementary polynucleotides are well known to those skilled in the art as antisense polynucleotides.

The polynucleotide or recombinant polynucleotide of the invention may be DNA or RNA, preferably DNA. The polynucleotide may or may not contain introns in the coding sequence; preferably the polynucleotide is a cDNA.

A "variation" of the polynucleotide includes one which is (i) usable to produce a protein or a fragment thereof which is in turn usable, for example a processed polypeptide as described above, or to prepare antibodies which specifically bind to the protein encoded by the said polynucleotide or (ii) an antisense sequence corresponding to the gene or to a variation of type (i) as just defined. For example, different codons can be substituted which code for the same amino acid(s) as the original codons. Alternatively, the substitute codons may code for a different amino acid that will not affect the activity or immunogenicity of the protein or which may improve or otherwise modulate its activity or immunogenicity. For example, site-directed mutagenesis or other techniques can be employed to create single or multiple mutations, such as replacements, insertions, deletions, and transpositions, as described in Botstein and Shortle, "Strategies and Applications of In Vitro Mutagenesis" Science, 229: 193-210 (1985). Since such modified polynucleotides can be obtained by the application of known techniques to the teachings contained herein, such modified polynucleotides are within the scope of the claimed invention.

Moreover, it will be recognised by those skilled in the art that the polynucleotide sequence (or fragments thereof) encoding a polypeptide of the invention can be used to obtain other polynucleotide sequences that hybridise with it under conditions of high stringency. Such polynucleotides includes any genomic DNA. Accordingly, the polynucleotide of the invention includes polynucleotide that shows at least 80%, preferably 85%, and more preferably at least 90% and most preferably at least 95% homology with the polynucleotide identified in the method of the invention, provided that such homologous polynucleotide encodes a polypeptide which is usable in at least some of the methods described below or is otherwise useful. Moreover, it will be recognised by those skilled in the art that the polynucleotide sequence (or fragments thereof) encoding a polypeptide of the invention can be used to obtain other polynucleotide sequences that hybridise with it under conditions of high stringency. Such polynucleotides includes any genomic DNA. Accordingly, the polynucleotide of the invention includes polynucleotide that shows at least 60%, preferably 70%, and more preferably at least 80% and most preferably at least 90% homology with the polynucleotide identified in the method of the invention, provided that such homologous polynucleotide encodes a polypeptide which is usable in at least some of the methods described below or is otherwise useful. As noted above, a polynucleotide encoding full length centaurin-β2 or AtPH1 is not a polynucleotide of the invention.

Per cent homology can be determined by, for example, the GAP program of the University of Wisconsin Genetic Computer Group.

DNA-DNA, DNA-RNA and RNA-RNA hybridisation may be performed in aqueous solution containing between 0.11XSSC and 6XSSC and at temperatures of between 55°C and 70°C. It is well known in the art that the higher the temperature or the lower the SSC concentration the more stringent the hybridisation conditions. By "high stringency" we mean 2XSSC and 65°C. 1XSSC is 0.15M NaCl/0.015M sodium citrate. Polynucleotides which hybridise at high stringency are included within the scope of the claimed invention.

"Variations" of the polynucleotide also include polynucleotide in which relatively short stretches (for example 20 to 50 nucleotides) have a high degree of homology (at least 80% and preferably at least 90 or 95%) with equivalent stretches of the polynucleotide of the invention even though the overall homology between the two polynucleotides may be much less. This is because important active or binding sites may be shared even when the general architecture of the protein is different.

A variety of methods have been developed to operably link polynucleotides, especially DNA, to vectors for example via complementary cohesive termini. Suitable methods are described in Sambrook et al(1989) Molecular Cloning, A Laboratory Manual, Cold Spring Harbor Laboratory, Cold Spring Harbor, NY.

A desirable way to modify the DNA encoding a polypeptide of the invention is to use the polymerase chain reaction as disclosed by Saiki et al (1988) Science 239, 487-491. This method may be used for introducing the DNA into a suitable vector, for example by engineering in suitable restriction sites, or it may be used to modify the DNA in other useful ways as is known in the art.

In this method the DNA to be enzymatically amplified is flanked by two specific primers which themselves become incorporated into the amplified DNA. The said specific primers may contain restriction endonuclease recognition sites which can be used for cloning into expression vectors using methods known in the art.

The DNA (or in the case of retroviral vectors, RNA) is then expressed in a suitable host to produce a polypeptide comprising the compound of the invention. Thus, the DNA encoding the polypeptide constituting the compound of the invention may be used in accordance with known techniques, appropriately modified in view of the teachings contained herein, to construct an expression vector, which is then used to transform an appropriate host cell for the expression and production of the polypeptide of the invention. Such techniques include those disclosed in US Patent Nos. 4,440,859 issued 3 April 1984 to Rutter et al*,* 4,530,901 issued 23 July 1985 to Weissman, 4,582,800 issued 15 April 1986 to Crowl, 4,677,063 issued 30 June 1987 to Mark et al*,* 4,678,751 issued 7 July 1987 to Goeddel, 4,704,362 issued 3 November 1987 to Itakura et al*,* 4,710,463 issued 1 December 1987 to Murray, 4,757,006 issued 12 July 1988 to Toole, et al, 4,766,075 issued 23 August 1988 to Goeddel et aland 4,810,648 issued 7 March 1989 to Stalker.

The DNA (or in the case of retroviral vectors, RNA) encoding the polypeptide constituting the compound of the invention may be joined to a wide variety of other DNA sequences for introduction into an appropriate host. The companion DNA will depend upon the nature of the host, the manner of the introduction of the DNA into the host, and whether episomal maintenance or integration is desired.

Generally, the DNA is inserted into an expression vector, such as a plasmid, in proper orientation and correct reading frame for expression. If necessary, the DNA may be linked to the appropriate transcriptional and translational regulatory control nucleotide sequences recognised by the desired host, although such controls are generally available in the expression vector. The vector is then introduced into the host through standard techniques. Generally, not all of the hosts will be transformed by the vector. Therefore, it will be necessary to select for transformed host cells. One selection technique involves incorporating into the expression vector a DNA sequence, with any necessary control elements, that codes for a selectable trait in the transformed cell, such as antibiotic resistance. Alternatively, the gene for such selectable trait can be on another vector, which is used to co-transform the desired host cell.

Host cells that have been transformed by the recombinant DNA of the invention are then cultured for a sufficient time and under appropriate conditions known to those skilled in the art in view of the teachings disclosed herein to permit the expression of the polypeptide, which can then be recovered.

Many expression systems are known, including bacteria (for example *E*. *coli* and *Bacillus subtilis*), yeasts (for example *Saccharomyces cerevisiae*), filamentous fungi (for example Aspergillus), plant cells, animal cells and insect cells.

The vectors include a prokaryotic replicon, such as the ColEl ori, for propagation in a prokaryote, even if the vector is to be used for expression in other, non-prokaryotic, cell types. The vectors can also include an appropriate promoter such as a prokaryotic promoter capable of directing the expression (transcription and translation) of the genes in a bacterial host cell, such as *E*. *coli,* transformed therewith.

A promoter is an expression control element formed by a DNA sequence that permits binding of RNA polymerase and transcription to occur. Promoter sequences compatible with exemplary bacterial hosts are typically provided in plasmid vectors containing convenient restriction sites for insertion of a DNA segment of the present invention.

Typical prokaryotic vector plasmids are pUC18, pUC19, pBR322 and pBR329 available from Biorad Laboratories, (Richmond, CA, USA) and pTrc99A and pKK223-3 available from Pharmacia, Piscataway, NJ, USA.

A typical mammalian cell vector plasmid is pSVL available from Pharmacia, Piscataway, NJ, USA. This vector uses the SV40 late promoter to drive expression of cloned genes, the highest level of expression being found in T antigen-producing cells, such as COS-1 cells.

An example of an inducible mammalian expression vector is pMSG, also available from Pharmacia. This vector uses the glucocorticoid-inducible promoter of the mouse mammary tumour virus long terminal repeat to drive expression of the cloned gene.

As described in Example 1, the pEBG-2T expression vector may be used to express GST fusion proteins in eukaryotic cells, for example in 293 cells (human embryonic kidney cells).

Useful yeast plasmid vectors are pRS403-406 and pRS413-416 and are generally available from Stratagene Cloning Systems, La Jolla, CA 92037, USA. Plasmids pRS403, pRS404, pRS405 and pRS406 are Yeast Integrating plasmids (YIps) and incorporate the yeast selectable markers HIS3, TRP1, LEU2 and URA3. Plasmids pRS413-416 are Yeast Centromere plasmids (YCps).

Useful yeast plasmid vectors are pRS403-406 and pRS413-416 and are generally available from Stratagene Cloning Systems, La Jolla, CA 92037, USA. Plasmids pRS403, pRS404, pRS405 and pRS406 are Yeast Integrating plasmids (YIps) and incorporate the yeast selectable markers HIS3, TRP1, LEU2 and URA3. Plasmids pRS413-416 are Yeast Centromere plasmids (YCps).

The present invention also relates to a host cell transformed with a polynucleotide vector construct of the present invention. The host cell can be either prokaryotic or eukaryotic. Bacterial cells are preferred prokaryotic host cells and typically are a strain of *E. coli* such as, for example, the *E*. *coli* strains DH5 available from Bethesda Research Laboratories Inc., Bethesda, MD, USA, and RR1 available from the American Type Culture Collection (ATCC) of Rockville, MD, USA (No ATCC 31343). Preferred eukaryotic host cells include yeast, insect and mammalian cells, preferably vertebrate cells such as those from a mouse, rat, monkey or human fibroblastic cell line. Yeast host cells include YPH499, YPH500 and YPH501 which are generally available from Stratagene Cloning Systems, La Jolla, CA 92037, USA. Preferred mammalian host cells include Chinese hamster ovary (CHO) cells available from the ATCC as CCL61, NIH Swiss mouse embryo cells NIH/3T3 available from the ATCC as CRL 1658, and monkey kidney-derived COS-1 cells available from the ATCC as CRL 1650. Preferred insect cells are Sf9 cells which can be transfected with baculovirus expression vectors.

Transformation of appropriate cell hosts with a DNA construct of the present invention is accomplished by well known methods that typically depend on the type of vector used. With regard to transformation of prokaryotic host cells, see, for example, Cohen et al(1972) Proc. Natl. Acad. Sci. USA 69, 2110 and Sambrook et al(1989) Molecular Cloning, A Laboratory Manual, Cold Spring Harbor Laboratory, Cold Spring Harbor, NY. Transformation of yeast cells is described in Sherman et al(1986) Methods In Yeast Genetics, A Laboratory Manual, Cold Spring Harbor, NY. The method of Beggs (1978) Nature 275, 104-109 is also useful. With regard to vertebrate cells, reagents useful in transfecting such cells, for example calcium phosphate and DEAE-dextran or liposome formulations, are available from Stratagene Cloning Systems, or Life Technologies Inc., Gaithersburg, MD 20877, USA.

Electroporation is also useful for transforming and/or transfecting cells and is well known in the art for transforming yeast cell, bacterial cells, insect cells and vertebrate cells.

For example, many bacterial species may be transformed by the methods described in Luchansky et al(1988) Mol. Microbiol. 2, 637-646 incorporated herein by reference. The greatest number of transformants is consistently recovered following electroporation of the DNA-cell mixture suspended in 2.5X PEB using 6250V per cm at 25:FD.

Methods for transformation of yeast by electroporation are disclosed in Becker & Guarente (1990) Methods Enzymol. 194, 182.

Successfully transformed cells, ie cells that contain a DNA construct of the present invention, can be identified by well known techniques. For example, cells resulting from the introduction of an expression construct of the present invention can be grown to produce the polypeptide of the invention. Cells can be harvested and lysed and their DNA content examined for the presence of the DNA using a method such as that described by Southern (1975) J. Mol. Biol. 98, 503 or Berent et al(1985) Biotech. 3, 208. Alternatively, the presence of the protein in the supernatant can be detected using antibodies as described below.

In addition to directly assaying for the presence of recombinant DNA, successful transformation can be confirmed by well known immunological methods when the recombinant DNA is capable of directing the expression of the protein. For example, cells successfully transformed with an expression vector produce proteins displaying appropriate antigenicity. Samples of cells suspected of being transformed are harvested and assayed for the protein using suitable antibodies.

Thus, in addition to the transformed host cells themselves, the present invention also contemplates a culture of those cells, preferably a monoclonal (clonally homogeneous) culture, or a culture derived from a monoclonal culture, in a nutrient medium.

A further aspect of the invention, provides a method of making the polypeptide of the preceding aspect of the invention the method comprising culturing a host cell comprising a recombinant polynucleotide or a replicable vector which encodes said polypeptide, and isolating said polypeptide from said host cell. Methods of cultivating host cells and isolating recombinant proteins are well known in the art.

The invention also includes a polypeptide, or a variant, fragment, derivative or fusion thereof, or fusion of a said variant or fragment or derivative obtainable by the above method of the invention.

A still further aspect of the invention provides an antibody reactive towards a polypeptide of the preceding aspect of the invention, for example TAPP or a fragment thereof. It is preferred that the antibody is not an antibody reactive towards centaurin-β2 or AtPH1.

It is preferred that the antibody does not react substantially with another polypeptide comprising a PH domain. Accordingly, it may be preferred if peptides based on the TAPP sequence are used which vary significantly from any peptides found in any other PH domains, for example in the polypeptides indicated in part A of Table 1.

Antibodies reactive towards the said polypeptide of the invention may be made by methods well known in the all In particular, the antibodies may be polyclonal or monoclonal.

Suitable monoclonal antibodies which are reactive towards the said polypeptide may be prepared by known techniques, for example those disclosed in "Monoclonal Antibodies: A manual of techniques", H Zola (CRC Press, 1988) and in "Monoclonal Hybridoma Antibodies: Techniques and Applications", SGR Hurrell (CRC Press, 1982).

In a preferred embodiment the antibody is raised using any suitable peptide sequence obtainable from the given amino acid sequence, for example of TAPP. It is preferred if polyclonal antipeptide antibodies are made. In a preferred embodiment of the invention, an antibody of the invention is capable of preventing or disrupting the interaction between a polypeptide of the invention or a fragment thereof and an interacting polypeptide identified by the method of the invention describe above, or a phosphoinositide. Such antibodies are believed to be useful in medicine, for example in treating cancer or promoting apoptosis.

Peptides in which one or more of the amino acid residues are chemically modified, before or after the peptide is synthesised, may be used providing that the function of the peptide, namely the production of specific antibodies *in vivo,* remains substantially unchanged. Such modifications include forming salts with acids or bases, especially physiologically acceptable organic or inorganic acids and bases, forming an ester or amide of a terminal carboxyl group, and attaching amino acid protecting groups such as N-t-butoxycarbonyl. Such modifications may protect the peptide from *in vivo* metabolism. The peptides may be present as single copies or as multiples, for example tandem repeats. Such tandem or multiple repeats may be sufficiently antigenic themselves to obviate the use of a carrier. It may be advantageous for the peptide to be formed as a loop, with the N-terminal and C-terminal ends joined together, or to add one or more Cys residues to an end to increase antigenicity and/or to allow disulphide bonds to be formed. If the peptide is covalently linked to a carrier, preferably a polypeptide, then the arrangement is preferably such that the peptide of the invention forms a loop.

According to current immunological theories, a carrier function should be present in any immunogenic formulation in order to stimulate, or enhance stimulation of, the immune system. It is thought that the best carriers embody (or, together with the antigen, create) a T-cell epitope. The peptides may be associated, for example by cross-linking, with a separate carrier, such as serum albumins, myoglobins, bacterial toxoids and keyhole limpet haemocyanin. More recently developed carriers which induce T-cell help in the immune response include the hepatitis-B core antigen (also called the nucleocapsid protein), presumed T-cell epitopes such as Thr-Ala-Ser-Gly-Val-Ala-Glu-Thr-Thr-Asn-Cys, beta-galactosidase and the 163-171 1 peptide of interleuldn-1. The latter compound may variously be regarded as a carrier or as an adjuvant or as both. Alternatively, several copies of the same or different peptides of the invention may be cross-linked to one another; in this situation there is no separate carrier as such, but a carrier function may be provided by such cross-linking. Suitable cross-linking agents include those listed as such in the Sigma and Pierce catalogues, for example glutaraldehyde, carbodiimide and succinimidyl 4-(N-maleimidomethyl)cyclohexane-1-carboxylate, the latter agent exploiting the -SH group on the C-terminal cysteine residue (if present).

If the peptide is prepared by expression of a suitable nucleotide sequence in a suitable host, then it may be advantageous to express the peptide as a fusion product with a peptide sequence which acts as a carrier. Kabigen's "Ecosec" system is an example of such an arrangement.

The peptide of the invention may be linked to other antigens to provide a dual effect.

It will be appreciated that other antibody-like molecules may be useful in the practice of the invention including, for example, antibody fragments or derivatives which retain their antigen-binding sites, synthetic antibody-like molecules such as single-chain Fv fragments (ScFv) and domain antibodies (dAbs), and other molecules with antibody-like antigen binding motifs. Such antibody-like molecules are included by the term antibody as used herein.

It will be appreciated that peptidomimetic compounds may also be useful in the practice of the invention. Thus, by "polypeptide" or "peptide" we include not only molecules in which amino acid residues are joined by peptide (-CO-NH-) linkages but also molecules in which the peptide bond is reversed Such retro-inverso peptidomimetics may be made using methods known in the art, for example such as those described in Mézière et al (1997) J. Immunol. 159, 3230-3237. This approach involves making pseudopeptides containing changes involving the backbone, and not the orientation of side chains. Mézière *et al* (1997) show that, at least for MHC class II and T helper cell responses, these pseudopeptides are useful. Retro-inverse peptides, which contain NH-CO bonds instead of CO-NH peptide bonds, are much more resistant to proteolysis.

Similarly, the peptide bond may be dispensed with altogether provided that an appropriate linker moiety which retains the spacing between the Cα atoms of the amino acid residues is used; it is particularly preferred if the linker moiety has substantially the same charge distribution and substantially the same planarity as a peptide bond.

It will be appreciated that the peptide may conveniently be blocked at its N- or C-terminus so as to help reduce susceptibility to exoproteolytic digestion.

A derivative or fusion of a polypeptide of the invention or variant, fragment or fusion thereof which may be particularly useful may comprise the polypeptide of the invention or variant, fragment or fusion therof and a further portion. Said further portion may confer a desirable feature on the said molecule; for example, the portion may useful in detecting or isolating the molecule, or promoting cellular uptake of the molecule. The portion may be, for example, a radioactive moiety, a fluorescent moiety, for example a small fluorophore or a green fluorescent protein (GFP) fluorophore, as well known to those skilled in the art. The moiety may be an immunogenic tag, for example a Myc, FLAG or HA (haemagglutinin) tag, as known to those skilled in the art or may be a lipophilic molecule or polypeptide domain that is capable of promoting cellular uptake of the molecule, as known to those skilled in the art, for example as characterised for a *Drosophila* polypeptide (see, for example, Derossi et al (1998) Trends Cell Biol 8, 84-87). Further useful tags include a tag that is capable of being phosphorylated, for example a tag capable of being phosphorylated by protein kinase A. Such a tag may be useful in introducing a radioactive label, for example ³²P or ³³P, onto the polypeptide.

Compounds, identifiable in the screening method, which mimic the effect of a particular phosphoinositide on a polypeptide, for example TAPP are believed to be useful in treating diabetes and/or other conditions, as indicated above. Compounds identifiable in the screening methods of the invention that inhibit binding of a phosphoinositide to the said polypeptide are believed to be useful in treating cancer. Compounds may be useful, for example, in the treatment of diabetes by switching on insulin-stimulated signal transduction pathways or in the treatment of cancer by inhibiting cell proliferation or promoting apoptosis. Compounds may also be useful in the modulation or resolution of inflammation or platelet activation, as discussed above.

It will be appreciated that certain compounds found in the screening methods may be able to enhance cell proliferation in a beneficial way and may be useful, for example in the regeneration of nerves or in wound healing.

The invention further provides a compound capable of altering the expression of a polypeptide of the invention, for example TAPP. The said compound may be an antisense molecule or ribozyme directed (for example, capable of binding to a polynucleotide encoding TAPP under physiological conditions) against a polynucleotide encoding a polypeptide of the invention, for example TAPP.

It will be appreciated that the nucleic acid of the invention may be an antisense oligonucleotide, for example an antisense oligonucleotide directed against a nucleic acid encoding a polypeptide of the invention such as the human TAPP gene. Antisense oligonucleotides are single-stranded nucleic acid, which can specifically bind to a complementary nucleic acid sequence. By binding to the appropriate target sequence, an RNA-RNA, a DNA-DNA, or RNA-DNA duplex is formed. These nucleic acids are often termed "antisense" because they are complementary to the sense or coding strand of the gene. Recently, formation of a triple helix has proven possible where the oligonucleotide is bound to a DNA duplex. It was found that oligonucleotides could recognise sequences in the major groove of the DNA double helix. A triple helix was formed thereby. This suggests that it is possible to synthesise a sequence-specific molecules which specifically bind double-stranded DNA *via* recognition of major groove hydrogen binding sites.

The nucleic acid of the invention may be an antisense oligonucleotide, for example an antisense oligonucleotide directed against a nucleic acid encoding a polypeptide of the invention such as the human TAPP gene. Antisense oligonucleotides are single-stranded nucleic acid, which can specifically bind to a complementary nucleic acid sequence.

By binding to the target nucleic acid, the above oligonucleotides can inhibit the function of the target nucleic acid. This could, for example, be a result of blocking the transcription, processing, poly(A)addition, replication, translation, or promoting inhibitory mechanisms of the cells, such as promoting RNA degradations.

Antisense oligonucleotides are prepared in the laboratory and then introduced into cells, for example by microinjection or uptake from the cell culture medium into the cells, or they are expressed in cells after transfection with plasmids or retroviruses or other vectors carrying an antisense gene. Antisense oligonucleotides were first discovered to inhibit viral replication or expression in cell culture for Rous sarcoma virus, vesicular stomatitis virus, herpes simplex virus type 1, simian virus and influenza virus. Since then, inhibition of mRNA translation by antisense oligonucleotides has been studied extensively in cell-free systems including rabbit reticulocyte lysates and wheat germ extracts. Inhibition of viral function by antisense oligonucleotides has been demonstrated *in vitro* using oligonucleotides which were complementary to the AIDS HIV retrovirus RNA (Goodchild, J. 1988 "Inhibition of Human Immunodeficiency Virus Replication by Antisense Oligodeoxynucleotides", Proc. Natl. Acad. Sci. (USA) 85(15), 5507-11). The Goodchild study showed that oligonucleotides that were most effective were complementary to the poly(A) signal; also effective were those targeted at the 5' end of the RNA, particularly the cap and 5' untranslated region, next to the primer binding site and at the primer binding site. The cap, 5' untranslated region, and poly(A) signal lie within the sequence repeated at the ends of retrovirus RNA (R region) and the oligonucleotides complementary to these may bind twice to the RNA.

Oligonucleotides are subject to being degraded or inactivated by cellular endogenous nucleases. To counter this problem, it is possible to use modified oligonucleotides, eg having altered internucleotide linkages, in which the naturally occurring phosphodiester linkages have been replaced with another linkage. For example, Agrawal et al (1988) Proc. Natl. Acad. Sci. USA 85, 7079-7083 showed increased inhibition in tissue culture of HIV-1 using oligonucleotide phosphoramidates and phosphorothioates. Sarin et al (1988) Proc. Natl. Acad. Sci. USA 85, 7448-7451 demonstrated increased inhibition of HIV-1 using oligonucleotide methylphosphonates. Agrawal et al (1989) Proc. Natl. Acad Sci. USA 86, 7790-7794 showed inhibition of ERV-1 replication in both early-infected and chronically infected cell cultures, using nucleotide sequence-specific oligonucleotide phosphorothioates. Leither et al (1990) Proc. Natl. Acad. Sci. USA 87, 3430-3434 report inhibition in tissue culture of influenza virus replication by oligonucleotide phosphorothioates.

Oligonucleotides having artificial linkages have been shown to be resistant to degradation in *viva.* For example, Shaw et al (1991) in Nucleic Acids Res. 19, 747-750, report that otherwise unmodified oligonucleotides become more resistant to nucleases *in vivo* when they are blocked at the 3□ end by certain capping structures and that uncapped oligonucleotide phosphorothioates are not degraded *in vivo.*

A detailed description of the H-phosphonate approach to synthesising oligonucleoside phosphorothioates is provided in Agrawal and Tang (1990) Tetrahedron Letters 31, 7541-7544. Syntheses of oligonucleoside methylphosphonates, phosphorodithioates, phosphoramidates, phosphate esters, bridged phosphoramidates and bridge phosphorothioates are known in the art. See, for example, Agrawal and Goodchild (1987) Tetrahedron Letters 28, 3539; Nielsen et al (1988) Tetrahedron Letters 29, 2911; Jager et al (1988) Biochemistry 27, 7237; Uznanski et al (1987) Tetrahedron Letters 28, 3401; Bannwarth (1988) Helv. Chim. Acta. 71, 1517; Crosstick and Vyle (1989) Tetrahedron Letters 30, 4693; Agrawal et al (1990) Proc. Natl. Acad. Sci. USA 87, 1401-1405. Other methods for synthesis or production also are possible. In a preferred embodiment the oligonucleotide is a deoxyribonucleic acid (DNA), although ribonucleic acid (RNA) sequences may also be synthesised and applied.

The oligonucleotides useful in the invention preferably are designed to resist degradation by endogenous nucleolytic enzymes. *In vivo* degradation of oligonucleotides produces oligonucleotide breakdown products of reduced length. Such breakdown products are more likely to engage in non-specific hybridization and are less likely to be effective, relative to their full-length counterparts. Thus, it is desirable to use oligonucleotides that are resistant to degradation in the body and which are able to reach the targeted cells. The present oligonucleotides can be rendered more resistant to degradation *in vivo* by substituting one or more internal artificial internucleotide linkages for the native phosphodiester linkages, for example, by replacing phosphate with sulphur in the linkage. Examples of linkages that may be used include phosphorothioates, methylphosphonates, sulphone, sulphate, ketyl, phosphorodithioates, various phosphoramidates, phosphate esters, bridged phosphorothioates and bridged phosphoramidates. Such examples are illustrative, rather than limiting, since other internucleotide linkages are known in the art. See, for example, Cohen, (1990) Trends in Biotechnology. The synthesis of oligonucleotides having one or more of these linkages substituted for the phosphodiester internucleotide linkages is well known in the art, including synthetic pathways for producing oligonucleotides having mixed internucleotide linkages.

Oligonucleotides can be made resistant to extension by endogenous enzymes by "capping" or incorporating similar groups on the 5' or 3' terminal nucleotides. A reagent for capping is commercially available as Amino-Link II^{™} from Applied BioSystems Inc, Foster City, CA. Methods for capping are described, for example, by Shaw et al (1991) Nucleic Acids Res. 19, 747-750 and Agrawal et al (1991) Proc. Natl. Acad. Sci. USA 88(17), 7595-7599.

A further method of making oligonucleotides resistant to nuclease attack is for them to be "self-stabilised" as described by Tang et al (1993) Nucl. Acids Res. 21, 2729-2735. Self-stabilised oligonucleotides have hairpin loop structures at their 3' ends, and show increased resistance to degradation by snake venom phosphodiesterase, DNA polymerase. I and fetal bovine serum. The self-stabilised region of the oligonucleotide does not interfere in hybridization with complementary nucleic acids, and pharmacokinetic and stability studies in mice have shown increased *in vivo* persistence of self-stabilised oligonucleotides with respect to their linear counterparts.

By "selectively hybridising" is meant that the nucleic acid has sufficient nucleotide sequence similarity with the said human nucleic acid that it can hybridise under moderately or highly stringent conditions, as discussed above. As is well known in the art, the stringency of nucleic acid hybridization depends on factors such as length of nucleic acid over which hybridisation occurs, degree of identity of the hybridizing sequences and on factors such as temperature, ionic strength and CG or AT content of the sequence. Thus, any nucleic acid which is capable of selectively hybridising as said is useful in the practice of the invention.

Nucleic acids which can selectively hybridise to the said human nucleic acid include nucleic acids which have >95% sequence identity, preferably those with >98%, more preferably those with >99% sequence identity, over at least a portion of the nucleic acid with the said human nucleic acid. As is well known, human genes usually contain introns such that, for example, a mRNA or cDNA derived from a gene would not match perfectly along its entire length with the said human genomic DNA but would nevertheless be a nucleic acid capable of selectively hybridising to the said human DNA.

Conveniently, the nucleic acid capable of selectively hybridising to the said human nucleic acid such as mRNA and which is used in the methods of the invention further comprises a detectable label.

By "detectable label" is included any convenient radioactive label such as ³²P, ³³P or ³⁵S which can readily be incorporated into a nucleic acid molecule using well known methods; any convenient fluorescent or chemiluminescent label which can readily be incorporated into a nucleic acid is also included. In addition the term "detectable label" also includes a moiety which can be detected by virtue of binding to another moiety (such as biotin which can be detected by binding to streptavidin); and a moiety, such as an enzyme, which can be detected by virtue of its ability to convert a colourless compound into a coloured compound, or *vice versa* (for example, alkaline phosphatase can convert colourless o-nitrophenylphosphate into coloured o-nitrophenol).

Primers which are suitable for use in a polymerase chain reaction (PCR; Saiki et al (1988) Science 239, 487-491) are preferred. Properties of suitable PCR primers are discussed above.

Where *in vivo* imaging is used in the methods of the invention, it may be preferable to use "humanized" chimeric monoclonal antibodies. Such antibodies can be produced using genetic constructs derived from hybridoma cells producing the monoclonal antibodies described above. Methods for producing chimeric antibodies are known in the art. See, for review, Morrison, Science 229:1202 (1985); Oi et al, BioTechniques 4:214 (1986); Cabilly et al., U.S. Patent No. 4,816,567; Taniguchi et al, EP 171496; Morrison et al, EP 173494; Neuberger et al, WO 8601533; Robinson et al, WO 870267 1; Boulianne et al., Nature 312:643 (1984); Neuberger et al, Nature 314:268 (1985).

Typical techniques for binding the above-described labels to antibodies are provided by Kennedy et al., Clin. Chim. Acta 70:1-31 (1976), and Schurs et al, Clin. Chim. Acta 81:1-40 (1977). Coupling techniques mentioned in the latter are the glutaraldehyde method, the periodate method, the dimaleintide method, the m-maleimidobenzyl-N-hydroxy-succinimide ester method.

The invention will now be describe in detail with reference to the following Examples and Figures:

### Figure Legends

**Figure 1****. SDS Polyacrylamide gel of purified GST-PH domains.** 2 µg of the indicated purified GST PH domain fusions, except for TAPP1[W281L] mutant (0.5 µg), which expressed poorly, were electrophoresed on a 4-12% SDS polyacrylamide gel and stained with Coomassie blue. The positions of the molecular mass markers (Biorad Precision markers) are indicated. TAPP1, TAPP2, centaurin-β2 and pleckshin-2 constructs were expressed in 293 cells and FAPP1, PEPP1, AtPH1, LL5α, LL5β, evectin-2 and PH30 were expressed in *E. coli*.

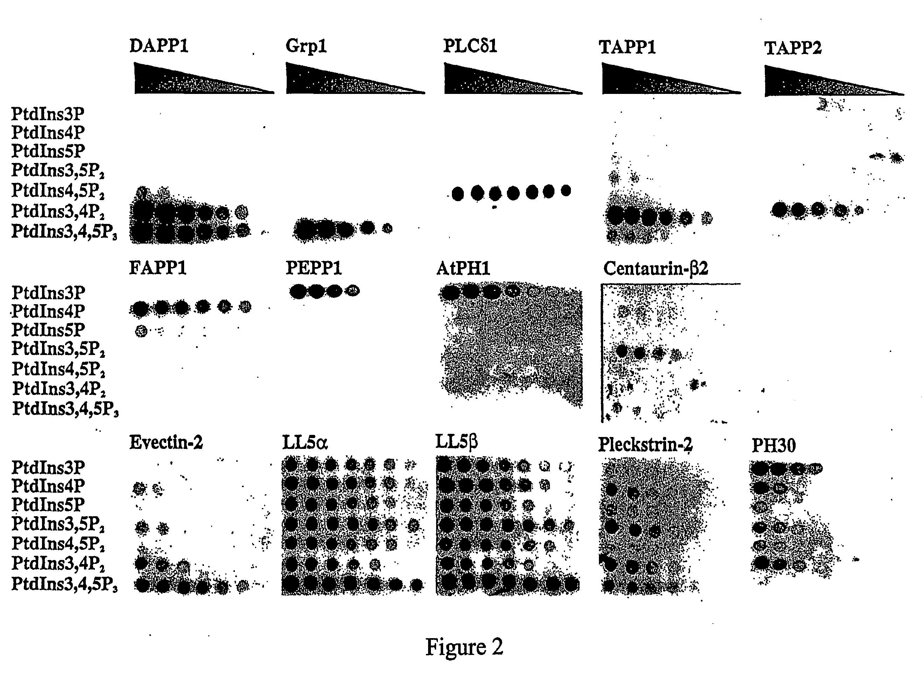

**Figure 2****. Phosphoinositide binding properties of the novel PH domains.** The ability of the indicated GST fusion proteins to bind a variety of phosphoinositides was analysed using a protein-lipid overlay. Serial dilutions of the indicated phosphoinositides (100 pmol, 50 pmol, 25 pmol, 12.5 pmol, 6.3 pmol, 3.1 pmol and 1.6 pmol) were spotted onto a nitrocellulose membranes which were then incubated with the purified GST fusion proteins. The membranes were washed and the GST-fusion proteins bound to the membrane by virtue of their interaction with lipid were detected using a GST antibody. A representative of at least 3 separate experiments carried out is shown.
**Figure 3****. Amino acid sequence and tissue distribution of TAPP1 and TAPP2**. (A) The alignment of the human and mouse TAPP1 and TAPP2 sequences are shown. The identities are shaded in black. The DNA sequences encoding the human (h) and mouse (m) TAPP1 shown are available from the NCBI database (accession numbers for human TAPP1 AF286160, mouse TAPP1 AF286165, human TAPP2 AF286164 and mouse TAPP2 AF286161). The amino acid residues corresponding to the N-terminal and C-terminal PH domains are indicated by a solid line and a dotted line respectively. The residues that comprise the putative SH3 domain binding proline rich motif of TAPP2 are boxed. The residues of the C-terminal PH domain of TAPP1 and TAPP2 that make up the PPBM are marked indicated (+). The C-terminal Ser-Asp-Val sequence of TAPP1 and TAPP2 that could interact with proteins possessing a PDZ domain(s) is marked with asterisks. The sequence of mouse TAPP1 and human TAPP2 is a partial sequence and the residues that are not known are indicated by a blank space. (B); TAPP1 and TAPP2 cDNAs were labelled with ³²P using random primers (see experimental section) and used to probe a Northern blot containing polyA+ RNA isolated from the indicated human tissues and cancer cell lines. The blot was washed and autoradiographed. The TAPP1 and TAPP2 probes were observed to hybridise to a 4 kb and a 6 kb message, respectively.
**Figure 4****. Comparison of the phosphoinositide binding properties of the N-terminal and C-terminal PH domains of TAPP1 and TAPP2.** The ability of wild type and mutant forms of full length (FL) and isolated N-terminal (NT) and C-terminal (CT) PH domains of TAPP1 and TAPP2 GST-fusion proteins to interact with phosphoinositides were analysed using a protein-lipid overlay. Serial dilutions of the indicated phosphoinositides (100 pmol, 50 pmol, 25 pmol, 12.5 pmol, 6.3 pmol, 3.1 pmol and 1.6 pmol) were spotted onto a nitrocellulose membrane which was then incubated with the indicated purified GST fusion proteins. The membranes were washed and the GST-fusion proteins bound to the membrane by virtue of their interactions with lipid were detected using a GST antibody. A representative experiment of three is shown. The isolated N-terminal PH domain of human TAPP1 comprises residues 1 to 147, the isolated C-terminal PH domain of human TAPP1 comprises residues 95 to 404, the isolated N-terminal PH domain of mouse TAPP2 comprises residues 1 to 131 and the isolated C-terminal PH of mouse TAPP2 comprises residues 174 to 425.

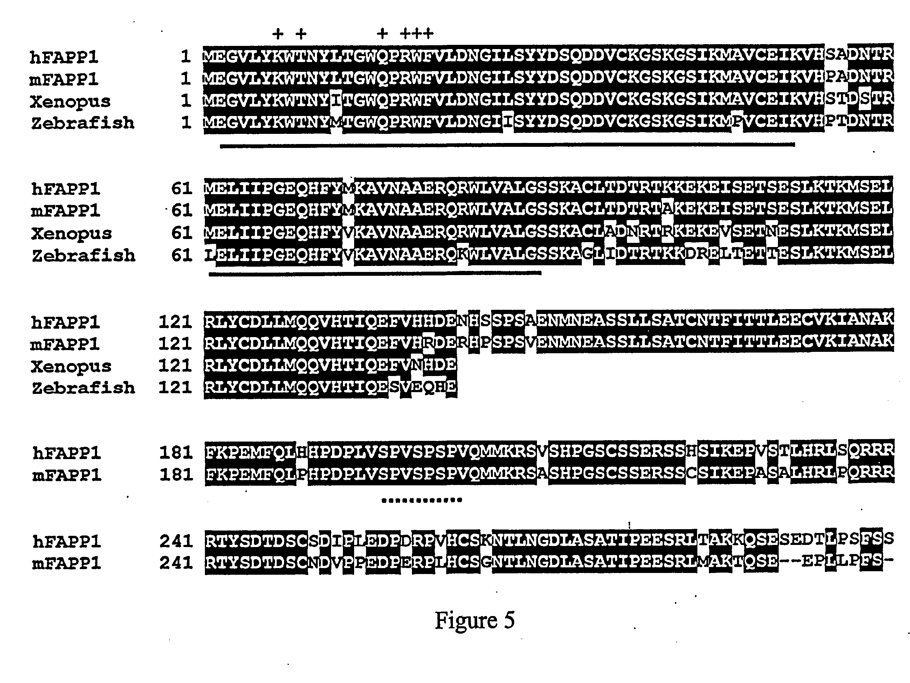

**Figure 5** **Amino acid sequence of human and mouse FAPP1.** The alignment of the full length human and mouse FAPP1 and partial Xenoupus and zebrafish sequences are shown. The identities are shaded in black. The DNA sequences of human (accession number AF2861621 and mouse FAPP1 (accession number AF286163) are available from the NCBI database. The partial *Xenopus* and *zebrafish* FAPP1 sequences are predicted from the EST sequences with NCBI accession numbers AW644282 and AW174299 respectively. The amino acid residues corresponding to the PH domain are underlined and the residues that comprise the putative SH3 domain binding motif are indicated by a dotted line. The residues of the PH domain of FAPP1 that make up the PPBM are marked indicated (+).

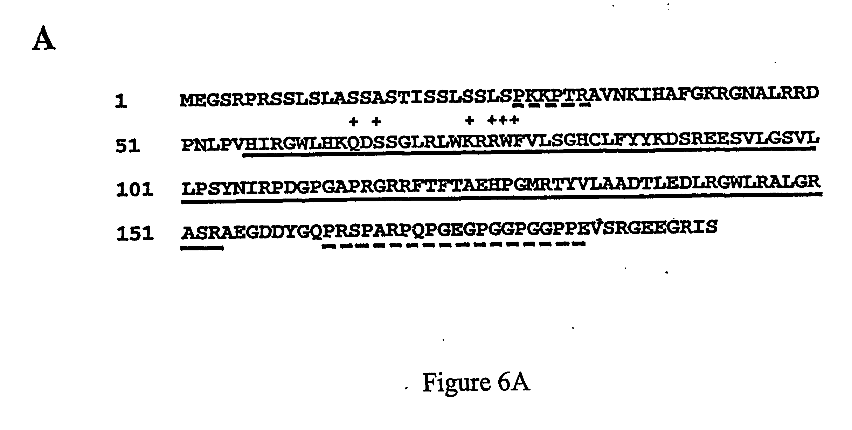

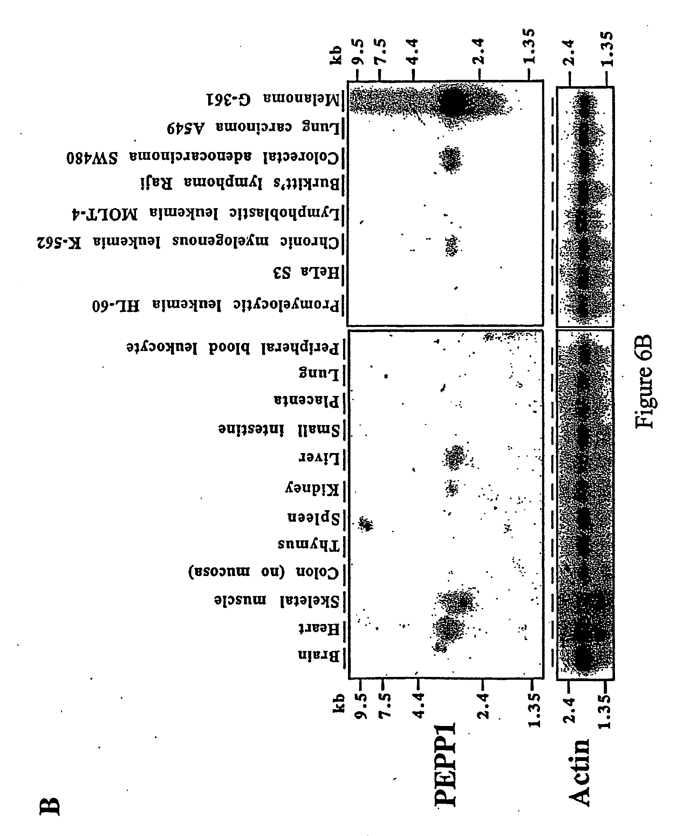

**Figure 6****. Amino acid sequence and tissue distribution of PEPP1.** (A) The partial sequence of human PEPP1 that has been sequenced thus far is shown. The amino acid residues corresponding to the PH domain are indicated by a solid line and the residues that could form a putative SH3 domain binding motif are indicated by a dotted line. The DNA sequence is available from the NCBI database (accession number AF286166). The residues of the PH domain of PEPP1 that make up the PPBM are marked indicated (+). (B) The partial cDNA for PEPP1 shown above was labelled with ³²P, using random primers, and used to probe a Northern blot containing polyA+ RNA isolated from the indicated human tissues and cancer cell lines. The blot was washed and autoradiographed. The PEPP1 probe was observed to hybridise with a 3 kb message in the melanoma G-361 cell line.
**Figure 7****. Alignment of PH domains**. Identities are indicated in black and homolgies in grey. Residues making up the PPBM are indicated with asterisks. Abbreviations: h, human; m, mouse; b2-cent, β2-centaurin.

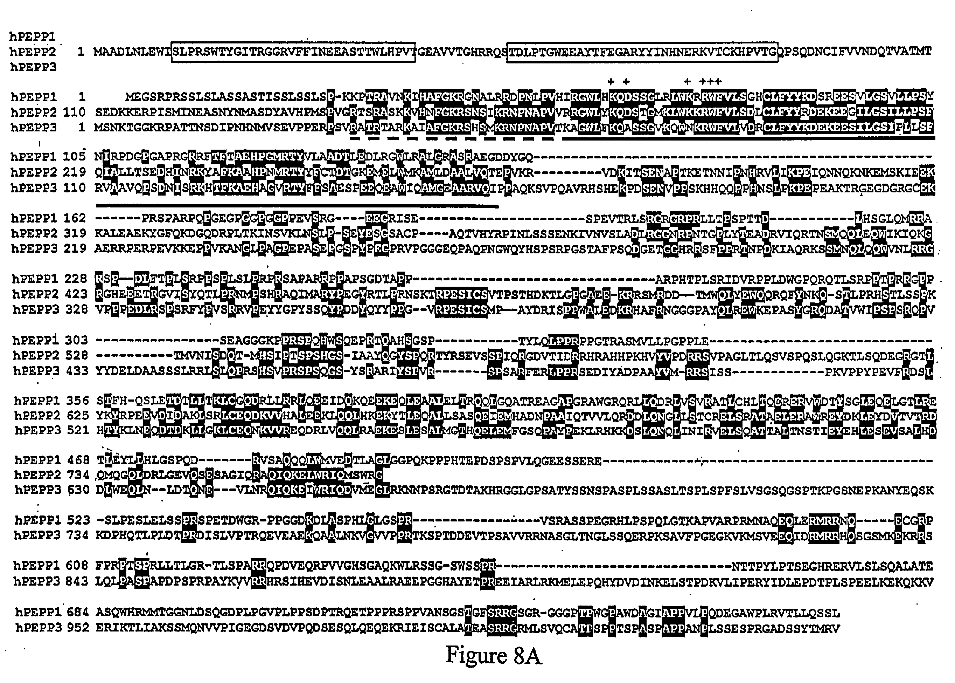

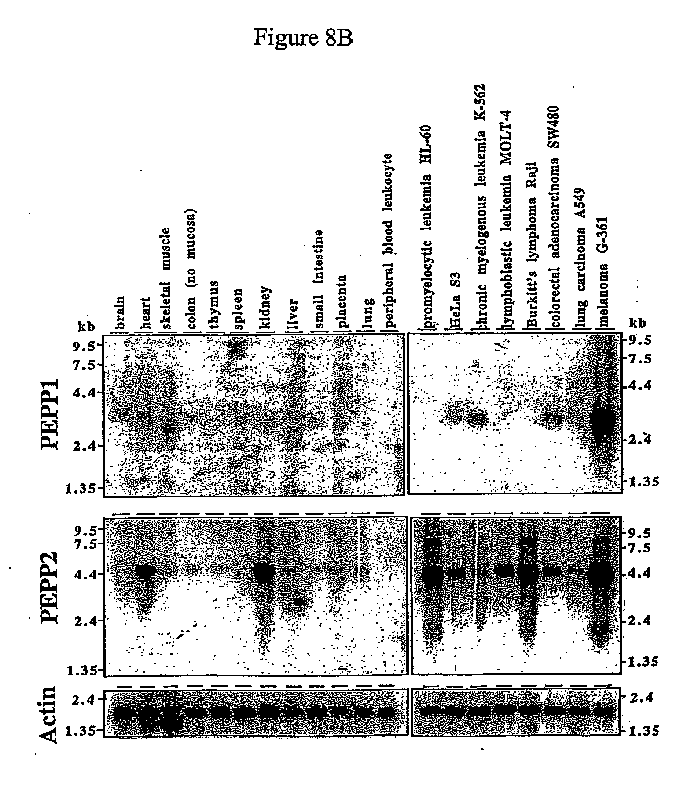

**Figure 8****: Amino acid sequence and tissue distribution of PEPP1, 2 and 3.** (A) The alignment of the full length human sequences of PEPP1, PEPP2 and PEPP3 are shown. The identities are shaded in black. The DNA sequences of human PEPP1 and human PEPP3 are indicated above and in NCBI database entries AF286166 and NM_014935. The amino acid residues corresponding to the PH domain are indicated by a solid line and the region of homology preceding the PH domain is indicated with a dotted line. The residues of the PH domain of PEPP1 that make up the PPBM are marked indicated (+) and the WW domains of PEPP2 are boxed. (B) The partial cDNA for PEPP1 and PEPP2 shown above was labelled with ³²P using random primers and used to probe a Northern blot containing polyA⁺ RNA isolated from the indicated human tissues and cancer cell lines. The blot was washed and autoradiographed. The PEPP1 probe was observed to hybridise with a 3kb message in the melanoma G-361 cell line and the PEPP2 probe hybridised with a 4.6 kb message.

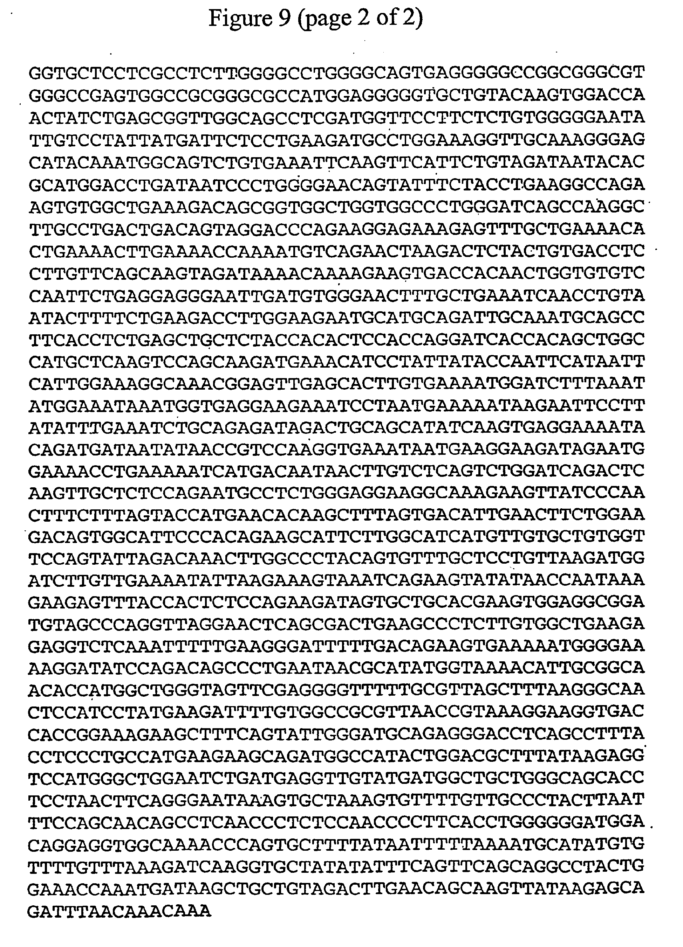

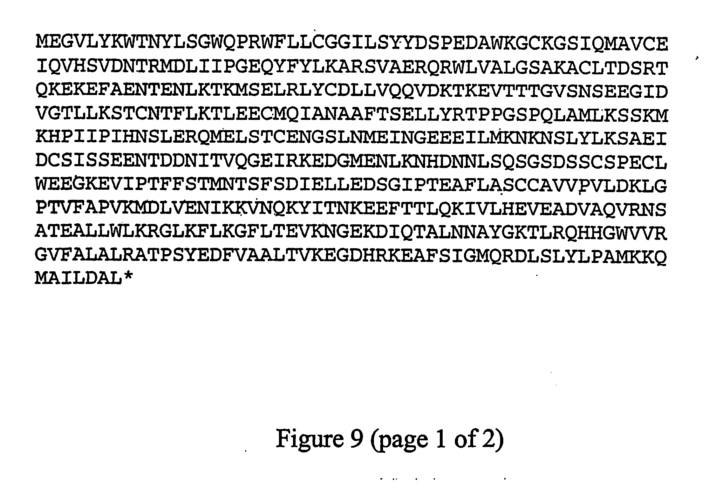

**Figure 9****: Amino acid and nucleotide sequences of human FAPP2.**

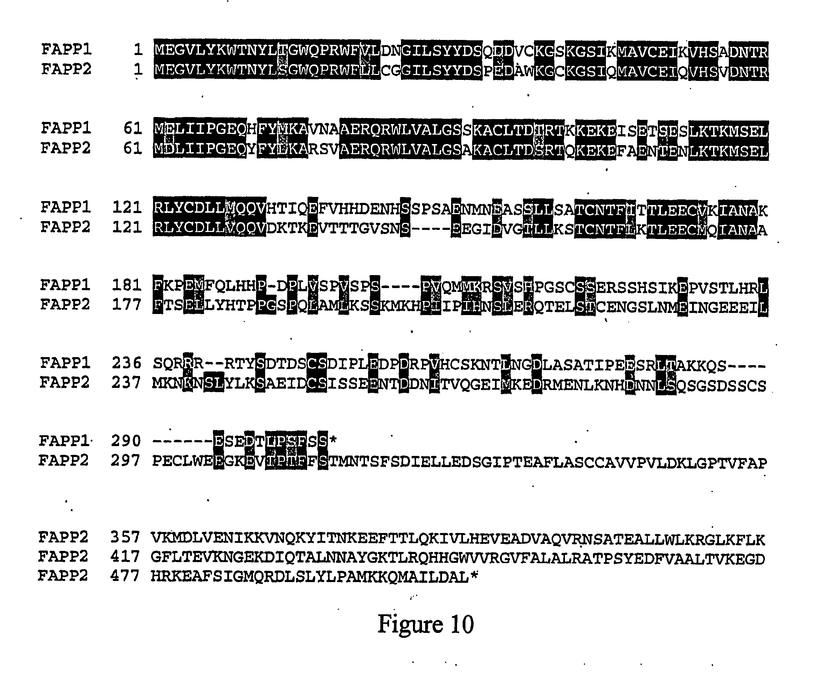

**Figure 10****: Amino acid sequecne alignment of human FAPP1 and human FAPP2.**

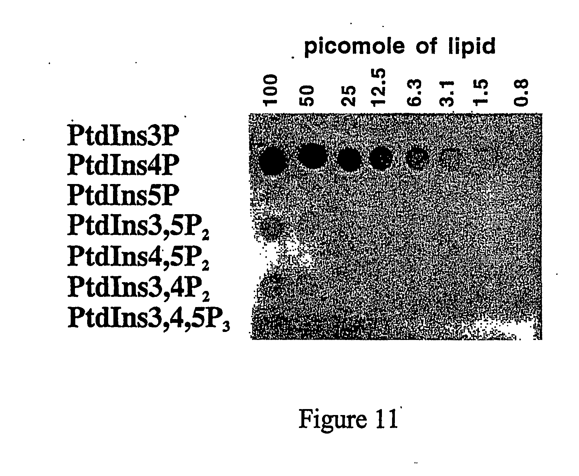

**Figure 11****: Human FAPP2 specifically binds phosphoinositol 4-monophosphate (PtdIns-4P).** Methods used are equivalent to those specified in the legend to Figure 2.

### Example 1: Identification of PH domains with novel phosphoinositide binding specificities.

The second messsenger phospatidylinositol (3,4,5)-trisphosphate (PtdIns(3,4,5)P₃) is generated by the action of phosphatidylinositol 3-kinase (PI 3-kinase) and regulates a plethora of cellular processes. An approach for dissecting the mechanisms by which these processes are regulated, is to identify proteins that interact specifically with PtdIns(3,4,5)P₃. The pleckstrin homology (PH) domain has become recognised as the specialised module used by many proteins to interact with PtdIns(3,4,5)P₃. Recent work has led to the identification of a Putative PtdIns(3,4,5)P₃ Binding Motif (PPBM) at the N-terminal regions of PH domains that interact with this lipid. We have identified novel or uncharacterised PH domains possessing a PPBM and determined their phosphoinositide binding properties. Surprisingly, many of the PH domains identified possess unexpected phosphoinositide binding specificities and do not bind PtdIns(3,4,5)P₃. These include PH domains that interact specifically with PtdIns(3,4)P₂ (TAPP1), PtdIns3P (PEPP1 & AtPH1 and also PEPP2 and PEPP3), PtdIns4P (FAPP1) and Ptdlns(3,5)P₂ (Centaurin-β2).

**Abbreviations:** ARF, ADP ribosylation factor; DAPP1, dual adaptor for phosphotyrosine and 3-phosphoinositides; EST, expressed sequence tag; FAPP1, PtdIns-Four-phosphate AdaPtor Protein-1; GAP, GTPase activating protein; GST, glutathione-S-transferase; NCBI, National Center for Biotechnology Information; PKC, protein kinase C; PDZ, postsynaptic density protein (PSD-95)/Drosophila disc large tumour suppressor (Dlg)/tight junction protein (ZO1); PDK1, 3-phosphoinositide-dependent protein kinase-1; PH, pleckstrin homology; PEPP1, PtdIns-thrEe-Phosphate binding PH domain Protein-1; PI 3-kinase, phosphoinositide 3-kinase; PKB, protein kinase B; PPBM, Putative PtdIns(3,4,5)P₃ binding motif; PtdIns, phosphatidylinositol; TAPP, TAndem PH domain containing Protein; Xaa, any amino acid.

**Materials** All phosphoinositides used in this study were dipalmitoyl derivatives obtained from Cell Signals, which were analysed by thin layer chromatography and found to migrate as single products. Hybond-C extra was from Amersham Pharmacia Biotech, High Fidelity PCR kit from Roche, Human tissue (Catalogue number 7780-1), mouse tissue (Catalogue number 7762-1) and human cancer cell line (Catalogue number 7757-1) Multiple Tissue Northern Blots from Clontech, Human Universal cDNA Library was from Strategene, pCR 2.1Topo vector and precast SDS polyacrylamide gels were from InVitrogen. DAPP1 and Grpl [8] were expressed as fusion proteins with glutathione-S-transferase (GST) in 293 cells [4]. The PH domain of human phospholipase C81 (residues 20 to 184) fused to GST was expressed *in E. coli.*

**General methods and buffers.** Restriction enzyme digests, DNA ligations, site directed mutagenesis and other recombinant DNA procedures were performed using standard protocols, as well known to those skilled in the art. All DNA constructs were verified by DNA sequencing.
Buffer A : 50 mM Tris-HCl pH 7.5, 1 mM EGTA, 1 mM EDTA, 1% (by mass) Triton-X 100, 1 mM sodium orthovanadate, 50 mM sodium fluoride, 5 mM sodium pyrophosphate, 0.27 M sucrose, 1 µM microcystin-LR, 0.1% (by vol) β-mercaptoethanol and 'complete' proteinase inhibitor cocktail (one tablet per 50 ml, Roche). Buffer B: 50 mM Tris/HCl pH 7.5, 0.1 mM EGTA, 10 mM β-mercaptoethanol and 0.27M sucrose.

**Cloning of PH domains and preparation of expression constructs.** All the human and mouse EST's were obtained from the I.M.A.G.E. Consortium [13] and sequenced. The plant EST (accession number T04439) encoding a full length clone of AtPH1 was obtained from the Arabidopsis Biological Research Centre (Ohio University). The sequence of each EST was verified and the full length PH domain of each EST was amplified by PCR using the Hi-fidelity PCR system with primers designed to incorporate a Kozak site, an initiating ATG codon followed by a myc epitope tag and a stop codon after the PH domain. The region of each protein that was amplified using the indicated EST as template was as follows: human TAPP1 (residues 95 to 404, accession number AI216176), mouse TAPP2 (residues 174 to 425, accession number AA111410), human FAPP1 (residues 1 to 99, accession number W32183), *Arabidopsis thaliana* AtPH1 (full length protein, residues 1 to 145, accession number, T04439), human PEPP1 (sequence in Fig 6 Ser-Ala-Ser to Arg-Pro-Gln, accession number N31123), mouse centaurin-β2 (residues 266 to 390, accession number AA967911), putative human homologue of rat LL5α (sequence Ser-Glu-Ser-Ala to Gln-Phe-Met-Asn, accession number AA863428), putative human isoform of LL5α which we have termed LL5β (sequence Arg-Lys-Glu-Asp to His-Phe-Leu-Leu, accession number AA461369), mouse pleckstrin-2 (residues 1 to 249, accession number AI326844), human evectin-2 (residues 1 to 167, accession number AA101447) and human PH30 (sequence Asn-Ser-Ser-Ile to Ile-Ser-Asp-Ala, accession number AI827615). The PCR products were resolved on 1% agarose, gel purified, cloned into the pCR2.1 TOPO vector, sequenced and subcloned into the *E.coli* pGEX-4T-1 expression vector or the mammalian pEBG2T vector that codes for the expression of these proteins with a GST tag at the N-terminus.

**Expression of GST-PH domains in *E.coli.*** The pGEX-4T-1 constructs encoding the PH domains of FAPP1, AtPH1, PEPP1, LL5α, IL5β, evectin-2 and PH30 were transformed into BL21 E.*coli* cells and a 0.5L culture was grown at 37 °C in Luria Broth containing 100 µg/ml ampicillin, until the absorbance at 600 nm was 0.6. 250 µM isopropyl-β-D-galactosidase was added and the cells cultured for a further 16 h at 26 °C. The cells were resuspended in 25 ml of ice-cold Buffer A and lysed by one round of freeze thawing and the lysates sonicated to fragment the DNA. The lysates were centrifuged at 4°C for 30 min at 20, 000 x g, the supernatant filtered through a 0.44 micron filter and incubated for 60 min on a rotating platform with 1 ml of glutathione-Sepharose previously equilibrated in Buffer A. The suspension was centrifuged for 1 min at 3000 x g, the beads washed three times with 15 ml of Buffer A containing 0.5 M NaCl, and then a further ten times with 15 ml of Buffer B. The protein was eluted from the resin at ambient temperature by incubation with 2 ml of Buffer B containing 20 mM glutathione, and the beads removed by filtration through a 0.44 micron filter. The eluate was divided into aliquots, snap frozen in liquid nitrogen, and stored at -80°C.

**Expression of GST-PH domains in human embryonic kidney 293 cells.** As the PH domains of TAPP1, TAPP2, centaurin-β2, and pleckstrin-2 were significantly degraded when expressed in bacteria (data not shown), these were expressed as GST fusion proteins in human embryonic kidney 293 cells. For the expression of each construct, twenty 10 cm diameter dishes of 293 cells were cultured and each dish transfected with 5 µg of the pEBG-2T construct, using a modified calcium phosphate method [14]. 36 h post-transfection, the cells were lysed in 1 ml of ice-cold Buffer A, the lysates pooled, centrifuged at 4°C for 10 min at 13, 000 x g and the GST-fusion proteins were purified by affinity chromatography on glutathione-Sepharose and stored as described above.

**Cloning TAPP1, TAPP2 , FAPP1 and PEPP1.** Full length human TAPP1, full length mouse TAPP2, partial mouse TAPP1, partial human TAPP2, and full length human and mouse FAPP1 sequences were deduced by sequencing the EST clones listed in Table 3. Several EST clones possessed identical sequences, and had the same in-frame stop codon 5' to the predicted initiating ATG codon and possessed a stop codon at the same position at the 3' end of the gene. The constructs used to express full length and deletion mutants of TAPP1 and TAPP2 were generated by PCR, using as a template ESTs encoding full length human TAPP1 (accession number AI216176) and full length mouse TAPP2 (accession number AA111410). The PCR primers used were designed to incorporate a Kozak site, and an initiating ATG codon followed by a Flag epitope tag and the resulting PCR product was subcloned into the pEBG2T mammalian expression vector.

**Cloning of PEPP1 and FAPP1.** A Stratagene Human Universal cDNA Library was screened with a DNA probe corresponding to the PH domains of PEPP1 and FAPP1 and we were able to isolate a clone encoding each of these proteins using this approach. The partial sequence of PEPP1 that contains the 5'end of the coding sequence was obtained by sequencing of ESTs with NCBI accession numbers N49341and N31123. To obtain a full length cDNA encoding PEPP1, we screened a Stratagene Human Universal cDNA Library with a DNA probe corresponding to the N-terminal 15 to 169 residues of PEPP1 and we isolated a full length PEPP1 cDNA which had a stop codon 5' to the predicted initiating ATG codon an open reading frame encoding 779 amino acids followed by a stop codon. Interrogation of the EST databases with the full length PEPP1 sequ4ence identified 2 closely related isoforms of this protein termed PEPP2 and PEPP3. The sequence of human PEPP2 was deduced by sequencing the following EST clones: A1808805 (kidney), AA232124(brain), W91917 (foetal liver and spleen) and AI638629 (germ cell line). The sequence of PEPP2 is likely to be full length as there is a stop codon 5' to the predicted initiating ATG codon. ESTs relating to PEPP3 are AI739438, BE303674 and F23241.

**Northern Blot Analysis.** cDNA corresponding to full length human TAPP1, partial human TAPP2 (residues 18 to 304), partial human PEPP1 (residues encoding sequence Ser-Ala-Ser to Arg-Pro-Gln), partial human PEPP2 (residues 154 to 654) and mouse partial mouse centaurin-β2 (residues 266 to 390) were ³²P-labelled by random priming using a multi-prime DNA labelling kit (Amersham Pharmacia). These probes were then used to screen Northern blots using Rapid-Hyb Buffer (Amersham Pharmacia) according to the protocol provided by the manufacturer.

**Protein-Lipid overlay.** To assess the phosphoinositide binding properties of each PH domain, a protein-lipid overlay assay was performed using the GST fusion proteins as described previously [4, 15]. Briefly, 1 µl of lipid solution containing 1-100 pmol of phospholipids dissolved in a mixture of choroform:methanol:water (1:2:0.8) was spotted onto Hybond-C extra membrane and allowed to dry at room temperature for 1 h. The membrane was blocked in 3% (by mass) fatty acid-free BSA in TBST (50 mM Tris/HCl pH 7.5, 150 mM NaCl and 0.1% Tween-20 (by vol) for 1h. The membrane was then incubated overnight at 4 °C with gentle agitation in the same solution containing 0.2 µg/ml of the indicated GST fusion protein. The membranes were washed 6 times over 30 min in TBST and then incubated for lh with 1/1000 dilution of anti-GST monoclonal antibody (Sigma). The membranes were washed as before, then incubated for 1h with 1/5000 dilution of anti-mouse-HRP conjugate (Pierce). Finally, the membranes were washed 12 times over lh in TBST and the GST-fusion protein bound to the membrane by virtue of its interaction with phospholipid was detected by enhanced chemiluminescence.

### BIACore measurements of PH domain-lipid interactions.

Kinetic analyses of the interactions between the GST PH domain fusions and the polyphosphoinositides were made using surface plasmon resonance based procedures as described previously [4, 16], with the following modifications. The mole percentage of the test polyphosphoinositide was reduced from 1 % to 0.1%. This helped to minimise any mass transport limitation in the binding interaction and increased the rate of lipid immobilisation on the chip. The intracellular buffer was supplemented to 0.27 M sucrose to reduce the bulk refractive index changes associated with the addition of Buffer B. Proteins were injected over the monolayers at concentrations ranging from 1 µM to 10 nM. Data were analysed using the bimolecular interaction model and the global fitting feature of the BIAevaluation 3 software for several sensorgrams at different protein concentrations. GST PH domain binding to phosphoinositides does not fit well to this model due to the slow dissociation of the protein from the surface [4, 16]. Therefore, the affinity of binding of these proteins to polyphosphoinositides is likely to be overestimated by this method and the results are therefore stated as apparent equilibrium dissociation constants for comparative purposes. The relative binding affinities of each protein relative to the binding of full length GST-TAPP1 to PtdIns(3,4)P₂ were also calculated.

### Results.

**Identification of novel or uncharacterised PH domains.** The NCBI/EMBL/PDB EST databases were interrogated with the amino acid sequences encoding the PH domains of human PKBα, PDK1, Grp1 and DAPP1. These searches revealed 11 partial sequences (see Table 1) encoding either novel or previously uncharacterised PH domain-containing proteins possessing at least 5 of the 6 conserved residues in the PPBM (Table 1). We cloned the entire PH domain of each of these proteins (see experimental section) which are named in Table 1. They were expressed in *E. coli* or human embryonic 293 cells as fusions to glutathione S-transferase (GST) and purified by affinity chromatography on glutathione-Sepharose. Homogeneous Coomassie blue-staining bands were observed for each product and these proteins migrated with the expected molecular masses on SDS-polyacrylamide gel electrophoresis (Fig 1).

We studied the specificity and affinity of interaction of the PH domains for phosphoinositide lipids using either a "protein-lipid overlay" assay [4] (Fig 2) or the more quantitative surface plasmon resonance based approach [16] (Table 2). For the protein-lipid overlay assay, serial dilutions of phosphoinositides were spotted on to a nitrocellulose membrane and incubated with the indicated GST PH domain fusion protein or GST-DAPP1 (that binds PtdIns(3,4,5)P₃ and PtdIns(3,4)P₂ [4]), GST-GRP1 (that binds only PtdIns(3,4,5)P₃ [8]) and GST-phospholipase Cδ1 (that binds only PtdIns(4,5)P₂ (Ferguson et al (1995) Cell 83, 1037-1046)) as controls. The membranes were then washed and immunoblotted with a GST antibody to detect GST fusion proteins bound to the membrane by virtue of their interaction with lipid. For the surface plasmon resonance based assay, the apparent K_{d} values of the GST PH domain fusion proteins resulting from their interaction with a supported lipid monolayer containing a low mole fraction of phosphoinositide, was determined (Table 2). Both these assays yielded comparable results for the lipid binding specificities and relative affinities of the PH domains that we have isolated. As discussed below, 6 of the PH domains we identified, did not bind to PtdIns(3,4,5)P₃ or *sn*-1-stearoyl-2-arachidonyl-D-PtdIns(3,4,5)P₃ (data not shown), but interacted with other phosphoinositides with varying affinity and specificity. In contrast, the PH domains derived from proteins termed LL5α [17], a previously undescribed closely related isoform to LL5α which we have termed LL5β, pleckstrin-2 [18, 19], and a protein that we have called PH30, which displays 70% identity to the nuclear dual-specificity phosphatse [20] (accession number AAC39675), interacted with several phosphoinositides (Fig 2). The PH domain of a protein of unknown function, termed evectin-2, which localises to post-golgi membranes [21] showed moderate affinity for PtdIns(3,4,5)P3 but also interacted more weakly with several other phosphoinositides (Fig 2). None of the PH domains whose lipid binding properties were investigated in Fig 2, interacted with phosphatidylcholine, phosphatidylethanolamine, phosphatidylserine or phospatidylinositol in the protein-lipid overlay assay (data not shown).

**TAPP1 and TAPP2 bind specifically to PtdIns(3,4)P₂.** Two of the novel sequences identified encoded related proteins which were termed TAPP1 and TAPP2 (Table 1). Clones encoding the full length human TAPP1 (accession number AF286160) and mouse TAPP2 (accession number AF286161) as well as a partial mouse TAPP1 (accession number AF286165) and human TAPP2 (accession number AF286164), were isolated as described in the Methods section. Human TAPP1 is a protein of 404 amino acids and mouse TAPP2 is a protein of 425 amino acids (Fig 3A). A stop codon immediately 5' to the predicted initiating ATG codon indicates that both human and mouse TAPP1 and TAPP2 protein sequences are full length. Analysis of the TAPP1 and TAPP2 sequences revealed the presence in each protein of two PH domains, of which only the C-terminal PH domain possesses the PPBM (Fig 3A). Hence these proteins were termed TAPP for TAndem PH domain containing Protein. The amino acid sequences of TAPP1 and TAPP2 are 58% identical over the first 300 amino acids, which encompasses both of the PH domains. There is little homology between the C-terminal 100 residues of TAPP1 and TAPP2, except that 7 out of the 11 C-terminal amino acids of TAPP1 and TAPP2 are identical. The last 3 residues of TAPP1 and TAPP2 conform to the minimal sequence motif (Ser/Thr-Xaa-Val/Ile [22, 23]) required for binding to a PDZ domain. Apart from two proline rich regions towards the C-terminus of TAPP2, which could form a binding site for an SH3 domain (Fig 3), no other known catalytic domains are present. Interrogation of the NCBI human genome database with the TAPP1 sequence indicated that it is located on chromosome 10q25.3-q26.2. Although the genomic fragment that encompases TAPP2 (accession number AC067817) has been sequenced, its chromosomal location is not yet known.

The isolated C-terminal PH domains of TAPP1 and TAPP2 (which possess the PPBM), when expressed as GST-fusion proteins, interacted with PtdIns(3,4)P₂ but did not bind to PtdIns(3,4,5)P₃ or any other phosphoinositides tested (Fig 2). Surface plasmon resonance studies indicated that the isolated C-terminal PH domain of TAPP1 and TAPP2 interacted with PtdIns(3,4)P₂ with apparent Kd values of 5 nM and 30 nM, respectively (Table 2). The N-terminal PH domain of TAPP1 and TAPP2 failed to interact with any phosphoinositide tested (Fig 4A and Table 2). The full length GST-TAPP1 (Fig 4A and Table 2) and full length GST-TAPP2 (Fig 4C and Table 2) interacted specifically with PtdIns(3,4)P₂. Mutation of the conserved Arg212 to Leu in the PPBM of the C-terminal PH domain of TAPP1 abolished the interaction of both full length TAPP1 and the isolated C-terminal PH domain with PtdIns(3,4)P₂ (Fig 4B). Mutation to Leu of the residue (Arg28) in the N-terminal PH domain of TAPP1 that lies in the equivalent position to Arg212 in the C-terminal PH domain, did not affect the interaction of full length GST-TAPP1 with PtdIns(3,4)P₂ (Fig 4B). As expected, the mutation to Leu of the conserved Trp residue (Trp281) found in all PH domains, abolished the interaction of the isolated C-terminal PH domain of TAPP1 with PtdIns(3,4)P₂ (Fig 4B).

The tissue distribution of TAPP1 and TAPP2 mRNA was investigated by Northern blot analysis. TAPP1 was detected as a 4 kb transcript in all tissues examined with the highest levels observed in skeletal muscle, spleen, lung, thymus and placenta (Fig 3B). TAPP2 was detected as a 6 kb transcript in all tissues examined with the highest levels observed in heart and kidney (Fig 3B). We identified many ESTs encoding TAPP1 and TAPP2 in the databases derived from several tissues (Table 3), indicating that TAPP1 and TAPP2 are widely expressed proteins.

**FAPP1 is a specific PtdIns4P binding protein.** The identified PH domain termed FAPP1 (Table 1), possessing Gln instead of Lys or Arg at the third conserved residue of the PPBM, exhibited a high affinity for PtdIns4P (K_{d} 20 nM), but did not bind to any other phosphoinositide (Fig 2 & Table2). The full length human and mouse FAPP1 sequences (Fig 5) were deduced from the sequencing of ESTs listed in Table 3. Human FAPP1 encodes a protein of 300 amino acids and a stop codon immediately 5' to the predicted initiating ATG codon indicates that both the human and mouse FAPP1 protein sequences are full length. Interrogation of the human genome NCBI database indicated that the FAPP1 gene was located on an unmapped region of chromosome 2 (accession number NT_003398). Analysis of the FAPP1 sequence revealed the presence of an N-terminal PH domain and a proline rich region located towards the C-terminus that could mediate binding to SH3 domains (Fig 5). FAPP1 is likely to be expressed widely, because 27 EST clones encoding this protein were derived from several tissues (Table 3). However, FAPP1 may not be an abundant transcript as we were unable to detect significant levels of FAPP1 mRNA expression in any tissue or cell line examined (data not shown).

FAPP2 also binds specifically to PtdIns4P.

**Plant AtPH1 and mammalian PEPP bind PtdIns3P specifically.** Two of the PH domains that were identified, termed AtPH1 and PEPP1 (Table 1), exhibited significant affinity for PtdIns3P (K_{d} of 325 nM), but did not bind to any other phosphoinositide (Fig 2 and Table 2). AtPH1 is a small 145 residue *Arabidopsis* protein, whose physiological role is unknown. It consists of one PH domain with a short N-terminal extension and is expressed in all plant tissues [24]. PEPP1 is a novel mammalian protein, whose partial sequence (Fig 6A) and full length sequence (Fig 8A) we have deduced from sequencing of several ESTs (Table 3). The partial sequence is likely to comprise the N-terminal end of PEPP1 as there is an in-frame stop codon 5' to the predicted initiating ATG codon. The PH domain of PEPP1 is located at the N-terminal region of PEPP1. There are also 2 proline rich regions that could comprise SH3 binding sites. Analysis of the NCBI human genome database shows that the PEPP1 gene is located on an unmapped region of chromosome 19 (accession number AC026803). The tissue distribution of PEPP1 mRNA was first investigated by Northern blot analysis, which indicated that PEPP1 was either not expressed or only expressed to a very low level in the panel of 12 tissues that we examined (Fig 6B). We also carried out a Northern blot analysis using a panel of 8 different human cancer cell lines (Fig 6B). Interestingly, PEPP1 mRNA was expressed at very high levels in a melanoma cancer cell line as a 3 kb fragment, but was not significantly expressed in the other 7 non-melanoma cancer cell lines that were investigated (Fig 6B). Further evidence which suggests that PEPP1 may be selectively expressed in melanoma or melanocytes is that the three human EST clones encoding PEPP1 that we have identified thus far are derived from either a melanoma or a melanocyte cDNA library (Table 3).

Interrogation of the NCBI database with the PEPP1 sequence revealed 2 other proteins that appear to be related isoforms of PEPP1 termed PEPP2 and PEPP3. The identity between these proteins is most notable in the PH domain, especially in the region that encompasses the PPBM as well as a region of 30 amino acids that precedes the PH domain. PEPP1, PEPP2 and PEPP3 are poorly conserved in the region C-terminal to the PH domain (Fig 8A). PEPP2, but not PEPP1 or PEPP3 also possesses two WW domains (Rotin (1998) Curr Top Microbiol Immunol 228, 115-133) in a region N-terminal to the PH domain (Fig 8A). PEPP2 may be more widely expressed than PEPP1 as Northern Blot analysis shows that PEPP2 mRNA is present in high levels in heart and kidney and also expressed at a lower level in other tissues. PEPP3 may not be an abundant transcript as we were unable to detect significant levles of PEPP3 mRNA expression in any tissue or cell line examined (data not shown). The four PEPP3 ESTs that are present in the database are derived from brain, colon, mammary gland and skeletal muscle (see methods). PEPP2 and PEPP3 are also considered to bind PtdIns3P.

**Centaurin-**β**2 is a PtdIns(3,5)P₂ binding protein.** Human centaurin-β2 is an uncharacterised 778 amino acid protein (cloned by T. Jackson and colleagues, University College London, accession number CAB41450), possessing a PH domain (residues 267-363) followed by a putative ARF GAP domain (residues 399-520) and three ankyrin repeats at its C-terminus. The PH domains of both mouse and human centaurin-β2 possess Asn instead of a Lys or Arg at the third conserved residue of the PPBM (Table 1). The PH domain of mouse centaurin-β2 exhibited moderate affinity for PtdIns(3,5)P₂ but did not bind to any other phosphoinositide tested (Fig 2). Centaurin-β2 is likely to be a widely expressed protein as 12 EST clones encoding it were derived from several tissues and Northern blot analysis indicated that mouse centaurin-β2 was expressed as a 4.5 kb fragment in all tissues investigated (data not shown).

### Discussion

The PH domains identified thus far that bind specifically to PtdIns(3,4,5)P₃, or to PtdIns(3,4,5)P₃ and PtdIns(3,4)P₂, possess a PPBM (Table 1). However, the finding in this study that PH domains possessing a perfect or near perfect PPBM consensus, do not always interact with PtdIns(3,4,5)P₃ specifically, emphasises that residues lying outside the PPBM also influence the interaction of many PH domains with phosphoinositides. It therefore seems unlikely that it will be possible to predict the lipid binding specificity of a PH domain based on its primary amino acid sequence alone. This is consistent with structural studies showing that residues lying outside of the PPBM also form direct contacts with the inositol phosphate moieties of phosphoinositides [12, 25]. Previous studies have demonstrated that PLCδ₁ which also possesses a PPBM, does not bind to PtdIns(3,4,5)P₃ with high affinity [25]. It has been proposed that, in this case, the short loop between the β1 and β 2 strands of the PH domain of PLCδ₁ compared to that found in other PH domains that bind to PtdIns(3,4,5)P₃, may account for this observation [25].

There has been considerable debate as to whether PtdIns(3,4)P₂ regulates the same physiological processes as PtdIns(3,4,5)P₃, as it is formed as a breakdown product of PtdIns(3,4,5)P₃ and many of the PH domains that interact with PtdIns(3,4,5)P₃ also bind to PtdIns(3,4)P₂ (as discussed in the introductory section above). However, the finding that agonists such as hydrogen peroxide, [26] and crosslinking of platelet integrin receptors [27], elevate PtdIns(3,4)P₂ without increasing PtdIns(3,4,5)P₃, suggest that PtdIns(3,4)P₂ may be able to regulate physiological processes distinct from those controlled by PtdIns(3,4,5)P₃. TAPP1 and TAPP2 (Fig 3) are the first proteins to be identified that interact with PtdIns(3,4)P₂ specifically and may therefore be key mediators of cellular responses that are regulated specifically by this second messenger. Although, there are no apparent homologues of TAPP1 and TAPP2 present in the completed genome of *Drosophila, C.elegans* or *S. cerevisiae,* there are ESTs encoding a TAPP1 homologue derived from zebrafish and chicken (Table 3). Further studies are required to characterise the physiological role of TAPP1 and TAPP2, but it is possible that they function as adaptor proteins to recruit proteins that interact with them to cellular membranes in response to extracellular signals that lead to the generation of PtdIns(3,4)P₂. However, it is possible that the *in vitro* lipid binding properties of TAPP1 and TAPP2, as well as the other PH domain containing proteins that we have characterised in this study, could differ from their *in vivo* binding specificities. It is also possible that the inositol polyphosphate head groups of the phosphoinositides, rather than the phosphoinositides themselves, could be the natural ligands for these proteins. The N-terminal PH domain of TAPP1 and TAPP2, rather than interacting with lipids, may mediate protein-protein interactions as they did not interact with any phosphoinositide that we tested (Fig 4A). TAPP1 and TAPP2 could also potentially interact with proteins containing PDZ domains through their C-terminal Ser-Xaa-Val residues and TAPP2 could bind to SH3 domains through two proline rich motifs located towards its C-terminus.

To our knowledge, the only PH domain previously shown to bind PtdIns4P with some specificity is derived from a plant PtdIns 4-kinase which also interacts weakly with PtdIns(4,5)P₂ [28]. In contrast, FAPP1 (Fig 5) only binds PtdIns 4P and does not interact with PtdIns(4,5)P₂ (Fig 2, Table 2). A key role of PtdIns 4P in mammalian cells is to act as an intermediate in the synthesis of PtdIns(4,5)P₂. Apart from a PH domain and a putative SH3-binding proline-rich motif, FAPP1 does not possess a catalytic domain that would indicate a role in regulating the synthesis or breakdown of PtdIns4P in cells. There are no apparent homologues of FAPP1 in *Drosophila, C.elegans* or *S. cerevisiae*; however ESTs encoding FAPP1 have been identified in zebrafish and *Xenopus* (Fig 5 and Table 3).

Genetic studies carried out in yeast have demonstrated that PtdIns3P plays an important role in regulating golgi to vacuole or lysosome membrane trafficking as well as endosome function [29]. Several proteins (e.g. EEA1) regulating these processes have been found to interact with PtdIns3P through a particular type of Zinc finger domain (known as the FYVE domain) [30]. To our knowledge the only other PH domain-containing protein other than PEPP1 and AtPH1, previously reported to interact with PtdIns3P is phospholipase Cβ1[31]. However phospholipase Cβ1 may be less specific for PtdIns3P than PEPP1 and AtPH1, as it also possessed significant affinity for PtdIns(4,5)P₂ and PtdIns(3,4,5)P₃ [31]. The evidence indicates that phospholipase Cβ1 may be recruited to plasma membranes through an interaction of its PH domain with both PtdIns 3P (or other phosphoinositide) and the Gβγ regulatory subunits [31, 32].

A potentially interesting feature of PEPP1, is that its expression may be restricted to melanoma and or melanocytes as Northern blot analysis indicated that PEPP1 was expressed at very high levels in a melanoma cell line, but not in 7 other non-melanoma cancer cell lines or 12 tissues that were investigated (Fig 6B). Further work is required to determine whether PEPP1 expression is elevated in all melanoma cells compared to normal melanocytes. It is interesting that a closely related homologue of PEPP1, termed PEPP2, appears to be more widely expressed (Fig 8B). PEPP2 and PEPP3 possess a very similar sequence surrounding the PPBM of their PH domains indicating that they may also interact with PtdIns3P.

Plant cells contain high levels of PtdIns3P as well as PtdIns(3,4)P₂ but no PtdIns(3,4,5)P₃ has been detected [33], consistent with the apparent lack of Class 1A PI 3-kinases in plants. AtPH1 is the first plant protein that has been shown to interact with PtdIns3P and may play an important role as an adaptor protein in regulating signalling processes in plants that are mediated by PtdIns3P. There are no apparent homologues of PEPP1 or AtPH1 in *Drosophila, C.elegans* or *S. cerevisiae.*

The ARF family of GTP binding proteins regulate membrane trafficking and the actin cytoskeleton [34]. A family of ARF GAP proteins, collectively termed centaurins, have been identified and all possess one or more PH domains and an ARF GAP catalytic domain [35]. The PH domain on centaurin-α1 interacts with PtdIns(3,4,5)P₃ and centaurin-α1 is recruited to cell membranes after PI 3-kinase is activated [7]. Recently centaurin-β4 has been shown to be activated by the interaction of its PH domain with PtdIns(4,5)P₂ and, in contrast to centaurin-α1, does not bind to PtdIns(3,4,5)P₃ [36]. The finding in this paper that the uncharacterised ARF GAP protein named centaurin-β2 interacts with PtdIns(3,5)P₂ , albeit with moderate affinity, suggests that centaurin-β2 may be regulated by this lipid. Further investigation is required to establish whether PtdIns(3,5)P₂ can lead to the activation of centaurin-β2. No protein has previously been shown to interact specifically with PtdIns(3,5)P₂ and the physiological processes regulated by this lipid are not known. In yeast, PtdIns(3,5)P₂ is generated in response to osmotic stress [37] by phosphorylation of PtdIns3P at the D5 position by a kinase termed Fab1[38, 39]. There are putative homologues of centaurin-β2 in *Drosophila* (accession number 7595986) and *C.elegans* (accession number 4225944) which possess about 30% overall identity to human centaurin-β2.

In summary, this Example describes a group of novel PH domain containing proteins that possess interesting phosphoinositide binding specificities. TAPP1, TAPP2, FAPP1 and AtPH1 may function as adaptor molecules as they possess no obvious catalytic moieties. In order to further define the physiological processes that are regulated by the PH domain-containing proteins described in this paper it may not only be important to knock out these proteins in cells and mice but also to identify the proteins that they interact with.

### References

1 Leevers, S. J., Vanhaesebroeck, B. and Waterfield, M. D. (1999) Signalling through phosphoinositide 3-kinases: the lipids take centre stage. Curr Opin Cell Biol 11, 219-25
2 Vanhaesebroeck, B. and Alessi, D. R. (2000) The PI3K-PDK1 connection: more than just a road to PKB. Biochem J 346, 561-576
3 Li, Z., Wahl, M. I., Eguinoa, A., Stephens, L. R., Hawkins, P. T. and Witte, O. N. (1997) Phosphatidylinositol 3-kinase-gamma activates Bruton's tyrosine kinase in concert with Src family kinases. Proc Natl Acad Sci U S A 94, 13820-5
4 Dowler, S., Currie, R. A., Downes, C. P. and Alessi, D. R. (1999) DAPP1: a dual adaptor for phosphotyrosine and 3-phosphoinositides [In Process Citation]. Biochem J 342, 7-12
5 Dowler, S., Montalvo, L., Cantrell, D., Morrice, N. and Alessi, D. R. (2000) Phosphoinositide 3-kinase-dependent phosphorylation of the dual adaptor for phosphotyrosine and 3-phosphoinositides by the Src family of tyrosine kinase. Biochem J 349, 605-610
6 Rodrigues, G. A., Falasca, M., Zhang, Z., Ong, S. H. and Schlessinger, J. (2000) A novel positive feedback loop mediated by the docking protein Gab1 and phosphatidylinositol 3-kinase in epidermal growth factor receptor signaling. Mol Cell Biol 20, 1448-59
7 Venkateswarlu, K., Oatey, P. B., Tavare, J. M., Jackson, T. R. and Cullen, P. J. (1999) Identification of centaurin-alphal as a potential in vivo phosphatidylinositol 3,4,5-trisphosphate-binding protein that is functionally homologous to the yeast ADP-ribosylation factor (ARF) GTPase-activating protein, Gcsl. Biochem J 340, 359-63
8 Gray, A., Van Der Kaay, J. and Downes, C. P. (1999) The pleckstrin homology domains of protein kinase B and GRP1 (general receptor for phosphoinositides-1) are sensitive and selective probes for the cellular detection of phosphatidylinositol 3,4-bisphosphate and/or phosphatidylinositol 3,4,5-trisphosphate in vivo. Biochem J 344, 929-36
9 Klarlund, J. K., Rameh, L. E., Cantley, L. C., Buxton, J. M., Holik, J. J., Sakelis, C., Patki, V., Corvera, S. and Czech, M. P. (1998) Regulation of GRP1-catalyzed ADP ribosylation factor guanine nucleotide exchange by phosphatidylinositol 3,4,5-trisphosphate. J Biol Chem 273, 1859-62
10 Isakoff, S. J., Cardozo, T., Andreev, J., Li, Z., Ferguson, K. M., Abagyan, R., Lemmon, M. A., Aronheim, A. and Skolnik, E. Y. (1998) Identification and analysis of PH domain-containing targets of phosphatidylinositol 3-kinase using a novel in vivo assay in yeast. EMBO J 17, 5374-87
11 Fruman, D. A., Rameh, L. E. and Cantley, L. C. (1999) Phosphoinositide binding domains: embracing 3-phosphate. Cell 97, 817-20
12 Baraldi, E., Carugo, K. D., Hyvonen, M., Surdo, P. L., Riley, A. M., Potter, B. V., O'Brien, R., Ladbury, J. E. and Saraste, M. (1999) Structure of the PH domain from Bruton's tyrosine kinase in complex with inositol 1,3,4,5-tetrakisphosphate. Structure Fold Des 7, 449-60
13 Lennon, G., Auffray, C., Polymeropoulos, M. and Soares, M. B. (1996) The I.M.A.G.E. Consortium: an integrated molecular analysis of genomes and their expression. Genomics 33, 151-2
14 Alessi, D. R., Andjelkovic, M., Caudwell, B., Cron, P., Morrice, N., Cohen, P. and Hemmings, B. A. (1996) Mechanism of activation of protein kinase B by insulin and IGF-1. EMBO J 15, 6541-51
15 Deak, M., Casamayor, A., Currie, R. A., Downes, C. P. and Alessi, D. R. (1999) Characterisation of a plant 3-phosphoinositide-dependent protein kinase- 1 homologue which contains a pleckstrin homology domain. FEBS Lett 451, 220-6
16 Currie, R. A., Walker, K. S., Gray, A., Deak, M., Casamayor, A., Downes, C. P., Cohen, P., Alessi, D. R. and Lucocq, J. (1999) Role of phosphatidylinositol 3,4,5-trisphosphate in regulating the activity and localization of 3-phosphoinositide-dependent protein kinase-1. Biochem J 337, 575-83
17 Levi, L., Hanukoglu, I., Raikhinstein, M., Kohen, F. and Koch, Y. (1993) Cloning of LL5, a novel protein encoding cDNA from a rat pituitary library. Biochim Biophys Acta 1216, 342-4
18 Hu, M. H., Bauman, E. M., Roll, R. L., Yeilding, N. and Abrams, C. S. (1999) Pleckstrin 2, a widely expressed paralog of pleckstrin involved in actin rearrangement. J Biol Chem 274, 21515-8
19 Inazu, T., Yamada, K. and Miyamoto, K. (1999) Cloning and expression of pleckstrin 2, a novel member of the pleckstrin family. Biochem Biophys Res Commun 265, 87-93
20 Cui, X., De Vivo, I., Slany, R., Miyamoto, A., Firestein, R. and Cleary, M. L. (1998) Association of SET domain and myotubularin-related proteins modulates growth control [see comments]. Nat Genet 18, 331-7
21 Krappa, R., Nguyen, A., Burrola, P., Deretic, D. and Lemke, G. (1999) Evectins: vesicular proteins that carry a pleckstrin homology domain and localize to post-Golgi membranes. Proc Natl Acad Sci U S A 96, 4633-8
22 Kornau, H. C., Schenker, L. T., Kennedy, M. B. and Seeburg, P. H. (1995) Domain interaction between NMDA receptor subunits and the postsynaptic density protein PSD-95. Science 269, 1737-40
23 Songyang, Z., Fanning, A. S., Fu, C., Xu, J., Marfatia, S. M., Chishti, A. H., Crompton, A., Chan, A. C., Anderson, J. M. and Cantley, L. C. (1997) Recognition of unique carboxyl-terminal motifs by distinct PDZ domains. Science 275, 73-7
24 Mikami, K., Takahashi, S., Katagiri, T., Shinozaki, K. Y. and Shinozaki, K. (1999) Isolation of an Arabidopsis thaliana cDNA encoding a pleckstrin homology domain protein, a putative homologue of human pleckstrin. J. Exp. Bot. 50, 729-730.
25 Ferguson, K. M., Lemmon, M. A., Schlessinger, J. and Sigler, P. B. (1995) Structure of the high affinity complex of inositol trisphosphate with a phospholipase C pleckstrin homology domain. Cell 83, 1037-46
26 Van der Kaay, J., Beck, M., Gray, A. and Downes, C. P. (1999) Distinct phosphatidylinositol 3-kinase lipid products accumulate upon oxidative and osmotic stress and lead to different cellular responses. J Biol Chem 274, 35963-8
27 Banfic, H., Tang, X., Batty, I. H., Downes, C. P., Chen, C. and Rittenhouse, S. E. (1998) A novel integrin-activated pathway forms PKB/Akt-stimulatory phosphatidylinositol 3,4-bisphosphate via phosphatidylinositol 3- phosphate in platelets. J Biol Chem 273, 13-6
28 Stevenson, J. M., Perera, I. Y. and Boss, W. F. (1998) A phosphatidylinositol 4-kinase pleckstrin homology domain that binds phosphatidylinositol 4-monophosphate. J Biol Chem 273, 22761-7
29 Wurmser, A. E., Gary, J. D. and Emr, S. D. (1999) Phosphoinositide 3-kinases and their FYVE domain-containing effectors as regulators of vacuolar/lysosomal membrane trafficking pathways. J Biol Chem 274, 9129-32
30 Stenmark, H. and Aasland, R. (1999) FYVE-finger proteinseffectors of an inositol lipid. J Cell Sci 112, 4175-83
31 Razzini, G., Brancaccio, A., Lemmon, M. A., Guarnieri, S. and Falasca, M. (2000) The role of the pleckstrin homology domain in membrane targeting and activation of phospholipase Cβ1. J Biol Chem 275, 14873-81
32 Wang, T., Pentyala, S., Rebecchi, M. J. and Scarlata, S. (1999) Differential association of the pleckstrin homology domains of phospholipases C-β1, C-β 2, and C-δ1 with lipid bilayers and the β γ subunits of heterotrimeric G proteins. Biochemistry 38, 1517-24
33 Munnik, T., Irvine, R. F. and Musgrave, A. (1998) Phospholipid signalling in plants. Biochim Biophys Acta 1389, 222-72
34 Chavrier, P. and Goud, B. (1999) The role of ARF and Rab GTPases in membrane transport. Curr Opin Cell Biol 11, 466-75
35 Randazzo, P. A., Andrade, J., Miura, K., Brown, M. T., Long, Y. Q., Stauffer, S., Roller, P. and Cooper, J. A. (2000) The ARF GTPase-activating protein ASAP1 regulates the actin cytoskeleton [In Process Citation]. Proc Natl Acad Sci U S A 97, 4011-6
36 Kam, J. L., Miura, K., Jackson, T. R., Gruschus, J., Roller, P., Stauffer, S., Clark, J., Aneja, R. and Randazzo, P. A. (2000) Phosphoinositide-dependent activation of the ADP-ribosylation factor GTPase-activating protein ASAP1. Evidence for the pleckstrin homology domain functioning as an allosteric site. J Biol Chem 275, 9653-63
37 Dove, S. K., Cooke, F. T., Douglas, M. R., Sayers, L. G., Parker, P. J. and Michell, R. H. (1997) Osmotic stress activates phosphatidylinositol-3,5-bisphosphate synthesis [see comments]. Nature 390, 187-92
38 Odorizzi, G., Babst, M. and Emr, S. D. (1998) Fablp PtdIns(3)P 5-kinase function essential for protein sorting in the multivesicular body. Cell 95, 847-58
39 Cooke, F. T., Dove, S. K., McEwen, R. K., Painter, G., Holmes, A. B., Hall, M. N., Michell, R. H. and Parker, P. J. (1998) The stress-activated phosphatidylinositol 3-phosphate 5-kinase Fablp is essential for vacuole function in S. cerevisiae. Curr Biol 8, 1219-22

### Example 2: Identification of interacting polypeptides

Polypeptides interacting with TAPP1, TAPP2, PEPP1, PEPP2, PEPP3 or FAPP (for example FAPP1 or FAPP2) are identified using yeast two hybrid methods and/or immunoprecipitation/coprecipitation methods. The methods are performed on stimulated and unstimulated cells; polypeptides that interact with TAPP1, TAPP2, PEPP1, PEPP2, PEPP3 or FAPP (for example FAPP1 or FAPP2) in one cell state only (or to different extents in the different cell states) are of particular interest. The methods may also be performed (for comparison) with mutated TAPP1, TAPP2, PEPP1, PEPP2, PEPP3 or FAPP polypeptides, for example mutants which do not bind the relevant phosphoinositide. Coprecipitated polypeptides are analysed by microsequencing and mass spectrometry. The amino acid sequence information is used to identify/isolate polynucleotides encoding the amino acid sequence, using standard molecular biology techniques.

### Example 3: Phosphoinositide detection and enzyme assays

Particular enzymes, such as particular lipid phosphatases or inositol lipid kinases, may be assayed using the PH domains described herein, for example using TAPP1, TAPP2, PEPP1, PEPP2, PEPP3 or FAPP (for example FAPP1 or FAPP2). The assay system makes use of the ability of the PH domains to bind specifically to PtdIns(3,4)P₂, PtdIns3P, PtdIns4P or PtdIns(3,5)P₂ but not capable of binding to PtdIns(3,4,5)P₃, when the phosphoinositide is the product (or substrate) of a lipid kinase or phosphatase reaction. The PH domain may be used as a recombinant protein fused to a reporter tag such as a green fluorescent protein or labelled with a fluorescent chromophore.

For example, a Class II PI3 kinase may generate PtdIns3P, which may be measured using PEPP or AtPH1. A PI4 kinase generates PtdIns4P, which may be measured using FAPP. Fablp[38, 39] generate Ptd(3,5)P₂, which may be measured using centaurin-β2. Alternatively, changes in the substrate for an enzyme may be measured. For example, Fablp converts PtdIns3P to PtdIns(3,5)P₂ and a PH domain which binds to PtdIns3P (for example the PH domain of PEPP1 or AtPH1) may be used to monitor the level of PtdIns3P and thereby Fablp activity.

The group of 5' phosphatases target PtdIns(3,4,5)P₃ and also PtdIns4,5P₂, to yield PtdIns4P. Thus, FAPP may be used in measuring such 5' phosphatase activity. FAPP may also be useful in monitoring a 4' phosphatase, for example Saclp from yeast and homologues thereof, which appears to be specific for dephosphorylating PtdIns4P to phosphoinositide (see, for example, Hughes et al (2000) Biochem J 350(2), 337-352; Nemoto et al (2000) J Biol Chem 275(44), 34293-24305 (rat homologue); Hughes et al (2000) J Biol Chem 275(2), 801-808).

A FRET (fluorescence resonance energy transfer) system may be used. A soliud phase assay with the substrate lipid bound to the surface of a microtitre plate may be used. PH domain binding to the product formed in the immobilised lipid layer is detected by time resolved FRET.

For example, substrate lipids in a lipid layer incorporating a donor chromophore immobilised in wells of a 96 well microtitre plate are incubated with the appropriate enzyme (or sample to be tested for the appropriate enzyme) in the presence of the appropriate recombinant PH domain fused to green fluorescent protein (GFP; including mutant GFPs, as discussed above) and ATP. The PH-GFP binds specifically to the product (or in an alternative, the substrate) and in doing so is brought into close enough proximity with the chromophore in the lipid layer for FRET to occur. This may be detected using methods well known to those skilled in the art.

This system does not use radioisotopes; does not require separation of reaction products, allowing the system to be used in high throughput screens; does not use lipid vesicles, thereby reducing "false positives" in inhibitor screens due to vesicle disruption by the test compound; and may be used for several enzymes, depending on the lipid and PH domains chosen.

The system may be used for making real time measurements throughout the curse of the reaction. Other methods (for example using radioisotopes) may be suitable only for taking measurements at predetermined time points. This may make the present assay system more informative and easier to operate, for example because changes in the activity of the enzyme preparation can be more easily compensated for, for example by making measurements over a shorter or longer period depending on the level of activity of the enzyme, as well known to the skilled person.

In alternative arrangements, the PH domain may be "tagged" in other ways, for example with an alternative chromophore, an epitope tag or a detectable enzyme, as well known in interaction assays, for example immunoassays.

For example, the PH domain may be in the form of a GST fusion protein labelled with a terbium chelate (Terbium Lance Chelate, LKB Wallac) as energy donor and rhodamine labeleld phosphatidylethanolamine as energy acceptor.

It may not be necessary to tag the PH domain. The intrinsic fluorescence of tryptophan residues in the PH domain may change on binding to the phosphoinositide, and this may be used in monitoring the binding of the PH domain to the phosphoinositide, and thereby determining the amount of phosphoinositide present.

The assay configuration may consist of a microtitre plate coated with a mixture containing the substrate phosphoinositide, for example 0.8nmols, phosphatidylserine, 0.7nmols, and rhodamine labelled phosphatidylethanolamine, 01.5nmmols, giving a total of 2nmols lipid per well. The PH-GST terbium chelate is used at a concentration of 0.175µg/ml in a final volume of 50µl. In order to test the system, a well may be "spiked" with the product lipid at various concentrations. The labelled PH domain is added to the plate and time resolved measurements of fluorescence are taken. For example, excitation at 340nm, emission at 601 nm and a time gate of 50 to 800µsec may be used. Detection limits are in the low pmol range.

Enzyme activity can be determined by measuring fluroescence over time. The enzyme or sample is added with ATP (for example 0.1mM ATP). Data points may be the mean of measurements of several wells (for example eight) read at 30 second intervals over 30 minutes.

In a further alternative, the assay may be run as a homogenous fluid phase assay with the substrate lipid either in free solution or as lipid vesicles. The fluid phase assay relies on reaction product competing for binding in a preformed detection complex. The complex may be formed, for example, between Europium lance chelate labelled GST-PH domain, biotinylated short chain phosphoinositide (for example C6 product phosphoinositide) and streptavidin labelled allophycocyanin (APC). Enzyme activity is detected by the conversion of nonbiotinylated short chain substrate phosphoinositide to product phosphoinositide, which competes for binding with the GST-PH domain in the preformed complex, resulting in a decrease in the FRET signal. The system may be tested by adding biotinylated synthetic short chain product to the assay system. The assay may contain 1µl APC (for example 0.01 to 100 µg, preferably 0.1 to 10 µg), 1µl of the Europium labelled GST-PH domain (for example 0.01 to 100 µg, preferably 0.1 to 10 µg) and increasing concentrations (for example from 0 to 300 pmol) of the water soluble biotinylated short chain product phosphoinositide in a final volume of 50µl. An excitation wavelength of 340, emission wavelength of 665nm and cut-off of 630 nm may be used.

In the assay, non-biotinylated product phosphoinositide produced from the substrate phosphoinositide competes for binding to the GST-PH domain, reducing the observed signal. The system may be tested by addition of increasing amounts of non-biotinylated product phosphoinositide. The biotinylated product phosphoinositide may be present at 0.5µM (25pmol/assay).

A typical assay set-up may be as follows:
Buffer: 50mM HEPES pH7.4, 5mM DTT, 3.5mM MgCl₂, 0.02% CHAPS and 250µM ATP.
Detector mix: Eu chelate GST-PH domain (for example 0.01 to 100 µg, preferably 0.1 to 10 µg), streptavidin APC (for example 0.01 to 100 µg, preferably 0.1 to 10 µg), and biotinylated product phosphoinositide 0.5µM.
Enzyme: recombinant enzyme, for example at about 10 ng to 10 µg/ml.
The fluorimeter settings may be excitation 340nm, emission 665 nm, filter 630 nm, time gate 50 to 1050µsec.
The water soluble substrate phosphoinositide may be used at a concentration of 25 µM. The final assay volume may be 50µl.

The rate of decrease of time resolved FRET may be measured over 30 minutes at 30 sec intervals over a range of substrate phosphoinositide concentrations (for example 0 to 70µM) and the initial rates estimated.

As an alternative, the interaction of the components of an assay may be detected using the Alpha Screen™ bead system from BioSignal Packard (part of Packard Biscience), of 1744 rue William, Suite 600, Montreal, Quebec, Canada, H3J 1R4.

## Claims

1. A composition comprising a polypeptide capable of binding specifically to PtdIns(3,4)P₂ but not capable of binding to PtdIns(3,4,5)P₃, wherein the polypeptide comprises a PH domain capable of binding to PtdIns(3,4)P₂ but not capable of binding to PtdIns(3,4,5)P₃, wherein the polypeptide comprises the amino acid sequence 
MPYVDRQNRICGFLDIEEHENSGKFLRRYFILDTQANCLLWYMDNPQ
NLAMGAGAVGALQLTYISKVSIATPKQKPKTPFCFVINALSQRYFLQAND
QKDMKDWVEALNQASKITVPKGGGLPMTTEVLKSLAAPPALEKKPQVAYK
TEIIGGVVVHTPISQNGGDGQEGSEPGSHTILRRSQSYIPTSGCRASTGP
PLIKSGYCVKQGNVRKSWKRRFFALDDFTICYFKCEQDREPLRTIFFKDV
LKTHECLVKSGDLLMRDNLFEIITSSRTFYVQADSPEDMHSWIKEIGAAV
QALKCHP (partial human TAPP2 amino acid sequence)
 or 
MPYVDRQNRICGFLDIEDNENSGKFLRRYFILDTQANCLLWYMDNPQNLA
VGAGAVGSLQLTYISKVSIATPKQKPKTPFCFVINALSQRYFLQANDQKD
LKDWVEALNQASKITVPKAGTVPLATEVLKNLTAPPTLEKKPQVAYKTEI
IGGVVVQTPISQNGGDGQEGCEPGTHAFLRRSQSYIPTSGCRPSTGPPLI
KSGYCVKQGNVRKSWKRRFFALDDFTICYFKCEQDREPLRTIPLKDVLKT
HECLVKSGDLLMRDNLFEIITTSRTFYVQADSPEDMHSWIEGIGAAVQAL
KCHPREPSFSRSISLTRPGSSTLTSAPNSILSRRRPPAEEKRGLCKAPSV
ASSWQPWTPVPQAEEKPLSVEHAPEDSLFMPNPGESTATGVLASSRVRHR
SEPQHPKEKPFVFNLDDENIRTSDV (mouse TAPP2 amino acid sequence)
 or comprises a fragment thereof, said fragment being capable of binding specifically to PtdIns(3,4)P₂ but not capable of binding to PtdIns(3,4,5)P₃, or wherein the polypeptide comprises a fusion of a said fragment thereof, wherein the composition comprises a protein content that consists of at least 50% by weight of said polypeptide.

2. A recombinant polynucleotide suitable for expressing a polypeptide as defined in Claim 1.

3. A vector suitable for replication in a mammalian/eukaryotic cell comprising a polynucleotide encoding the polypeptide as defined in Claim 1.

4. A polynucleotide or vector according to Claim 2 or 3 which contains no introns.

5. A host cell comprising a recombinant polynucleotide or a replicable vector as defined in any one of Claims 2 to 4.

6. A method of making a polypeptide as defined in Claim 1 the method comprising culturing a host cell as defined in Claim 5 which expresses said polypeptide, and isolating said polypeptide.

7. The method of Claim 6 wherein the said host cell is a eukaryotic cell.

8. An antibody reactive towards a polypeptide having the amino acid sequence 
MPYVDRQNRICGFLDIEEHENSGKFLRRYFILDTQANCLLWYMDNPQ
NLAMGAGAVGALQLTYISKVSIATPKQKPKTPFCFVINALSQRYFLQAND
QKDMKDWVEALNQASKITVPKGGGLPMTTEVLKSLAAPPALEKKPQVAYK
TEIIGGVVVHTPISQNGGDGQEGSEPGSHTILRRSQSYIPTSGCRASTGP
PLIKSGYCVKQGNVRKSWKRRFFALDDFTICYFKCEQDREPLRTIFFKDV
LKTHECLVKSGDLLMRDNLFEIITSSRTFYVQADSPEDMHSWIKEIGAAV
QALKCHP (partial human TAPP2 amino acid sequence)
 or 
MPYVDRQNRICGFLDIEDNENSGKFLRRYFILDTQANCLLWYMDNPQNLA
VGAGAVGSLQLTYISKVSIATPKQKPKTPFCFVINALSQRYFLQANDQKD
LKDWVEALNQASKITVPKAGTVPLATEVLKNLTAPPTLEKKPQVAYKTEI
IGGVVVQTPISQNGGDGQEGCEPGTHAFLRRSQSYIPTSGCRPSTGPPLI
KSGYCVKQGNVRKSWKRRFFALDDFTICYFKCEQDREPLRTIPLKDVLKT
HECLVKSGDLLMRDNLFEIITTSRTFYVQADSPEDMHSWIEGIGAAVQAL
KCHPREPSFSRSISLTRPGSSTLTSAPNSILSRRRPPAEEKRGLCKAPSV
ASSWQPWTPVPQAEEKPLSVEHAPEDSLFMPNPGESTATGVLASSRVRHR
SEPQHPKEKPFVFNLDDENIRTSDV (mouse TAPP2 amino acid sequence),
 wherein the antibody does not react with a different polypeptide comprising a PH domain.

9. The use of a polypeptide as defined in Claim 1 in a screening method for identifying a compound suitable for modulating signalling by PtdIns(3,4)P₂.

10. The use according to Claim 9 wherein the method comprises the steps of (1) exposing the said polypeptide to PtdIns(3,4)P₂ in the presence of a test compound; (2) determining whether the test compound modulates binding of PtdIns(3,4)P₂ to the said polypeptide; and (3) selecting a compound which modulates binding of PtdIns(3,4)P₂ to the said polypeptide.

11. A method of identifying a compound that modulates the phospholipid binding activity of a polypeptide as defined in Claim 1, the method comprising contacting a compound with the said polypeptide and determining whether the phospholipid binding activity of the said polypeptide is changed in the presence of the compound from that in the absence of said compound.

12. The method according to Claim 11 in which the said binding activity is decreased.

13. The method according to Claim 11 in which the said binding activity is increased.

14. The method of claim any one of Claims 11 to 13 wherein the said method is performed in a cell.

15. A method of detecting and/or quantifying PtdIns(3,4)P₂ in a sample wherein the sample is exposed to a polypeptide as defined in Claim 1 and the binding of the said polypeptide to any PtdIns(3,4)P₂ present is detected.

16. A method according to Claim 15 wherein the method is performed in cells.

17. A method according to any one of Claims 15 to 16 wherein the said polypeptide comprises a chromophore.

18. A kit of parts useful in carrying out a method according to any one of Claims 16 to 17 comprising a polypeptide as defined in Claim 1.

## Patentansprüche

1. Zusammensetzung mit einem Polypeptid, das sich spezifisch an PtdIns(3,4)P₂ binden kann, sich aber nicht an PtdIns(3,4,5)P₃ binden kann, wobei das Polypeptid eine PH-Domäne umfasst, die sich an PtdIns(3,4)P₂ binden kann, sich aber nicht an PtdIns(3,4,5)P₃ binden kann, wobei das Polypeptid die folgende Aminosäuresequenz umfasst: 
MPYVDRQNRICGFLDIEEHENSGKFLRRYFILDTQANCLLWYMDNPQ
NLAMGAGAVGALQLTYISKVSIATPKQKPKTPFCFVINALSQRYFLQAND
QKDMKDWVEALNQASKITVPKGGGLPMTTEVLKSLAAPPALEKKPQVAYK
TEIIGGVVVHTPISQNGGDGQEGSEPGSHTILRRSQSYIPTSGCRASTGP
PLIKSGYCVKQGNVRKSWKRRFFALDDFTICYFKCEQDREPLRTIFFKDV
LKTHECLVKSGDLLMRDNLFEIITSSRTFYVQADSPEDMHSWIKEIGAAV
QALKCHP
(partielle humane TAPP2-Aminosäuresequenz)
 oder 
MPYVDRQNRICGFLDIEDNENSGKFLRRYFILDTQANCLLWYMDNPQNLA
VGAGAVGSLQLTYISKVSIATPKQKPKTPFCFVINALSQRYFLQANDQKD
LKDWVEALNQASKITVPKAGTVPLATEVLKNLTAPPTLEKKPQVAYKTEI
IGGVVVQTPISQNGGDGQEGCEPGTHAFLRRSQSYIPTSGCRPSTGPPLI
KSGYCVKQGNVRKSWKRRFFALDDFTICYFKCEQDREPLRTIPLKDVLKT
HECLVKSGDLLMRDNLFEIITTSRTFYVQADSPEDMHSWIEGIGAAVQAL
KCHPREPSFSRSISLTRPGSSTLTSAPNSILSRRRPPAEEKRGLCKAPSV
ASSWQPWTPVPQAEEKPLSVEHAPEDSLFMPNPGESTATGVLASSRVRHR
SEPQHPKEKPFVFNLDDENIRTSDV
(Maus-TAPP2-Aminosäuresequenz)
 oder ein Fragment hiervon umfasst, wobei sich das Fragment spezifisch an PtdIns(3,4)P₂ binden kann, sich aber nicht an PtdIns(3,4,5)P₃ binden kann, oder wobei das Polypeptid eine Fusion eines genannten Fragments davon aufweist, wobei die Zusammensetzung einen Proteingehalt oder -inhalt hat, der wenigstens aus 50 Gew.-% des genannten Polypeptids besteht.

2. Rekombinantes Polynucleotid, das zur Expression eines Polypeptids wie in Anspruch 1 definiert geeignet ist.

3. Vektor, der zur Replikation in einer Säugetier-/eukaryotischen Zelle geeignet ist, mit einem Polynucleotid, das das Polypeptid wie in Anspruch 1 definiert kodiert.

4. Polynucleotid oder Vektor nach Anspruch 2 oder 3, das/der keine Introns enthält.

5. Wirtszelle, die ein rekombinantes Polynucleotid oder einen replizierbaren Vektor nach einem der Ansprüche 2 bis 4 umfasst.

6. Verfahren zur Herstellung eines Polypeptids wie in Anspruch 1 definiert, wobei das Verfahren das Kultivieren einer Wirtszelle nach Anspruch 5, die das genannte Polypeptid exprimiert, und das Isolieren des genannten Polypeptids beinhaltet

7. Verfahren nach Anspruch 6, wobei die genannte Wirtszelle eine eukaryotische Zelle ist.

8. Antikörper, der gegenüber einem Polypeptid mit der folgenden Aminosäuresequenz reaktiv ist: 
MPYVDRQNRICGFLDIEEHENSGKFLRRYFILDTQANCLLWYMDNPQ
NLAMGAGAVGALQLTYISKVSIATPKQKPKTPFCFVINALSQRYFLQAND
QKDMKDWVEALNQASKITVPKGGGLPMTTEVLKSLAAPPALEKKPQVAYK
TEIIGGVVVHTPISQNGGDGQEGSEPGSHTILRRSQSYIPTSGCRASTGP
PLIKSGYCVKQGNVRKSWKRRFFALDDFTICYFKCEQDREPLRTIFFKDV
LKTHECLVKSGDLLMRDNLFEIITSSRTFYVQADSPEDMHSWIKEIGAAV
QALKCHP
(partielle humane TAPP2-Aminosäuresequenz)
 oder 
MPYVDRQNRICGFLDIEDNENSGKFLRRYFILDTQANCLLWYMDNPQNLA
VGAGAVGSLQLTYISKVSIATPKQKPKTPFCFVINALSQRYFLQANDQKD
LKDWVEALNQASKITVPKAGTVPLATEVLKNLTAPPTLEKKPQVAYKTEI
IGGVVVQTPISQNGGDGQEGCEPGTHAFLRRSQSYIPTSGCRPSTGPPLI
KSGYCVKQGNVRKSWKRRFFALDDFTICYFKCEQDREPLRTIPLKDVLKT
HECLVKSGDLLMRDNLFEIITTSRTFYVQADSPEDMHSWIEGIGAAVQAL
KCHPREPSFSRSISLTRPGSSTLTSAPNSILSRRRPPAEEKRGLCKAPSV
ASSWQPWTPVPQAEEKPLSVEHAPEDSLFMPNPGESTATGVLASSRVRHR
SEPQHPKEKPFVFNLDDENIRTSDV
(Maus-TAPP2-Aminosäuresequenz),
 wobei der Antikörper nicht mit einem anderen Polypeptid reagiert, das eine PH-Domäne umfasst.

9. Verwendung eines Polypeptids nach Anspruch 1 in einem Screeningverfahren zum Identifizieren einer Verbindung oder Mischung, die zum Modulieren der Signalisierung durch PtdIns(3,4)P₂ geeignet ist.

10. Verwendung nach Anspruch 9, wobei das Verfahren die folgenden Schritte beinhaltet: (1) Aussetzen des genannten Polypeptids mit PtdIns(3,4)P₂ in Anwesenheit einer Testverbindung oder -mischung; (2) Bestimmen, ob die Testverbindung oder -Mischung die Bindung von PtdIns(3,4)P₂ an das genannte Polypeptid moduliert; und (3) Auswählen einer Verbindung oder Mischung, die die Bindung von PtdIns(3,4)P₂ an das genannte Polypeptid moduliert.

11. Verfahren zum Identifizieren einer Verbindung oder Mischung, die die Phospholipidbindungsaktivität eines Polypeptids wie in Anspruch 1 definiert moduliert, wobei das Verfahren Folgendes beinhaltet: Inkontaktbringen einer Verbindung oder Mischung mit dem genannten Polypeptid und Bestimmen, ob die Phospholipidbindungsaktivität des genannten Polypeptids in Anwesenheit der Verbindung oder Mischung im Vergleich zu der in Abwesenheit der genannten Verbindung oder Mischung geändert wird oder ist.

12. Verfahren nach Anspruch 11, wobei die genannte Bindungsaktivität verringert wird oder ist.

13. Verfahren nach Anspruch 11, wobei die genannte Bindungsaktivität erhöht wird oder ist.

14. Verfahren nach einem der Ansprüche 11 bis 13, wobei das Verfahren in einer Zelle durchgeführt wird.

15. Verfahren für den Nachweis und/oder die quantitative Bestimmung von PtdIns(3,4)P₂ in einer Probe, wobei die Probe einem Polypeptid wie in Anspruch 1 definiert ausgesetzt wird und die Bindung des genannten Polypeptids an irgendein anwesendes PtdIns(3,4)P₂ nachgewiesen oder erfasst wird.

16. Verfahren nach Anspruch 15, wobei das Verfahren in Zellen durchgeführt wird.

17. Verfahren nach einem der Ansprüche 15 bis 16, wobei das genannte Polypeptid einen Chromophor umfasst.

18. Kit mit Teilen, die zur Durchführung eines Verfahrens nach einem der Ansprüche 16 bis 17 nützlich sind, der ein Polypeptid nach Anspruch 1 umfasst.

## Revendications

1. Composition comprenant un polypeptide capable de se lier spécifiquement à PtdIns(3,4)P₂ mais incapable de se lier à PtdIns(3,4,5)P₃, dans laquelle le polypeptide comprend un domaine PH capable de se lier à PtdIns(3,4)P₂ mais incapable de se lier à PtdIns(3,4,5)P₃, dans laquelle le polypeptide comprend la séquence d'acides aminés 
MPYVDRQNRICGFLDIEEHENSGKFLRRYFILDTQANCLLWYMDNPQ
NLAMGAGAVGALQLTYISKVSIATPKQKPKTPFCFVINALSQRYFLQAND
QKDMKDWVEALNQASKITVPKGGGLPMTTEVLKSLAAPPALEKKPQVAYK
TEIIGGVVVHTPISQNGGDGQEGSEPGSHTILRRSQSYIPTSGCRASTGP
PLIKSGYCVKQGNVRKSWKRRFFALDDFTICYFKCEQDREPLRTIFFKDV
LKTHECLVKSGDLLMRDNLFEIITSSRTFYVQADSPEDMHSWIKEIGAAV
QALKCHP (séquence d'acides aminés TAPP2 humaine
partielle)
 ou 
MPYVDRQNRICGFLDIEDNENSGKFLRRYFILDTQANCLLWYMDNPQNLA
VGAGAVGSLQLTYISKVSIATPKQKPKTPFCFVINALSQRYFLQANDQKD
LKDWVEALNQASKITVPKAGTVPLATEVLKNLTAPPTLEKKPQVAYKTEI
IGGVVVQTPISQNGGDGQEGCEPGTHAFLRRSQSYIPTSGCRPSTGPPLI
KSGYCVKQGNVRKSWKRRFFALDDFTICYFKCEQDREPLRTIPLKDVLKT
HECLVKSGDLLMRDNLFEIITTSRTFYVQADSPEDMHSWIEGIGAAVQAL
KCHPREPSFSRSISLTRPGSSTLTSAPNSILSRRRPPAEEKRGLCKAPSV
ASSWQPWTPVPQAEEKPLSVEHAPEDSLFMPNPGESTATGVLASSRVRHR
SEPQHPKEKPFVFNLDDENIRTSDV (séquence
d'acides aminés TAPP2 de souris)
 ou comprend un fragment de celles-ci, ledit fragment étant capable de se lier spécifiquement à PtdIns(3,4)P₂ mais incapable de se lier à PtdIns(3,4,5)P₃, ou dans laquelle le polypeptide comprend une fusion dudit fragment de celles-ci, dans laquelle la composition comprend une teneur en protéines constituée d'au moins 50 % en masse dudit polypeptide.

2. Polynucléotide recombinant approprié à l'expression d'un polypeptide tel que défini dans la revendication 1.

3. Vecteur approprié à la réplication dans une cellule de mammifère/eucaryote, comprenant un polynucléotide codant pour le polypeptide tel que défini dans la revendication 1.

4. Polynucléotide ou vecteur selon la revendication 2 ou 3, qui ne contient pas d'introns.

5. Cellule hôte comprenant un polynucléotide recombinant ou un vecteur réplicable tel que défini dans l'une quelconque des revendications 2 à 4.

6. Procédé de production d'un polypeptide tel que défini dans la revendication 1, le procédé comprenant la culture d'une cellule hôte telle que définie dans la revendication 5 qui exprime ledit polypeptide, et l'isolement dudit polypeptide.

7. Procédé selon la revendication 6, dans lequel ladite cellule hôte est une cellule eucaryote.

8. Anticorps réactif vis-à-vis d'un polypeptide ayant la séquence d'acides aminés 
MPYVDRQNRICGFLDIEEHENSGKFLRRYFILDTQANCLLWYMDNPQ
NLAMGAGAVGALQLTYISKVSIATPKQKPKTPFCFVINALSQRYFLQAND
QKDMKDWVEALNQASKITVPKGGGLPMTTEVLKSLAAPPALEKKPQVAYK
TEIIGGVVVHTPISQNGGDGQEGSEPGSHTILRRSQSYIPTSGCRASTGP
PLIKSGYCVKQGNVRKSWKRRFFALDDFTICYFKCEQDREPLRTIFFKDV
LKTHECLVKSGDLLMRDNLFEIITSSRTFYVQADSPEDMHSWIKEIGAAV
QALKCHP (séquence d'acides aminés TAPP2 humaine
partielle)
 ou 
MPYVDRQNRICGFLDIEDNENSGKFLRRYFILDTQANCLLWYMDNPQNLA
VGAGAVGSLQLTYISKVSIATPKQKPKTPFCFVINALSQRYFLQANDQKD
LKDWVEALNQASKITVPKAGTVPLATEVLKNLTAPPTLEKKPQVAYKTEI
IGGVVVQTPISQNGGDGQEGCEPGTHAFLRRSQSYIPTSGCRPSTGPPLI
KSGYCVKQGNVRKSWKRRFFALDDFTICYFKCEQDREPLRTIPLKDVLKT
HECLVKSGDLLMRDNLFEIITTSRTFYVQADSPEDMHSWIEGIGAAVQAL
KCHPREPSFSRSISLTRPGSSTLTSAPNSILSRRRPPAEEKRGLCKAPSV
ASSWQPWTPVPQAEEKPLSVEHAPEDSLFMPNPGESTATGVLASSRVRHR
SEPQHPKEKPFVFNLDDENIRTSDV (séquence d'acides aminés
TAPP2 de souris),
 dans lequel l'anticorps ne réagit pas avec un polypeptide différent comprenant un domaine PH.

9. Utilisation d'un polypeptide tel que défini dans la revendication 1 dans un procédé de ciblage pour identifier un composé approprié à la modulation de la signalisation par PtdIns(3,4)P₂.

10. Utilisation selon la revendication 9, dans laquelle le procédé comprend les étapes consistant à (1) exposer ledit polypeptide à PtdIns(3,4)P₂ en présence d'un composé d'essai ; (2) déterminer si le composé d'essai module la liaison de PtdIns(3,4)P₂ audit polypeptide ; et (3) sélectionner un composé qui module la liaison de PtdIns(3,4)P₂ audit polypeptide.

11. Procédé d'identification d'un composé qui module l'activité de liaison aux phospholipides d'un polypeptide tel que défini dans la revendication 1, le procédé comprenant la mise en contact d'un composé avec ledit polypeptide et la détermination du fait que l'activité de liaison aux phospholipides dudit polypeptide est modifiée en présence du composé par rapport à celle obtenue en l'absence dudit composé.

12. Procédé selon la revendication 11, dans lequel ladite activité de liaison est réduite.

13. Procédé selon la revendication 11, dans lequel ladite activité de liaison est augmentée.

14. Procédé selon l'une quelconque des revendications 11 à 13, lequel procédé est effectué dans une cellule.

15. Procédé de détection et/ou quantification de PtdIns(3,4)P₂ dans un échantillon, dans lequel l'échantillon est exposé à un polypeptide tel que défini dans la revendication 1 et la liaison dudit polypeptide à un quelconque PtdIns(3,4)P₂ présent est détectée.

16. Procédé selon la revendication 15, lequel procédé est effectué dans des cellules.

17. Procédé selon l'une quelconque des revendications 15 à 16, dans lequel ledit polypeptide comprend un chromophore.

18. Kit d'éléments utile pour effectuer un procédé selon l'une quelconque des revendications 16 à 17, comprenant un polypeptide tel que défini dans la revendication 1.
